# EUROPEAN PATENT SPECIFICATION

(11) **EP 3 867 647 B1**
(45) Date of publication and mention of the grant of the patent: **25.01.2023**
(21) Application number: 19831825.5
(22) Date of filing: 19.12.2019
(51) Int. Cl.: G01N 33/574

(54) **USE OF MCM5 AS A MARKER FOR GYNAECOLOGICAL CANCERS**
VERWENDUNG VON MCM5 ALS MARKER FÜR GYNÄKOLOGISCHE KREBSE
UTILISATION DE MCM5 COMME MARQUEUR POUR LES CANCERS GYNÉCOLOGIQUES

(30) Priority: 20.12.2018 GB 201820867
(43) Date of publication of application: 25.08.2021
(60) Divisional application: 22213562.6
(73) Proprietor: Arquer Diagnostics Limited, Sunderland, Tyne and Wear SR5 2TA (GB)
(72) Inventor: STOCKLEY, Jacqueline, Sunderland Tyne and Wear SR5 2TA (GB); NYBERG, Cheryl, Sunderland Tyne and Wear SR5 2TA (GB)
(74) Representative: J A Kemp LLP
(86) International application number: PCT/GB2019/053626
(87) International publication number: WO 2020/128485

(56) References cited:
- EP-A1- 1 025 444
- US-B1- 6 303 323
- Anonymous: "A study looking at the MCM5 test to diagnose gynaecological cancers", Cancer Research UK, 31 January 2018 (2018-01-31), XP55674930, Retrieved from the Internet: URL:https://www.cancerresearchuk.org/about -cancer/find-a-clinical-trial/a-study-look ing-at-the-mcm5-test-to-diagnose-gynaecolo gical-cancers [retrieved on 2020-03-09]
- Anonymous: "EVALUATION OF MCM5 ELISA IN THE DIAGNOSIS OF GYNAECOLOGICAL MALIGNANCY - Health Research Authority", , 22 November 2016 (2016-11-22), XP55674924, Retrieved from the Internet: URL:https://www.hra.nhs.uk/planning-and-im proving-research/application-summaries/res earch-summaries/evaluation-of-mcm5-elisa-i n-the-diagnosis-of-gynaecological-malignan cy/ [retrieved on 2020-03-09]
- Arquer: "Step change in oncology diagnostics", , 1 May 2018 (2018-05-01), pages 1-1, XP55873743, Retrieved from the Internet: URL:https://media.nature.com/original/maga zine-assets/d43747-020-00483-9/d43747-020- 00483-9.pdf [retrieved on 2021-12-16]

## Description

### Field of the Invention

The present invention relates to methods for detecting the presence or absence of a gynaecological cancer in a subject and the use of a lysis buffer in said method. The present invention also relates to a method for diagnosing a subject as having a gynaecological cancer or a benign gynaecological condition. The present invention also relates to a method for distinguishing between a urine, tampon or vaginal swab sample associated with a gynaecological cancer and a urine, tampon or vaginal swab sample associated with a benign gynaecological cancer. The present invention further relates to a method for stratifying a subject into one of two treatment groups. The present invention also relates to the use of a first monoclonal antibody and/or a second monoclonal antibody in a method of detecting the presence or absence of a gynaecological cancer in a subject. The present invention further relates to use of a kit comprising a lysis buffer, a first monoclonal antibody and/or a second monoclonal antibody in a method of detecting the presence or absence of a gynaecological cancer in a subject.

### Background of the Invention

There is an unmet medical need for methods for diagnosing gynaecological cancers, such as ovarian and endometrial cancers, at an early stage. Ovarian cancer is one of the leading causes of female cancer death in the UK with almost 4,300 deaths every year. This is in part owed to the late presentation of ovarian cancer. Of the 7,000 diagnosed ovarian cancers every year, the majority will present with advanced disease due to the non-specific symptoms associated with the disease. Consequently, there is interest in development of a screening programme to enable earlier detection, which has the potential to increase the likelihood of survival. Currently, only 40 % of patients diagnosed with ovarian cancer will survive 5 years.

The largest ever ovarian cancer screening trial (UKCTOCS) was published in 2016, and found that, with current screening methods, consisting of a CA125 blood test and transvaginal scans, there was no significant effect on mortality. The low specificity of the screen resulted in a number of patients with benign disease undergoing complicated surgery unnecessarily, when compared to the control population (Lancet 2016; 387: 945-56).

US 6,303,323 relates to the detection of dysplastic or neoplastic cells using anti-MCM5 antibodies.

EP 1025444 concerns a method of determining the presence or absence of abnormally proliferating cells or cellular growth abnormality in a sample from an individual, the method including detecting in the sample a target polypeptide, wherein the target polypeptide is a member of the preinitiation complex of DNA replication.

Information relating to a study looking at an MCM5 test to diagnose gynaecological cancers was made available at cancerresearchuk.org/about-cancer/find-a-clinical-trial/a-study-looking-at-the-mcm5-test-to-diagnose-gynaecological-cancers and hra.nhs.uk/planning-and-improving-research/application-summaries/research -summaries/evaluation-of-mcm5-elisa-in-the-diagnosis-of-gynaecological-malignancy/.

Therefore there is a real unmet need for a screening tool for ovarian cancer which can detect early stage disease.

Endometrial cancer is the 4^{th} most commonly diagnosed cancer in women in the UK with 9000 women being diagnosed every year. As a result of the more specific symptoms, endometrial cancer is diagnosed relatively early. Statistics show that when diagnosed at its earliest stage, 95 % of patients with endometrial cancer will survive for five years or more, versus only 15 % of patients diagnosed at the latest stage. Therefore, an increase in earlier detection through the use of a screening tool for endometrial cancer could help increase survival.

Thus, there remains a real unmet need for non-surgical methods that can diagnose gynaecological cancers with high specificity and sensitivity. Furthermore, there remains a real unmet need for such methods that can easily be performed in the clinic or even in the home.

### Summary of the Invention

The scope of the invention is defined by the claims. Any aspects, embodiments and examples of the present disclosure which do not fall under the scope of the appended claims do not form part of the invention and are merely provided for illustrative purposes. The present invention provides methods and uses for the early detection of gynaecological cancers without the need for invasive surgical procedures. The methods of the present invention can be performed using urine, tampon or vaginal swab samples and are therefore suitable for use in the clinical laboratory and/or for point-of-care applications. Furthermore, the methods of the present invention are easy to carry out as they do not require complicated processing steps.

In particular, the present inventors have demonstrated that gynaecological cancers can be readily detected directly in urine, tampon or vaginal swab samples with high accuracy. Particularly high sensitivity for low grade and early stage gynaecological cancers has also been demonstrated for the methods and uses of the present invention and the kits described herein. Furthermore, the present inventors have found that methods and uses of the present invention and the kits described herein are able to accurately detect gynaecological cancer in subjects having the most common benign gynaecological conditions. These results demonstrate that detection of gynaecological cancers in urine, tampon or vaginal swab samples has clear potential both as a diagnostic test in a symptomatic population and as a screening tool to identify gynaecological cancers in an asymptomatic population. Ultimately, this enables earlier diagnosis and treatment, which is known to improve survival in gynaecological cancer.

Thus, , the present invention provides a method for detecting the presence or absence of a gynaecological cancer in a subject, the method comprising steps of:
obtaining a urine, tampon or vaginal swab sample isolated from the subject; and
detecting minichromosome maintenance complex component 5 (MCM5) or determining the concentration of MCM5; wherein the step of detecting MCM5 or determining the concentration of MCM5 comprises performing an ELISA assay to detect MCM5 or
determine the concentration of MCM5 .

In one embodiment, the method further comprises a step of treating the urine, tampon or vaginal swab sample to release MCM5 from cells in the urine, tampon or vaginal swab sample. In one embodiment, the method further comprises comparing the concentration of MCM5 determined to a reference. The reference may be an average concentration of the MCM5 determined for one or more samples prepared from one or more healthy individuals.

In one embodiment, a gynaecological cancer is likely to be present if the concentration of MCM5 is abnormal compared to the reference. In one embodiment, a gynaecological cancer is likely to be present if the concentration of MCM5 is higher than the reference.

According to the present invention, the step of determining the concentration of MCM5 comprises: performing an ELISA assay to detect MCM5 or measure the concentration of MCM5. In one embodiment, the gynaecological cancer is likely to be present if the concentration of MCM5 is determined to be higher than 5 pg /mL, 6 pg/mL, 7 pg/mL, 8 pg/mL, 9 pg/mL, 10 pg/mL, 11 pg/mL, 12 pg/mL, 13 pg/mL, 14 pg/mL, 15 pg/mL, 16 pg/mL, 17 pg/mL, 18 pg/mL, 19 pg/mL, 20 pg/mL, 21 pg/mL, 22 pg/mL, 23 pg/mL, 24 pg/mL, 25 pg/mL, 26 pg/mL, 27 pg/mL, 28 pg/mL, 29 pg/mL, 30 pg/mL, 35 pg/mL, 45 pg/mL, 50 pg/mL, 55 pg/mL, 60 pg/mL, 65 pg/mL, or 70 pg/mL. In one embodiment the gynaecological cancer is likely to present if the concentration of MCM5 is determined to be higher than about 12 pg/mL. In one embodiment the gynaecological cancer is likely to present if the concentration of MCM5 is determined to be higher than about 17 pg/mL. In one embodiment the gynaecological cancer is likely to present if the concentration of MCM5 is determined to be higher than about 70 pg/mL.

In one embodiment, a 12 pg/mL, 17 pg/mL or 70 pg/mL cut-off is applied. In one embodiment, a 12 pg/mL cut-off is applied. In such cases, the user determines whether the concentration of MCM5 is above the cut-off and if it is determines that it is likely that a gynaecological cancer is present.

In one embodiment, the method is a method for diagnosing a gynaecological cancer in a subject, optionally wherein the method further comprises a step of:
diagnosing the subject as having a gynaecological cancer if a gynaecological cancer is likely to be present.

In one embodiment, the step of treating the urine, tampon or vaginal swab to release MCM5 comprises exposing the urine, tampon or vaginal swab sample to a lysis buffer capable of releasing MCM5 from cells in the urine, tampon or vaginal swab sample.

In a further aspect, the invention provides a method for diagnosing a subject as having a gynaecological cancer or a benign gynaecological condition, the method comprising the steps of:
providing a urine, tampon or vaginal swab sample previously isolated from the subject;
determining the concentration of MCM5 in the urine, tampon or vaginal swab sample;
comparing the concentration of MCM5 in the urine, tampon or vaginal swab sample to a reference;
diagnosing the subject as having a gynaecological cancer if the concentration of MCM5 in the urine, tampon or vaginal swab sample is higher than the reference or diagnosing the subject as having a benign gynaecological condition if the concentration of MCM5 in the urine, tampon or vaginal swab sample is lower than the reference.

In another aspect, the invention provides a method for distinguishing between a urine, tampon or vaginal swab sample associated with a gynaecological cancer and a urine, tampon or vaginal swab sample associated with a benign gynaecological cancer, the method comprising the steps of:
providing a urine, tampon or vaginal swab sample previously isolated from a subject;
determining the concentration of MCM5 in the urine, tampon or vaginal swab sample;
comparing the concentration of MCM5 in the urine, tampon or vaginal swab sample to a reference;
determining that the urine, tampon or vaginal swab sample is associated with a gynaecological cancer if the concentration of MCM5 in the urine, tampon or vaginal swab sample is higher than the reference or determining that the urine, tampon or vaginal swab sample is associated with a benign gynaecological condition if the concentration of MCM5 in the urine, tampon or vaginal swab sample is lower than the reference.

In further aspect, the invention provides method for stratifying a subject into a first or a second treatment group, the method comprising the steps of:
providing a urine, tampon or vaginal swab sample previously isolated from the subject;
determining the concentration of MCM5 in the urine, tampon or vaginal swab sample;
allocating the subject to one of the two treatment groups based on the concentration of MCM5 in the urine, tampon or vaginal swab sample, wherein the first treatment group is to receive treatment for a gynaecological cancer and the second treatment group is to receive no treatment or treatment for a benign gynaecological condition.

In one embodiment, the subject is stratified into the first treatment group if the concentration of MCM5 in the urine, tampon or vaginal swab sample is higher than a reference or the subject is stratified into the second treatment group if the concentration of MCM5 in the urine, tampon or vaginal swab sample is lower than the reference.

In specific embodiments of the methods for diagnosing a subject as having a gynaecological cancer or a benign gynaecological condition described herein, the methods for distinguishing between a urine, tampon or vaginal swab sample associated with a gynaecological cancer and a urine, tampon or vaginal swab sample associated with a benign gynaecological cancer described herein, or the methods for stratifying a subject into a first or a second treatment group described herein:
(i) the benign gynaecological condition is selected from post-menopausal bleeding (PMB), endometriosis, fibroids, and polycystic ovary syndrome (PCOS);
(ii) the urine, tampon or vaginal swab sample is a urine sample; and/or
(iii) the reference is about 12 pg/mL.

In one embodiment, the step of treating the urine, tampon or vaginal swab sample to release MCM5 comprises:
passing the urine, tampon or vaginal swab sample through a filter for capturing cells, such that cells are captured in the filter;
passing a lysis buffer through the filter, such that the captured cells are exposed to the lysis buffer; and/or
incubating the filter for a period of time, such that the lysis buffer causes the cells to release MCM5.

In one embodiment, the lysis buffer is capable of releasing MCM5 from cells in the urine, tampon or vaginal swab sample.

In one embodiment, the lysis buffer is capable of releasing MCM5 from cells in the urine, tampon or vaginal swab sample and does not substantially denature MCM5 protein. In one embodiment, the lysis buffer does not denature an antibody.

In one embodiment, the lysis buffer comprises a detergent. The detergent may comprises or consists of Triton X-100. The detergent may comprises Triton X-100 at a concentration between 0.01 % and 25 %, between 0.01 % and 10 %, between 0.05 % and 5 %, between 0.1 % and 2 %, between 0.5 % and 2 %, between 0.75% and 1.25%, or about 1 %. The detergent may comprise sodium deoxycholate. The detergent may comprise sodium deoxycholate at a concentration between 0.1 % and 20 %, between 0.1 and 10 %, between 0.1 and 5 %, between 0.5 % and 5 %, between 0.5 % and 2.5 %, between 0.75 % and 2.5 %, between 0.75 % and 1.25 %, or about 1 %. The detergent may comprise sodium dodecyl sulphate (SDS). The detergent may comprise sodium dodecyl sulphate (SDS) at a concentration between 0.001 % and 10 %, between 0.01 % and 5 %, between 0.05 % and 5 %, between 0.01 % and 1 %, between 0.05 % and 1 %, between 0.05 % and 0.5%, between 0.075% and 0.25 %, or about 0.1 %. In one embodiment, the detergent consists of of Triton X-100, sodium deoxycholate, and sodium dodecyl sulphate (SDS). In one embodiment, the detergent consists of between 0.5 % and 2 % of Triton X-100, between 0.5 % and 2 % of sodium deoxycholate, and between 0.05 % and 0.5 % of sodium dodecyl sulphate (SDS).

In one embodiment, the lysis buffer comprises a buffer component. The buffer component may have a pH of between pH 4 and pH 9, between pH 5 and pH 8.5, between pH 6 and pH 8, between pH 6.5 and pH 8, between pH 7 and pH 8, between pH 7.3 and pH 7.9, between pH 7.4 and pH 7.8, between pH 7.5 and pH 7.7, or about pH 7.6; and/or maintains the pH of the lysis buffer at between pH 4 and pH 9, between pH 5 and pH 8.5, between pH 6 and pH 8, between pH 6.5 and pH 8, between pH 7 and pH 8, between pH 7.3 and pH 7.9, between pH 7.4 and pH 7.8, between pH 7.5 and pH 7.7, or about pH 7.6. The buffer component may comprises or consists of Tris. The buffer component may comprise or consist of Tris at a concentration greater than 1 mM, between 1 mM and 350 mM, between 5 and 200 mM, between 5 and 100 mM, between 5 and 50 mM, between 5 mM and 40 mM, between 5 mM and 35 mM, between 10 mM and 35 mM, between 15 mM and 35 mM, between 15 mM and 30 mM, between 20 mM and 30 mM, or about 25 mM. In one embodiment, the buffer component consists of Tris at a concentration of between 15 mM and 35 mM.

In one embodiment, the lysis buffer comprises a salt. The salt may be sodium chloride. In one embodiment, the lysis buffer comprises sodium chloride at a concentration between 10 mM and 350 mM, between 20 mM and 300 mM, between 50 mM and 250 mM, between 100 mM and 250, between 100 mM and 200 mM, between 125 mM and 175 mM, or about 150 mM.

In one embodiment, the lysis buffer comprises:
(i) between 1 mM and 100 mM Tris;
(ii) between 50 mM and 300 mM sodium chloride;
(iii) between 0.1 and 5 % sodium deoxycholate;
(iv) between 0.01 and 1 % sodium dodecyl sulphate; and/or
(v) between 0.1 and 5 % Triton-X 100.

In one embodiment, the lysis buffer comprises:
(i) between 10 mM and 40 mM Tris;
(ii) between 100 mM and 200 mM sodium chloride;
(iii) between 0.5 and 2 % sodium deoxycholate;
(iv) between 0.05 and 0.5 % sodium dodecyl sulphate; and/or
(v) between 0.5 and 2 % Triton-X 100.

In one embodiment, the lysis buffer comprises:
(i) about 25 mM Tris;
(ii) about 150 mM sodium chloride;
(iii) about 1 % sodium deoxycholate;
(iv) about 0.1 % sodium dodecyl sulphate; and/or
(v) about 1 % Triton-X100.

In one embodiment, the step of detecting MCM5 or determining the concentration of MCM5 comprises:
exposing the urine, tampon or vaginal swab sample to a first monoclonal antibody and/or a second monoclonal antibody; and
detecting MCM5 bound to the first monoclonal antibody and/or the second monoclonal antibody or determining the concentration of MCM5 bound to the first monoclonal antibody and/or the second monoclonal antibody. In one embodiment, the first monoclonal antibody and the second monoclonal antibody bind to MCM5.

In one embodiment, the first monoclonal antibody is be an antibody which:
(i) binds to a polypeptide having an amino acid sequence of SEQ ID NO: 1;
(ii) comprises at least one Complementary Determining Region (CDR) selected from the group consisting of:
   (a) 12A7 CDRH1 which has a sequence of SEQ ID NO: 9 or a sequence that differs from SEQ ID NO:9 by a single amino acid substitution;
   (b) 12A7 CDRH2 which has a sequence of SEQ ID NO: 11 or a sequence that differs from SEQ ID NO: 11 by a single amino acid substitution;
   (c) 12A7 CDRH3 which has a sequence of SEQ ID NO: 13 or a sequence that differs from SEQ ID NO: 13 by a single amino acid substitution;
   (d) 12A7 CDRL1 which has a sequence of SEQ ID NO: 3 or a sequence that differs from SEQ ID NO:3 by a single amino acid substitution;
   (e) 12A7 CDRL2 which has a sequence of SEQ ID NO: 5 or a sequence that differs from SEQ ID NO:5 by a single amino acid substitution; and
   (f) 12A7 CDRL3 which has a sequence of SEQ ID NO: 7 or a sequence that differs from SEQ ID NO:7 by a single amino acid substitution;
(iii) comprises a heavy chain variable region having a sequence at least 85%, 90%, 95%, 98%, 99%, or 100% identical to SEQ ID NO: 29;
(iv) comprises a light chain variable region sequence having a sequence at least 85%, 90%, 95%, 98%, 99%, or 100% identical to SEQ ID NO: 27; or
(v) competes with the antibody of (i), (ii), (iii), or (iv).

In one embodiment, the second monoclonal antibody is an antibody which:
(i) binds to a polypeptide having an amino acid sequence of SEQ ID NO: 2;
(ii) comprises at least one Complementary Determining Region (CDR) selected from the group consisting of:
   (a) 4B4 CDRH1 which has a sequence of SEQ ID NO: 21 or a sequence that differs from SEQ ID NO:21 by a single amino acid substitution;
   (b) 4B4 CDRH2 which has a sequence of SEQ ID NO: 23 or a sequence that differs from SEQ ID NO:23 by a single amino acid substitution;
   (c) 4B4 CDRH3 which has a sequence of SEQ ID NO: 25 or a sequence that differs from SEQ ID NO:25 by a single amino acid substitution;
   (d) 4B4 CDRL1 which has a sequence of SEQ ID NO: 15 or a sequence that differs from SEQ ID NO: 15 by a single amino acid substitution;
   (e) 4B4 CDRL2 which has a sequence of SEQ ID NO: 17 or a sequence that differs from SEQ ID NO: 17 by a single amino acid substitution; and
   (f) 4B4 CDRL3 which has a sequence of SEQ ID NO: 19 or a sequence that differs from SEQ ID NO: 19 by a single amino acid substitution;
(iii) comprises a heavy chain variable region having a sequence at least 85%, 90%, 95%, 98%, 99%, or 100% identical to SEQ ID NO: 33;
(iv) comprises a light chain variable region sequence having a sequence at least 85%, 90%, 95%, 98%, 99%, or 100% identical to SEQ ID NO: 31; or
(v) competes with the antibody of (i), (ii), (iii), or (iv).

In one embodiment, the first monoclonal antibody and/or second monoclonal antibody has an affinity for MCM5 in the range of 0.001-10 nM, 0.001-5 nM, 0.001-1 nM, 0.005-1 nM, 0.01-0.5 nM, 0.01-0.25 nM, 0.025-0.25 nM, or 0.04-0.25 nM. In one embodiment, the first and/or second monoclonal antibody has an affitinity for MCM5 of about 0.01 nM, 0.02 nM, 0.03 nM, 0.04 nM, 0.05 nM, 0.06 nM, 0.07 nM, 0.08 nM, 0.09 nM, 0.1 nM, 0.2 nM, 0.3 nM, 0.4 nM, or 0.5 nM. In one embodiment, the first monoclonal antibody and/or second monoclonal antibody has an affinity for MCM5 of about 0.05 nM. In one embodiment, the first monoclonal antibody and/or second monoclonal antibody has an affinity for MCM5 of about 0.2 nM. In one embodiment, the first monoclonal antibody and/or second monoclonal antibody has an affinity for MCM5 of about 0.233 nM. In one embodiment, the first monoclonal antibody has an affinity for MCM5 of about 0.05 nM and the second monoclonal antibody has an affinity for MCM5 of about 0.2 nM. In one embodiment, the first monoclonal antibody has an affinity for MCM5 of about 0.05 nM and the second monoclonal antibody has an affinity for MCM5 of about 0.233 nM. In one embodiment, the first monoclonal antibody is 12A7 and has an affinity for MCM5 of about 0.05 nM and the second monoclonal antibody is 4B4 and has an affinity for MCM5 of about 0.2 nM. In one embodiment, the first monoclonal antibody is 12A7 and has an affinity for MCM5 of about 0.05 nM and the second monoclonal antibody is 4B4 and has an affinity for MCM5 of about 0.233 nM.

In one embodiment, the first monoclonal antibody and/or the second monoclonal antibody is a Fab'₂, a F'(ab)₂, an Fv, a single chain antibody or a diabody.

In one embodiment, the first monoclonal antibody comprises 12A7 CDRH1, 12A7 CDRH2, and 12A7 CDRH3. In one embodiment, the first monoclonal antibody comprises 12A7 CDRL1, 12A7 CDRL2 and 12A7 CDRL3. In one embodiment, the first monoclonal antibody comprises a 12A7 CDRH1 which has a sequence of SEQ ID NO: 9, a 12A7 CDRH2 which has a sequence of SEQ ID NO: 11, and a 12A7 CDRH3 which has a sequence of SEQ ID NO: 13. In one embodiment, the first monoclonal antibody comprises a 12A7 CDRL1 which has a sequence of SEQ ID NO: 3, a 12A7 CDRL2 which has a sequence of SEQ ID NO: 5, and a 12A7 CDRL3 which has a sequence of SEQ ID NO: 7.

In one embodiment, the second monoclonal antibody comprises 4B4 CDRH1, 4B4 CDRH2, and 4B4 CDRH3. In one embodiment, the second monoclonal antibody comprises 4B4 CDRL1, 4B4 CDRL2 and 4B4 CDRL3. In one embodiment, the second monoclonal antibody comprises a 4B4 CDRH1 which has a sequence of SEQ ID NO: 21, a 4B4 CDRH2 which has a sequence of SEQ ID NO: 23, and a 4B4 CDRH3 which has a sequence of SEQ ID NO: 25. In one embodiment, the second monoclonal antibody has a 4B4 CDRL1 which has a sequence of SEQ ID NO: 15, a 4B4 CDRL2 which has a sequence of SEQ ID NO: 17, and a 4B4 CDRL3 which has a sequence of SEQ ID NO: 19.

In one embodiment, the first monoclonal antibody comprises a heavy chain variable region having a sequence at least 95 % identical to SEQ ID NO: 29. In one embodiment, first monoclonal antibody comprises a heavy chain variable region having a sequence at least 98 % identical to SEQ ID NO: 29. In one embodiment, the first monoclonal antibody comprises a light chain variable region having a sequence at least 95 % identical to SEQ ID NO: 27. In one embodiment, the first monoclonal antibody comprises a light chain variable region having a sequence at least 98 % identical to SEQ ID NO: 27.

In one embodiment, the second monoclonal antibody comprises a heavy chain variable region having a sequence at least 95 % identical to SEQ ID NO: 33. In one embodiment, the second monoclonal antibody comprises a heavy chain variable region having a sequence at least 98 % identical to SEQ ID NO: 33. In one embodiment, the second monoclonal antibody comprises a light chain variable region having a sequence at least 95 % identical to SEQ ID NO: 31. In one embodiment, the second monoclonal antibody comprises a light chain variable region having a sequence at least 98 % identical to SEQ ID NO: 31.

In one embodiment, the urine, tampon or vaginal swab sample is a urine sample, and the method has a higher sensitivity than an equivalent method performed using a swab sample and/or an equivalent method performed using a tampon sample. In one embodiment, the urine, tampon or vaginal swab sample is a urine sample, and the method has a higher specificity than an equivalent method performed using a swab sample and/or an equivalent method performed using a tampon sample.

In one embodiment, the method has a sensitivity for the gynaecological cancer of at least about 40 %, 42 %, 44 %, 46 %, 48 %, or 50 %. In one embodiment, the method has a sensitivity for the gynaecological cancer of at least about 50 %, 52 %, 54 %, 55 %, 56 %, 57 %, 58 %, 59 %, 60 %, or 61%. In one embodiment, the method has a sensitivity for the gynaecological cancer of at least about 75 %, 76 %, 77 %, 78 %, 79 %, 80 %, or 81 %. In one embodiment, the method has a sensitivity for the gynaecological cancer of at least about 90 %, 92 %, 93 %, 94 %, 95 %, or 96 %. In one embodiment, the method has a specificity for the gynaecological cancer of at least about 40 %, 41 %, 42 %, or 43 %. In one embodiment, the method has a specificity for the gynaecological cancer of at least about 50 %, 52 %, 54 %, 55 %, 56 %, 57 %, 58 %, 59 %, or 60 %. In one embodiment, the method has a specificity for the gynaecological cancer of at least about 60 %, 62 %, 64 %, 66 %, 68 %, 70 %, 71%, 72 %, 73 %, 74 %, or 75 %. In one embodiment, the method has a specificity for the gynaecological cancer of at least about 80 %, 82 %, 83 %, 84 %, 85 %, or 86%.

In one embodiment, the urine, tampon or vaginal swab sample is a urine sample and the method has a sensitivity for the gynaecological cancer of at least about 75 % and a specificity for the gynaecological cancer of at least about 55 %.

In one embodiment, the urine, tampon or vaginal swab sample is a vaginal swab sample and the method has a sensitivity for the gynaecological cancer of at least about 55 % and a specificity for the gynaecological cancer of at least about 70 %.

In one embodiment, the urine, tampon or vaginal swab sample is a tampon sample and the method has a sensitivity for the gynaecological cancer of at least about 90 % and a specificity for the gynaecological cancer of at least about 35 %.

In one embodiment, the urine, tampon or vaginal swab sample is a tampon sample and the method has a sensitivity for the gynaecological cancer of at least about 45 % and a specificity for the gynaecological cancer of at least about 80 %.

In one embodiment, the urine, tampon or vaginal swab sample is a tampon sample and the method has a sensitivity for the gynaecological cancer of at least about 90 % and a specificity for the gynaecological cancer of at least about 80 %.

In another aspect, the present invention provides the use of a lysis buffer as described herein in a method of detecting the presence or absence of a gynaecological cancer in a subject.

In a further aspect, the present invention provides the use of a first monoclonal antibody as described herein and/or a second monoclonal antibody as described herein in a method of detecting the presence or absence of a gynaecological cancer in a subject.

In a further aspect, the present invention provides the use of a kit in a method of detecting the presence or absence of a gynaecological cancer in a subject, wherein the kit comprises:
(a) a lysis buffer as described herein;
(b) a first monoclonal antibody as described herein; and/or
(c) a second monoclonal antibody as described herein.

In specific embodiments of the methods and uses of the present invention, the gynaecological cancer is a gynaecological malignancy. In specific embodiments of the methods and uses of the present invention, the gynaecological cancer is a gynaecological cancer selected from the group consisting of ovarian cancer, endometrial cancer, uterine cancer, cervical cancer, vulval cancer, vaginal cancer, fallopian tube tumour, epithelial ovarian tumour, ovarian teratoma, immature ovarian teratoma, stromal tumour, germ cell ovarian tumour, undifferentiated tumours, borderline ovarian tumour, endometrial carcinoma, endometrial adenocarcinoma, endometrioid adenocarcinoma, endometrial squamous cell carcinoma, serous carcinoma, serous endometrial intraepithelial carcinoma, endometrial carcinosarcoma, clear cell carcinoma, mucinous carcinoma, undifferentiated endometrial carcinoma, mixed endometrial carcinoma, mucinous tumour, malignant mixed Müllerian tumour, endometrial clear cell sarcoma. granulosa cell tumour, serous tubal intraepithelial carcinoma, immature cystic teratoma, serous ovarian tumour, and small cell carcinoma.

In specific embodiments of the methods and uses of the present invention, the gynaecological cancer is an ovarian cancer or an endometrial cancer.

In specific embodiments of the methods and uses of the present invention, the gynaecological cancer is an ovarian cancer. The ovarian cancer may be selected from the group consisting of epithelial ovarian tumour, ovarian teratoma, immature ovarian teratoma, stromal tumour, germ cell ovarian tumour, undifferentiated tumours, borderline ovarian tumour, mucinous tumour, granulosa cell tumour, and immature cystic teratoma, serous ovarian tumour, and small cell carcinoma.

In specific embodiments of the methods and uses of the present invention, the gynaecological cancer is an endometrial cancer. The endometrial cancer may be selected from the group consisting of endometrial carcinoma, endometrial adenocarcinoma, endometrioid adenocarcinoma, endometrial squamous cell carcinoma, serous carcinoma, serous endometrial intraepithelial carcinoma, endometrial carcinosarcoma, clear cell carcinoma, mucinous carcinoma, undifferentiated endometrial carcinoma, mixed endometrial carcinoma, malignant mixed Müllerian tumour, and endometrial clear cell sarcoma.

In specific embodiments of the methods and uses of the present invention, the gynaecological cancer is a low grade cancer. In specific embodiments of the methods and uses of the present invention, the gynaecological cancer is a G1 cancer. In specific embodiments of the methods and uses of the present invention, the gynaecological cancer is a high grade cancer. In specific embodiments of the methods, uses and kits of the present invention, the gynaecological cancer is a G2 or above cancer.

In specific embodiments of the methods and uses of the present invention, the gynaecological cancer is an early stage cancer. In specific embodiments of the methods and uses of the present invention, the gynaecological cancer is a S1 cancer. In specific embodiments of the methods and uses of the present invention, the gynaecological cancer is a late stage cancer. In specific embodiments of the methods and uses of the present invention, the gynaecological cancer is a S2 or above cancer.

In specific embodiments of the methods and uses of the present invention, the gynaecological cancer is an ovarian cancer and the method has a specificity for the ovarian cancer of at least about 25 %. In specific embodiments of the methods and uses of the present invention, the gynaecological cancer is an ovarian cancer and the method has a sensitivity for the ovarian cancer of at least about 50 %. In specific embodiments of the methods and uses of the present invention, the gynaecological cancer is an ovarian cancer and the method has a specificity for the ovarian cancer of at least about 25 % and a sensitivity for the ovarian cancer of at least about 50 %.

In specific embodiments of the methods and uses of the present invention, the gynaecological cancer is an endometrial cancer and the method has a specificity for the endometrial cancer of at least about 40 %. In specific embodiments of the methods and uses of the present invention, the gynaecological cancer is an endometrial cancer and the method has a sensitivity for the endometrial cancer of at least about 80 %. In specific embodiments of the methods and uses of the present invention, the gynaecological cancer is an endometrial cancer and the method has a specificity for the endometrial cancer of at least about 40 % and a sensitivity for the endometrial cancer of at least about 80 %.

In specific embodiments of the methods of the present invention, the urine, tampon or vaginal swab sample is a urine sample, the gynaecological cancer is an ovarian cancer, and the method has a sensitivity for the ovarian cancer of at least about 60 % and a specificity for the ovarian cancer of at least about 70 %, optionally wherein an MCM5 concentration reference of 12 pg/ml is applied.

In specific embodiments of the methods of the present invention, the urine, tampon or vaginal swab sample is a urine sample, the gynaecological cancer is an endometrial cancer, and the method has a sensitivity for the endometrial cancer of at least about 80 % and a specificity for the endometrial cancer of at least about 70 %, optionally wherein an MCM5 concentration reference of 12 pg/ml is applied.

In specific embodiments of the methods of the present invention, the urine, tampon or vaginal swab sample is a urine sample, the gynaecological cancer is a low grade ovarian cancer and the method has a sensitivity for the low grade ovarian cancer of at least about 70 %, optionally wherein an MCM5 concentration reference of about 12 pg/ml is applied.

In specific embodiments of the methods of the present invention, the urine, tampon or vaginal swab sample is a urine sample, the gynaecological cancer is an early stage ovarian cancer and the method has a sensitivity for the early stage ovarian cancer of at least about 70 %, optionally wherein an MCM5 concentration reference of about 12 pg/ml is applied.

In specific embodiments of the methods of the present invention, the urine, tampon or vaginal swab sample is a urine sample, the gynaecological cancer is a low grade endometrial cancer and the method has a sensitivity for the low grade endometrial cancer of at least about 80 %, optionally wherein an MCM5 concentration reference of about 12 pg/ml is applied.

In specific embodiments of the methods of the present invention, the urine, tampon or vaginal swab sample is a urine sample, the gynaecological cancer is an early stage endometrial cancer and the method has a sensitivity for the early stage endometrial cancer of at least about 85 %, optionally wherein an MCM5 concentration reference of about 12 pg/ml is applied.

In specific embodiments of the methods and uses of the present invention, the subject is a subject suffering from a benign gynaecological condition. The benign gynaecological condition may be selected from post-menopausal bleeding (PMB), endometriosis, fibroids, and polycystic ovary syndrome (PCOS).

### Brief Description of the Drawings

Figure 1 shows graphs comparing MCM5 levels in the urine of patients with endometrial cancer and urine from healthy individuals.

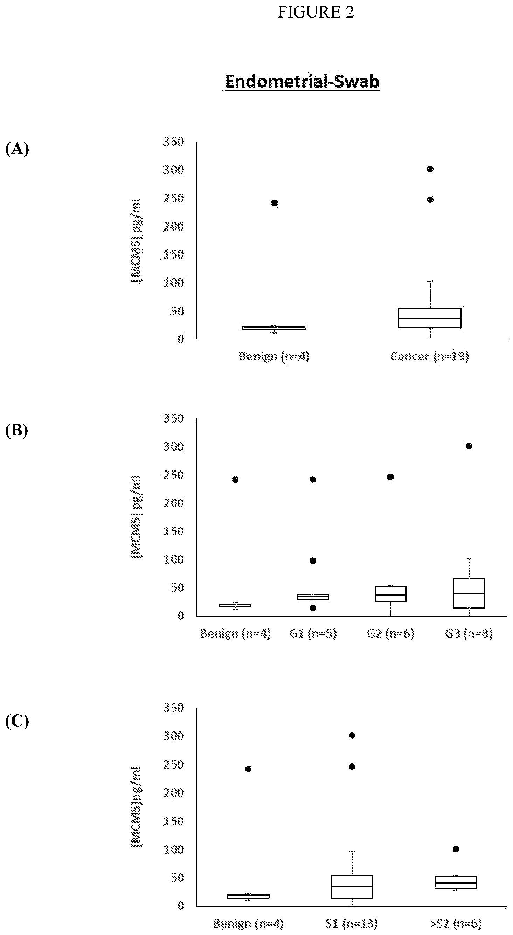

Figure 2 shows a graphs comparing MCM5 levels in vaginal swab samples from patients with endometrial cancer and healthy individuals.
Figure 3 shows a graphs comparing MCM5 levels in vaginal tampon samples from patients with endometrial cancer and healthy individuals.
Figure 4 shows graphs comparing MCM5 levels in the urine of patients with ovarian cancer and urine from healthy individuals.

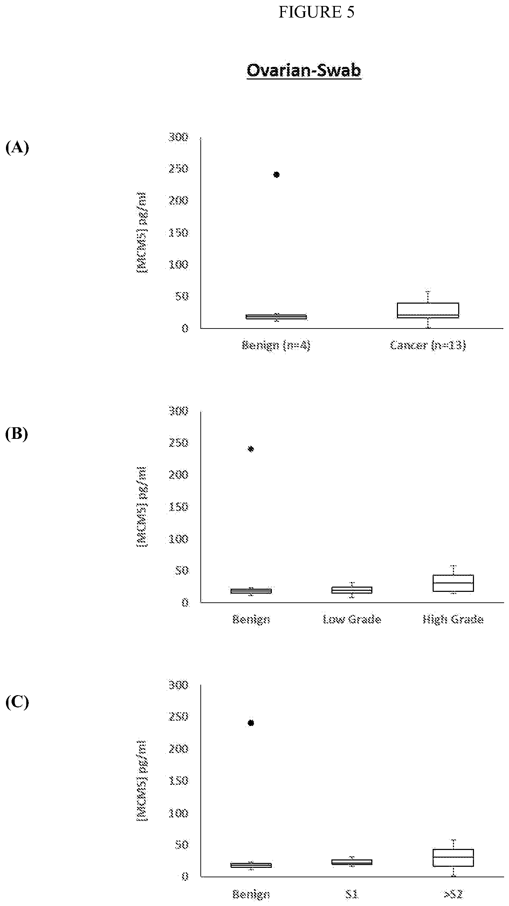

Figure 5 shows graphs comparing MCM5 levels in vaginal swab samples from patients with ovarian cancer and healthy individuals.
Figure 6 shows graphs comparing MCM5 levels in vaginal tampon samples from patients with ovarian cancer and healthy individuals.
Figure 7 shows graphs comparing MCM5 levels in urine samples (A), vaginal swab samples (B) and vaginal tampon samples (C) from patients with gynaecological cancers and equivalent samples from healthy individuals.

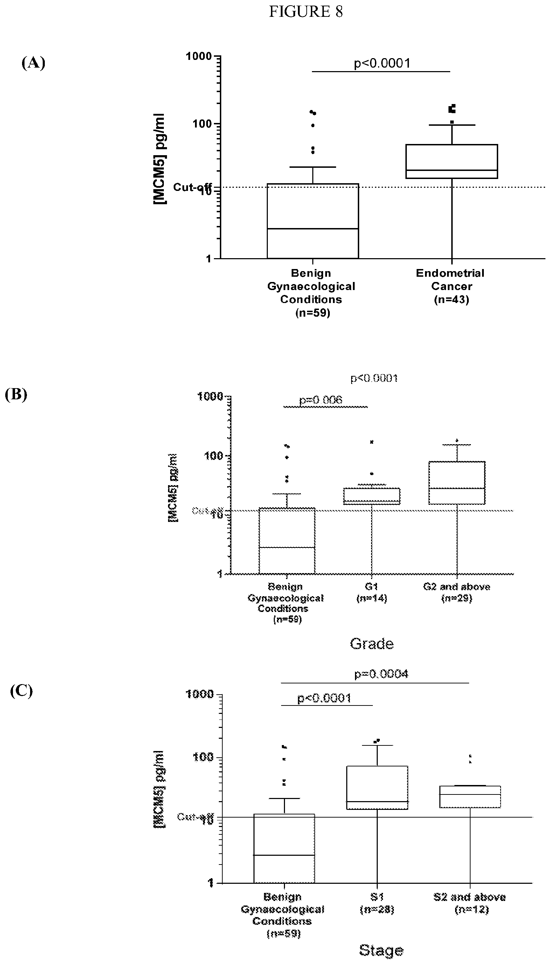

Figure 8 shows graphs comparing MCM5 levels in patient urine samples (horizontal "cut-off' line indicates 12 pg/mL). (A) MCM5 levels in urine samples from endometrial cancer patients and patients with benign gynaecological conditions. (B) MCM5 levels in urine samples from patients with benign gynaecological conditions and endometrial cancer patients of grade 1 (G1) and grade 2 (G2) and above. (C) MCM5 levels in urine samples from patients with benign gynaecological conditions and endometrial cancer patients stage 1 (S1), and stage 2 (S2) and above (N.B. data relating to three cancers of unknown stage was not included in Figure 8C).
Figure 9 shows a graph presenting the results of an MCM5 ELISA to measure MCM5 levels in urine samples from ovarian cancer patients and patients with benign gynaecological conditions (horizontal "cut-off' line indicates 12 pg/mL).
Figure 10 shows a graph comparing MCM5 levels in urine from patients with benign gynaecological conditions, *i.e.* endometriosis, polycystic ovary syndrome (PCOS), fibroids and post-menopausal bleeding and patients with endometrial cancer (horizontal "cut-off' line indicates 12 pg/mL).

### Detailed Description

### Definitions

The term "*comprises*" (comprise, comprising) should be understood to have its normal meaning in the art, *i.e.* that the stated feature or group of features is included, but that the term does not exclude any other stated feature or group of features from also being present. For example, a lysis buffer comprising a detergent may contain other components.

The term "*consists of*" should also be understood to have its normal meaning in the art, *i.e.* that the stated feature or group of features is included, to the exclusion of further features. For example a lysis buffer consisting of a detergent contains detergent and no other components. A lysis buffer comprising a detergent consisting of polysorbate 80 may comprise components other than detergents but the only detergent in the lysis buffer is polysorbate 80.

For every embodiment in which "*comprises*" or "*comprising*" is used, we anticipate a further embodiment in which "*consists* of" or "*consisting of*" is used. Thus, every disclosure of "*comprises*" should be considered to be a disclosure of "*consists of*"*.*

### Sequence Homology/Identity

Although sequence homology can also be considered in terms of functional similarity (i.e., amino acid residues having similar chemical properties/functions), in the context of the present document it is preferred to express homology in terms of sequence identity.

Sequence comparisons can be conducted by eye or, more usually, with the aid of readily available sequence comparison programs. These publicly and commercially available computer programs can calculate percent homology (such as percent identity) between two or more sequences.

Percent identity may be calculated over contiguous sequences, *i.e.,* one sequence is aligned with the other sequence and each amino acid in one sequence is directly compared with the corresponding amino acid in the other sequence, one residue at a time. This is called an "*ungapped*" alignment. Typically, such ungapped alignments are performed only over a relatively short number of residues (for example less than 50 contiguous amino acids). For comparison over longer sequences, gap scoring is used to produce an optimal alignment to accurately reflect identity levels in related sequences having insertion(s) or deletion(s) relative to one another. A suitable computer program for carrying out such an alignment is the GCG Wisconsin Bestfit package (University of Wisconsin, U.S.A; Devereux et al.., 1984, Nucleic Acids Research 12:387). Examples of other software than can perform sequence comparisons include, but are not limited to, the BLAST package, FASTA (Altschul et al.., 1990, J. Mol. Biol. 215:403-410) and the GENEWORKS suite of comparison tools.

Typically sequence comparisons are carried out over the length of the reference sequence. For example, if the user wished to determine whether a given sequence is 95 % identical to SEQ ID NO: 27, SEQ ID NO: 27 would be the reference sequence. For example, to assess whether a sequence is at least 80 % identical to SEQ ID NO: 27 (an example of a reference sequence), the skilled person would carry out an alignment over the length of SEQ ID NO: 27, and identify how many positions in the test sequence were identical to those of SEQ ID NO: 27. If at least 80 % of the positions are identical, the test sequence is at least 80 % identical to SEQ ID NO: 27. If the sequence is shorter than SEQ ID NO: 27, the gaps or missing positions should be considered to be non-identical positions.

The skilled person is aware of different computer programs that are available to determine the homology or identity between two sequences. For instance, a comparison of sequences and determination of percent identity between two sequences can be accomplished using a mathematical algorithm. In an embodiment, the percent identity between two amino acid or nucleic acid sequences is determined using the Needleman and Wunsch (1970) algorithm which has been incorporated into the GAP program in the Accelrys GCG software package (available at http://www.accelrys.com/products/gcg/), using either a Blosum 62 matrix or a PAM250 matrix, and a gap weight of 16, 14, 12, 10, 8, 6, or 4 and a length weight of 1, 2, 3, 4, 5, or 6.

For the purposes of the present invention, the term "*fragment*" refers to a contiguous portion of a reference sequence. For example, a fragment of SEQ ID NO: 28 of 50 nucleotides in length refers to 50 contiguous nucleotides of SEQ ID NO: 28. In another example, a fragment of SEQ ID NO: 27 of 50 amino acids in length refers to 50 contiguous amino acids of SEQ ID NO: 27.

In the context of the present document, a homologous amino acid sequence is taken to include an amino acid sequence which is at least 40, 50, 60, 70, 80 or 90 % identical. Most suitably a polypeptide having at least 90 % sequence identity to the biomarker of interest will be taken as indicative of the presence of that biomarker; more suitably a polypeptide which is 95 % or more suitably 98 % identical at the amino acid level will be taken to indicate presence of that biomarker. Suitably said comparison is made over at least the length of the polypeptide or fragment which is being assayed to determine the presence or absence of the biomarker of interest. Most suitably the comparison is made across the full length of the polypeptide of interest.

### Reference / Healthy individuals

In some embodiments, the methods of the present invention involve a step of detecting MCM5 or determining the concentration of MCM5 released from cells in a urine, tampon or vaginal swab sample. Where the method involves determining the concentration of MCM5, the method may further comprise a step of comparing the concentration of MCM5 to a reference.

In some embodiments, where the method involves a step of detecting MCM5, the step of detecting MCM5 is a qualitative step. In the context of the present invention, a qualitative step of detecting MCM5 is one that is performed without determining a numerical value for the concentration MCM5 in the urine, tampon or vaginal swab sample and/or without determining, or using a previously determined, numerical value for the concentration of MCM5 as a reference. For example, the user may not determine an actual value for the amount or concentration of MCM5, but may merely determine whether the amount or concentration of MCM5is different compared to a reference. Optionally, detecting MCM5comprises determining whether the amount or concentration of MCM5is higher than a reference.

In some embodiments, where the present invention involves a step of detecting MCM5, the step of detecting MCM5 comprises performing a qualitative comparison of the amount or concentration of MCM5released from cells in the urine, tampon or vaginal swab sample to a reference.

Suitably, the reference may be a concentration of MCM5determined for a sample obtained from one or more healthy individuals. A healthy individual is one who is not presently suffering from a gynaecological cancer. For example, the healthy individual may be one who has never suffered from a gynaecological cancer.

Where the reference used in the methods of the invention is the concentration of MCM5 determined for a sample prepared from one or more healthy individuals, the reference may be an average concentration of MCM5determined for multiple samples obtained from a single healthy individual. Alternatively, the reference may be an average concentration of MCM5 determined for multiple samples prepared from multiple healthy individuals. In this context, "*one or more samples*" may be at least 10, 100, 500, 1000, 10000, 100 000 or 1 000 000 samples.

In one embodiment, the reference can be an average concentration of MCM5determined from one or more samples prepared from one or more healthy individuals in parallel with detecting MCM5or determining the concentration of MCM5 released from the urine, tampon or vaginal swab sample obtained in the methods of the invention, e.g. the urine, tampon or vaginal swab sample being subject to a method of the present invention for detecting the presence or absence of gynaecological cancer.

In another embodiment, the reference can be an average concentration of MCM5previously determined for one or more samples prepared from a healthy individual. In such embodiments, a numeric comparison may be made by comparing the concentration of MCM5 determined for the urine, tampon or vaginal swab sample obtained in the invention to the reference. The advantage of this is not having to duplicate the analysis by determining a reference in parallel each time a urine, tampon or vaginal swab sample from a subject is analysed.

Suitably the reference may be matched to the subject being analysed *e.g.* by gender *e.g.* by age *e.g.* by ethnic background or other such criteria which are well known in the art. For example, the reference may suitably be matched to specific patient sub-groups e.g. elderly subjects, or those with a previous relevant history such as a predisposition to gynaecological cancer.

Suitably the reference may be matched to the sample type being analysed. For example, the concentration of MCM5 in the urine, tampon or vaginal swab sample obtained from the subject may vary depending on the type or nature of the sample. Thus, in some embodiments, it is desirable that the sample type used to determine the reference and the urine, tampon or vaginal swab sample type are the same. For example, where a urine sample is used, the reference may be determined from one or more urine samples prepared from one or more healthy individuals.

In some embodiments, the concentration of MCM5 determined may be compared to a concentration of MCM5 previously determined from a urine, tampon or vaginal swab sample obtained from the same subject. In these embodiments, the previously determined concentration of MCM5 is used as the reference. This can be beneficial in monitoring the possibility of recurrence in the subject, which can in turn be beneficial in monitoring the course and/or effectiveness of a treatment of the subject.

### Biomarker Detection

The skilled person may use any suitable technique known in the art to detect the at least one biomarker or determine the concentration of the at least one biomarker. For example, the at least one biomarker may be detected or the concentration of the at least one biomarker may be determined by interaction with a ligand or ligands, 1-D or 2-D gel-based analysis systems, liquid chromatography, combined liquid chromatography and any mass spectrometry techniques including MSMS, ICAT(R) or iTRAQ(R), agglutination tests, thin-layer chromatography, NMR spectroscopy, sandwich immunoassays, enzyme linked immunosorbent assays (ELISAs), radioimmunoassays (RAI), enzyme immunoassays (EIA), lateral flow/immunochromatographic strip tests, Western Blotting, immunoprecipitation, particle-based immunoassays including using gold, silver, or latex particles and magnetic particles or Q-dots, or any other suitable technique known in the art.

In the methods of the invention, the step of detecting MCM5 or determining the concentration of MCM5 comprises performing an ELISA assay to detect MCM5 or determine the concentration of MCM5. In an embodiment, the ELISA assay is a sandwich ELISA assay. A sandwich ELISA assay comprises steps of capturing the MCM5 to be detected or whose concentration is to be determined using a "*capture antibody*" already bound to a plate, and detecting how much MCM5 has been captured using a "*detection antibody*"*.* The detection antibody may be pre-conjugated to a label such as the enzyme HRP (Horse Radish Peroxidase). The ELISA plate may then be exposed to the labelled detection antibody, such that the labelled detection antibody binds to the captured MCM5. After exposure to the labelled detection antibody, the ELISA plate should then be washed to remove any excess unbound labelled detection antibody. The washed plate can then be exposed to an agent whose properties are changed by the label (of the detection antibody) in a measurable manner. The concentration of the detection antibody may then be determined. For example, if the detection antibody is labelled by e.g. conjugation to HRP, the ELISA plate may be exposed to 3,3',5,5'-Tetramethylbenzidine (TMB) substrate. The concentration of the detection antibody, and therefore the concentration of MCM5 in the original urine, tampon or vaginal swab sample, may then be determined by quantitation of the colour change corresponding to the conversion of TMB into a coloured product.

The ELISA assay used in the present invention may be a qualitative ELISA assay. In the context of the present invention, a qualitative ELISA assay is one that is performed without determining a numerical value for the concentration of MCM5 in the urine, tampon or vaginal swab sample and/or without determining, or using a previously determined, numerical value for the concentration of MCM5 as a reference. For example, in a qualitative ELISA assay, the intensity of a coloured product (e.g. a coloured product produced by an enzyme linked to a detection antibody) may be compared to a reference. In this embodiment, the intensity of the coloured product is indicative of the concentration of MCM5 without the need to determine a numerical value for the concentration of MCM5. The reference used in a qualitative ELISA of this type may be a previously determined threshold intensity of the coloured product.

According to the present invention an ELISA assay is used to detect MCM5 or measure the concentration of MCM5 and, in some embodiments, the gynaecological cancer is likely to be present if the concentration of MCM5 is higher than about 5 pg/mL, 6 pg/mL, 7 pg/mL, 8 pg/mL, 9 pg/mL, 10 pg/mL, 11 pg/mL, 12 pg/mL, 13 pg/mL, 14 pg/mL, 15 pg/mL, 16 pg/mL, 17 pg/mL, 18 pg/mL, 19 pg/mL, 20 pg/mL, 25 pg/mL, 30 pg/mL, 35 pg/mL, 45 pg/mL, 50 pg/mL, 55 pg/mL, 60 pg/mL, 65 pg/mL, or 70 pg/mL, optionally about 12 pg/mL. In one embodiment, the gynaecological cancer is likely to be present if the concentration of MCM5 is higher than about 12 pg/mL. In one embodiment, the gynaecological cancer is likely to be present if the concentration of MCM5 is higher than about 17 pg/mL. In one embodiment, the gynaecological cancer is likely to be present if the concentration of MCM5 is higher than about 70 pg/mL.

In one embodiment, where the non-invasive sample is a urine sample and an ELISA assay is used to detect the at least one biomarker or measure the concentration of the at least one biomarker, the gynaecological cancer is likely to be present if the concentration of the at least one biomarker is higher than about 12 pg/mL. In one embodiment, where the non-invasive sample is a urine sample and an ELISA assay is used to detect the at least one biomarker or measure the concentration of the at least one biomarker, the gynaecological cancer is likely to be present if the concentration of the at least one biomarker is higher than about 70 pg/mL.

In one embodiment, where the non-invasive sample is a swab sample or a tampon sample and an ELISA assay is used to detect the at least one biomarker or measure the concentration of the at least one biomarker, the gynaecological cancer is likely to be present if the concentration of the at least one biomarker is higher than about 12 pg/mL. In one embodiment, where the non-invasive sample is a swab sample or a tampon sample and an ELISA assay is used to detect the at least one biomarker or measure the concentration of the at least one biomarker, the gynaecological cancer is likely to be present if the concentration of the at least one biomarker is higher than about 17 pg/mL.

In one embodiment, where the non-invasive sample is a swab sample or a tampon sample and an ELISA assay is used to detect the at least one biomarker or measure the concentration of the at least one biomarker, the gynaecological cancer is likely to be present if the concentration of the at least one biomarker is higher than about 70 pg/mL. In one embodiment, where the non-invasive sample is a tampon sample and an ELISA assay is used to detect the at least one biomarker or measure the concentration of the at least one biomarker, the gynaecological cancer is likely to be present if the concentration of the at least one biomarker is higher than about 17 pg/mL. In one embodiment, where the non-invasive sample is a tampon sample and an ELISA assay is used to detect the at least one biomarker or measure the concentration of the at least one biomarker, the gynaecological cancer is likely to be present if the concentration of the at least one biomarker is higher than about 70 pg/mL.

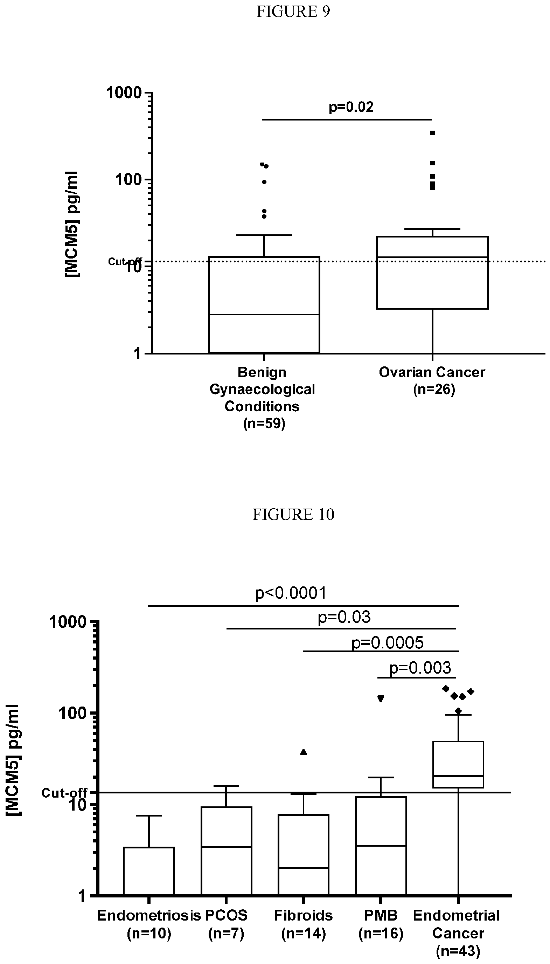

Where the at least one biomarker is MCM5 and the non-invasive sample is a urine sample and the assay to detect the at least one biomarker is an ELISA assay, it is particularly preferred to use a reference of about 12 pg/mL. It has been found that using a 12 pg/mL reference allows gynaecological cancers (or samples associated with gynaecological cancers) to be detected with high specificity and sensitivity. Furthermore, as discussed in Example 5 below, the inventors have observed that urine samples from patients with benign gynaecological conditions have statistically significantly lower MCM5 levels when compared to the MCM5 levels in urine samples from patients with a gynaecological cancer. In particular, the inventors have found that almost all of the urine samples obtained from subjects having benign gynaecological conditions had a concentration of MCM5 less than 12 pg/mL - see Figures 8 and 10 (horizontal cut-offline at 12 pg/mL). In contrast however, a significant majority of the urine samples obtained from endometrial cancer patients had an MCM5 concentration higher than 12 pg/mL - see Figures 8 and 10. Thus, using a 12 pg/mL reference allows gynaecological cancer (or samples associated with gynaecological cancer) to be accurately distinguished from benign gynaecological conditions (or samples associated with benign gynaecological conditions), such as endometriosis, polycystic ovary syndrome (PCOS), fibroids, post-menopausal bleeding (PMB).

According to the present invention, the at least one biomarker is MCM5 protein, and the step of determining the concentration of the MCM5 protein comprises performing an ELISA assay. In some embodiments, the step of determining the concentration of the MCM5 protein comprises performing a sandwich ELISA assay.

In some embodiments, the ELISA assay or sandwich ELISA assay comprises the use of one or more of the monoclonal antibodies described herein. In one embodiment, the ELISA assay or sandwich ELISA assay comprises exposing the non-invasive sample to a first monoclonal antibody described herein. In one embodiment, the ELISA assay or sandwich ELISA assay comprises exposing the non-invasive sample to a second monoclonal antibody described herein. In one embodiment, the ELISA assay or sandwich ELISA assay comprises exposing the non-invasive sample to first monoclonal antibody described herein and/or second monoclonal antibody described herein. In one embodiment, the ELISA assay or sandwich ELISA assay comprises exposing the non-invasive sample to first monoclonal antibody described herein and second monoclonal antibody described herein. In some embodiments, the first monoclonal antibody and/or the second monoclonal antibody used in the ELISA assay or sandwich ELISA assay bind specifically to MCM5.

In some embodiments, the first monoclonal antibody used in the ELISA assay or sandwich ELISA assay binds to SEQ ID NO: 1. In some embodiments, the second monoclonal antibody used in the ELISA assay or sandwich ELISA assay binds to SEQ ID NO: 2. In some embodiments, the first monoclonal antibody used in the ELISA assay or sandwich ELISA assay binds to SEQ ID NO: 1 and the second monoclonal antibody used in the ELISA assay or sandwich ELISA assay binds to SEQ ID NO: 2.

In some embodiment, the first monoclonal antibody used in the ELISA assay or sandwich ELISA comprises at least one of the CDRs of 12A7 described herein. In some embodiments, the second monoclonal antibody used in the ELISA assay or sandwich ELISA assay comprises at least one of the CDRs of 4B4 described herein. In some embodiments, the first monoclonal antibody used in the ELISA assay or sandwich ELISA assay comprises at least one of the CDRs of 12A7 described herein and the second monoclonal antibody used in the ELISA assay or sandwich ELISA assay comprises at least one of the CDRs of 4B4 described herein.

In some embodiments, the first monoclonal antibody used in the ELISA assay or sandwich ELISA is a monoclonal antibody described herein which comprises at least one of the CDRs of 12A7. In some embodiments, the second monoclonal antibody used in the ELISA assay or sandwich ELISA assay is a monoclonal antibody described herein which comprises at least one of the CDRs of 4B4. In some embodiments, the first monoclonal antibody used in the ELISA assay or sandwich ELISA assay is a monoclonal antibody described herein which comprises at least one of the CDRs of 12A7 and the second monoclonal antibody used in the ELISA assay or sandwich ELISA assay is a monoclonal antibody described herein which comprises at least one of the CDRs of 4B4.

In some embodiments, the first monoclonal antibody used in the ELISA assay or sandwich ELISA is a monoclonal antibody described herein which comprises at least one of the CDRs of 12A7 and binds to SEQ ID NO: 1. In some embodiments, the second monoclonal antibody used in the ELISA assay or sandwich ELISA assay is a monoclonal antibody described herein which comprises at least one of the CDRs of 4B4 and binds to SEQ ID NO: 2. In some embodiments, the first monoclonal antibody used in the ELISA assay or sandwich ELISA assay is a monoclonal antibody described herein which comprises at least one of the CDRs of 12A7 and binds to SEQ ID NO: 1 and the second monoclonal antibody used in the ELISA assay or sandwich ELISA assay is a monoclonal antibody which comprises at least one of the CDRs of 4B4 and binds to SEQ ID NO: 2.

In some embodiments, the first monoclonal antibody used in the ELISA assay or sandwich ELISA is 12A7. In some embodiments, the second monoclonal antibody used in the ELISA assay or sandwich ELISA assay is 4B4. In some embodiments, the first monoclonal antibody used in the ELISA assay or sandwich ELISA assay is 12A7 and the second monoclonal antibody used in the ELISA assay or sandwich ELISA assay is 4B4.

### Detection of Gynaecological Cancer

The present methods are for "*detecting the presence or absence of a gynaecological cancer in a subject*"*.* In one embodiment, the subject is a female human. In one embodiment, the subject is a human having one or more components of the female reproductive system. For example, in one embodiment, the subject is a human having one or more ovaries, one or more fallopian tubes, an endometrium, a uterus, a cervix, a vulva, a vagina, and/or a placenta.

In some embodiments, the subject under the age of 18, 17, 16, 15, 14, 13, 12, 11, 10, 9, 8, 7, 6, 5, 4, 3, 2, or 1 year(s). In some embodiments, the subject is pre-pubescent. In some embodiments, the subject is an adult. In some embodiments, the subject is menopausal. In some embodiments, the subject is post-menopausal. In some embodiments, the subject is elderly, for example at least 65, 70, 75, 80, 85, or 90 years in age.

In some embodiments, the subject is suffering from a gynaecological cancer. In some embodiments, the subject is in remission from a gynaecological cancer. In some embodiments, the subject is suspected of having a gynaecological cancer. In some embodiments, the subject is healthy. In some embodiments, the subject is at risk of developing a gynaecological cancer. In some embodiments, the subject has an increased risk of developing a gynaecological cancer. The subject may be at risk or have an increased risk of developing a gynaecological cancer where the subject has been previously identified as carrying a genetic marker indicating an increased risk of developing a gynaecological cancer. The subject may be at risk or have an increased risk of developing a gynaecological cancer where the subject has a family history of gynaecological cancer. The subject may be at risk or have an increased risk of developing a gynaecological cancer where the subject is suffering from, or has previously been diagnosed with a cancer, for example, a non-gynaecological cancer.

In some embodiments, the subject is suffering from a disease selected from the group consisting of post-menopausal bleeding, endometriosis, fibroids, and polycystic ovary syndrome. In some embodiments, the subject has been previously diagnosed with disease selected from the group consisting of post-menopausal bleeding, endometriosis, fibroids, and polycystic ovary syndrome.

In any of the methods or uses of the present invention, the subject may be suffering from post-menopausal bleeding (PMB). In any of the methods or uses of the present invention, the subject may be suffering from endometriosis. In any of the methods or uses of the present invention the subject may be suffering from fibroids. In any of the methods or uses of the present invention, the subject may be suffering from polycystic ovary syndrome (PCOS).

In some embodiments, the subject is at risk of developing a disease selected from the group consisting of post-menopausal bleeding, endometriosis, fibroids, and polycystic ovary syndrome. In some embodiments, the subject has an increased risk of developing a disease selected from the group consisting of post-menopausal bleeding, endometriosis, fibroids, and polycystic ovary syndrome. The subject may be at risk or have an increased risk of developing post-menopausal bleeding, endometriosis, fibroids, and polycystic ovary syndrome where the subject has been previously identified as carrying a genetic marker indicating an increased risk of developing a post-menopausal bleeding, endometriosis, fibroids, or polycystic ovary syndrome, respectively. The subject may be at risk or have an increased risk of developing post-menopausal bleeding, endometriosis, fibroids, or polycystic ovary syndrome where the subject has a family history of post-menopausal bleeding, endometriosis, fibroids, and polycystic ovary syndrome, respectively. The subject may be at risk or have an increased risk of developing post-menopausal bleeding, endometriosis, fibroids, and polycystic ovary syndrome where the subject is suffering from or has previously been diagnosed with a disease or condition related to post-menopausal bleeding, endometriosis, fibroids, and polycystic ovary syndrome, respectively.

Collectively, post-menopausal bleeding (PMB), endometriosis, fibroids, and polycystic ovary syndrome (PCOS) may be referred to as benign gynaecological conditions. Such conditions are referred to as benign as, although they may be medically serious, the conditions are not malignant because said conditions do not have the capacity to spread to, or invade, other tissues. Thus, in any of the methods and use described herein, the subject may be suffering from a benign gynaecological condition, optionally selected from post-menopausal bleeding (PMB), endometriosis, fibroids, and polycystic ovary syndrome (PCOS). The subject may have been previously diagnosed with a benign gynaecological condition, optionally selected from post-menopausal bleeding (PMB), endometriosis, fibroids, and polycystic ovary syndrome (PCOS). The subject may be at risk or have an increased risk of developing of developing a benign gynaecological condition, optionally selected from post-menopausal bleeding (PMB), endometriosis, fibroids, and polycystic ovary syndrome (PCOS). The subject may be at risk or have an increased risk of developing of developing a benign gynaecological condition, optionally selected from post-menopausal bleeding (PMB), endometriosis, fibroids, and polycystic ovary syndrome (PCOS), if the subject has been previously identified as carrying a genetic marker indicating an increased risk of developing the benign gynaecological condition, and/or if the subject has a family history of the benign gynaecological condition, and/or if the subject is suffering from or has previously been diagnosed with a disease or condition related to the benign gynaecological condition.

In other embodiments, the subject is a female non-human mammal or a non-human mammal having one or more components of the female reproductive system common to the said non-human mammal. The non-human mammal may be a primate, feline, canine, bovine, equine, murine, ovine, or porcine.

Typically, it is not possible for a diagnostic method to predict with 100 % accuracy whether or a cancer is present in a subject. Thus, the wording "*detecting the presence or absence of a gynaecological cancer in a subject*" can be understood to refer to determining whether a gynaecological cancer is likely to be present or absent in a subject.

In some embodiments, a gynaecological cancer is likely to be present if the concentration of MCM5 is abnormal compared to the reference. The concentration of MCM5 may be said to be abnormal compared to the reference if there is a statistically significant difference between the concentration of MCM5 and the reference. For example, the concentration of MCM5 is abnormal if it is significantly lower than the reference. Alternatively, the concentration of MCM5 is abnormal if it is significantly higher than the reference. Thus, in one embodiment of the invention, a gynaecological cancer is likely to be present if the concentration of MCM5 is higher than the reference. For example, where the reference is an average concentration of MCM5, a gynaecological cancer is likely to be present when the concentration of MCM5 is higher than the reference by at least one, at least two, at least three, or at least four standard deviations.

In one embodiment, a gynaecological cancer is likely to be present if the concentration of MCM5 is at least 1.05 times, at least 1.10 times, at least 1.15 time, at least 1.2 times, at least 1.3 times, at least 1.4 times, at least 1.5 time, at least 1.6 time, at least on 1.7 time, at least 1.8 times, at least 1.9 times, at least 2.0 times, at least 2.1 times at least 2.2 times, at least 2.3 times, at least 2.5 times, at least 3 times, at least 3.5 times, at least 4 times, at least 5 times, at least 10 times, at least 25 times, at least 50 times, at least 75 time, or at least 100 times, higher than the reference.

In some embodiments, a gynaecological cancer is likely to be absent if the concentration of t MCM5 is normal compared to the reference. The concentration of MCM5 may be said to be normal compared to the reference if there is no statistically significant difference between the concentration of MCM5 and the reference. For example, the concentration of MCM5 may be said to be normal compared to the reference if the difference between the concentration of MCM5 and the reference is less than two, or less than one standard deviations.

In some embodiments, the method of the invention is a method for diagnosing a gynaecological cancer in a subject. In such embodiments, the subject may be diagnosed as having a gynaecological cancer if the concentration of MCM5 determined for the non-invasive sample is significantly higher than the mean value determined for healthy individuals. In such embodiments, the "*mean value determined for healthy individuals*" is a type of "*reference*" as defined herein. It should be understood that the various embodiments and characteristics of a "*reference*" as defined herein may also apply to the "*mean value determined for healthy individuals*"*.* In some embodiments, the concentration of MCM5 is higher than the mean value determined for healthy individuals if it is higher than the mean value determined for healthy individuals plus a multiple of the standard deviation of the mean value determined for healthy individuals, for example, higher than the mean value plus one, two, three, four, or five standard deviations of the mean value determined for healthy individuals. Alternatively, the subject may be diagnosed as not having a gynaecological cancer if the concentration of MCM5 is not significantly different to the mean value determined for healthy individuals.

### Sensitivity and Specificity

The method of the present invention allow gynaecological cancers to be detected with high specificity and high sensitivity. In the field of medical diagnostics and as used herein the term "*sensitivity*" (also referred to as the true positive rate) refers to a measure of the proportion of actual positives that are correctly identified as such. In other words, the sensitivity of a diagnostic test may be expressed as the number of true positives *i.e.* individuals correctly identified as having a disease as a proportion of all the individuals having the disease in the test population (*i.e.* the sum of true positive and false negative outcomes). Thus, a high sensitivity diagnostic test is desirable as it rarely misidentifies individuals having the disease. This means that a negative result obtained by a highly sensitive test has a high likelihood of ruling out the disease.

In the field of medical diagnostics, and as used herein, the term "*specificity*" (also referred to as the true negative rate) refers to a measure of the proportion of actual negatives that are correctly identified as such. In other words, the specificity of a diagnostic test may be expressed as the number of true negatives *(i.e.* healthy individuals correctly identified as not having a disease) as a proportion of all the healthy individuals in the test population *(i.e.* the sum of true negative and false positive outcomes). Thus, a high specificity diagnostic test is desirable as it rarely misidentifies healthy individuals. This means that a positive result obtained by a highly specific test has a high likelihood of ruling in the disease.

In the field of medical diagnostics, and as used herein, the term "*positive predictive value (PPV)*" refers to the proportion of all positive outcomes generated by diagnostic test that are true positive outcomes. Put another way, PPV can be defined as the number of true positive outcomes divided by the sum of true positive outcomes and false positive outcomes. In the field of medical diagnostics, and as used herein, the term "*negative predictive value (NPV)*" refers to the proportion of all negatives outcomes generated by diagnostic test that are true negative outcomes. Put another way, NPV can be defined as the number of true negative outcomes divided by the sum of true negative outcomes and false negative outcomes.

In the field of medical diagnostics, and as used herein, a "*Receiver Operating Characteristic (ROC) curve*" refers to a plot of true positive rate (sensitivity) against the false positive rate (1 - specificity) for all possible cut-off values. In the field of medical diagnostics, and as used herein, a "*Youdens index*" refers to the cut-off point at which the distance between the ROC curve and the line of chance (45 degree line) is highest. These terms are well known in the art and to the skilled person. The specificity and/or sensitivity of a method may be determined by performing said method on samples which are known to be positive samples (e.g. samples from patients having a gynaecological cancer) and/or samples which are known to be negative samples (e.g. samples from healthy individuals). The extent to which the method correctly identifies the known positive samples *(i.e.* the sensitivity / true positive rate of the method) and/or the known negative samples (*i.e.* the specificity / true negative rate of the method) can thus be determined.

In some embodiments of the methods of the present invention, the method has a sensitivity for the gynaecological cancer of at least about 40 %, 42 %, 44 %, 46 %, 48 %, or 50 %. In some embodiments of the methods of the present invention, the non-invasive sample is a tampon sample, and the method has a sensitivity for the gynaecological cancer of at least about 40 %, 42 %, 44 %, 46 %, 48 %, 50 %.

In some embodiments of the methods of the present invention, the method has a sensitivity for the gynaecological cancer of at least about 50 %, 52 %, 54 %, 55 %, 56 %, 57 %, 58 %, 59 %, 60 %, or 61%. In some embodiments of the methods of the present invention, the non-invasive sample is a vaginal swab sample, and the method has a sensitivity for the gynaecological cancer of at least about 50 %, 52 %, 54 %, 55 %, 56 %, 57 %, 58 %, 59 %, 60 %, or 61 %.

In some embodiments of the methods of the present invention, the method has a sensitivity for the gynaecological cancer of at least about 75 %, 76 %, 77 %, 78 %, 79 %, 80 %, or 81 %. In some embodiments of the methods of the present invention, the non-invasive sample is a urine sample, and the method has a sensitivity for the gynaecological cancer of at least about 75 %, 76 %, 77 %, 78 %, 79 %, 80%, or 81 %.

In some embodiments of the methods of the present invention, the method has a sensitivity for the gynaecological cancer of at least about 90 %, 92 %, 93 %, 94 %, 95 %, or 96%. In some embodiments of the methods of the present invention, the non-invasive sample is a tampon sample, and the method has a sensitivity for the gynaecological cancer of at least about 90 %, 92 %, 93 %, 94 %, 95 %, or 96 %.

In some embodiments of the methods of the present invention, the method has a specificity for the gynaecological cancer of at least about 40 %, 41 %, 42 %, or 43 %. In some embodiments of the methods of the present invention, the non-invasive sample is a tampon sample, and the method has a specificity for the gynaecological cancer of at least about 40 %, 41 %, 42 %, or 43 %.

In some embodiments of the methods of the present invention, the method has a specificity for the gynaecological cancer of at least about 50 %, 52 %, 54 %, 55 %, 56 %, 57 %, 58 %, 59 %, or 60 %. In some embodiments of the methods of the present invention, the non-invasive sample is a urine sample, and the method has a specificity for the gynaecological cancer of at least about 50 %, 52 %, 54 %, 55 %, 56 %, 57%, 58 %, 59 %, or 60 %

In some embodiments of the methods of the present invention, the method has a specificity for the gynaecological cancer of at least about 60 %, 62 %, 64 %, 66 %, 68 %, 70 %, 71 %, 72 %, 73 %, 74 %, or 75 %. In some embodiments of the methods of the present invention, the non-invasive sample is a vaginal swab sample, and the method has a specificity for the gynaecological cancer of at least about 60 %, 62 %, 64 %, 66 %, 68 %, 70 %, 71%, 72 %, 73 %, 74 %, or 75 %.

In some embodiments of the methods of the present invention, the method has a specificity for the gynaecological cancer of at least about 80 %, 82 %, 83 %, 84 %, 85 %, or 86 %. In some embodiments of the methods of the present invention, the non-invasive sample is a tampon sample, and the method has a specificity for the gynaecological cancer of at least about 80%, 82 %, 83 %, 84 %, 85 %, or 86 %.

In some embodiments of the methods of the present invention, the non-invasive sample is a urine sample, and the method has a sensitivity for the ovarian cancer of at least about 60 %. In some embodiments of the methods of the present invention, the non-invasive sample is a urine sample, and the method has a sensitivity for the ovarian cancer of at least about 60 % when a 12 pg/mL cut-off is applied.

In some embodiments of the methods of the present invention, the non-invasive sample is a urine sample, and the method has a specificity for the ovarian cancer of at least about 65 %. In some embodiments of the methods of the present invention, the non-invasive sample is a urine sample, and the method has a specificity for the ovarian cancer of at least about 65 % when a 12 pg/mL cut-off is applied.

In some embodiments of the methods of the present invention, the non-invasive sample is a urine sample, and the method has a sensitivity for the ovarian cancer of at least about 60 % and a specificity for the ovarian cancer of at least about 65 %. In some embodiments of the methods of the present invention, the non-invasive sample is a urine sample, and the method has a sensitivity for the ovarian cancer of at least about 60 % and a specificity for the ovarian cancer of at least about 65 % when a 12 pg/mL cut-off is applied.

In some embodiments of the methods of the present invention, the non-invasive sample is a urine sample, and the method has a sensitivity for the ovarian cancer of at least about 60 %, a specificity for the ovarian cancer of at least about 65 %, a positive predictive value (PPV) of at least about 15 %, and negative predictive value (NPV) of at least about 90 %. In some embodiments of the methods of the present invention, the non-invasive sample is a urine sample, and the method has a sensitivity for the ovarian cancer of at least about 60 %, a specificity for the ovarian cancer of at least about 65 %, a positive predictive value (PPV) of at least about 15 %, and negative predictive value (NPV) of at least about 90 % when a 12 pg/mL cut-off is applied.

In some embodiments of the methods of the present invention, the non-invasive sample is a urine sample, and the method has a sensitivity for the endometrial cancer of at least about 80 %. In some embodiments of the methods of the present invention, the non-invasive sample is a urine sample, and the method has a sensitivity for the endometrial cancer of at least about 80 % when a 12 pg/mL cut-off is applied.

In some embodiments of the methods of the present invention, the non-invasive sample is a urine sample, and the method has a specificity for the endometrial cancer of at least about 65 %. In some embodiments of the methods of the present invention, the non-invasive sample is a urine sample, and the method has a specificity for the endometrial cancer of at least about 65 % when a 12 pg/mL cut-off is applied.

In some embodiments of the methods of the present invention, the non-invasive sample is a urine sample, and the method has a sensitivity for the endometrial cancer of at least about 80 % and a specificity for the endometrial cancer of at least about 65 %. In some embodiments of the methods of the present invention, the non-invasive sample is a urine sample, and the method has a sensitivity for the endometrial cancer of at least about 80 % and a specificity for the endometrial cancer of at least about 65 % when a 12 pg/mL cut-off is applied.

In some embodiments of the methods of the present invention, the non-invasive sample is a urine sample, and the method has a sensitivity for the endometrial cancer of at least about 80 %, a specificity for the endometrial cancer of at least about 65 %, a positive predictive value (PPV) of at least about 35 %, and negative predictive value (NPV) of at least about 90 %. In some embodiments of the methods of the present invention, the non-invasive sample is a urine sample, and the method has a sensitivity for the endometrial cancer of at least about 80 %, a specificity for the endometrial cancer of at least about 65 %, a positive predictive value (PPV) of at least about 35 %, and negative predictive value (NPV) of at least about 90 % when a 12 pg/mL cut-off is applied.

In some embodiments of the methods of the present invention, the non-invasive sample is a tampon sample, and the method has a sensitivity for the ovarian cancer of at least about 55 %. In some embodiments of the methods of the present invention, the non-invasive sample is a tampon sample, and the method has a sensitivity for the ovarian cancer of at least about 55 % when a 70 pg/mL cut-off is applied. In some embodiments of the methods of the present invention, the non-invasive sample is a tampon sample, and the method has a specificity for the ovarian cancer of at least about 75 %. In some embodiments of the methods of the present invention, the non-invasive sample is a tampon sample, and the method has a specificity for the ovarian cancer of at least about 75 % when a 70 pg/mL cut-off is applied. In some embodiments of the methods of the present invention, the non-invasive sample is a tampon sample, and the method has a sensitivity for the ovarian cancer of at least about 55 % and a specificity for the ovarian cancer of at least about 75 %. In some embodiments of the methods of the present invention, the non-invasive sample is a tampon sample, and the method has a sensitivity for the ovarian cancer of at least about 55 % and a specificity for the ovarian cancer of at least about 75 % when a 70 pg/mL cut-off is applied.

In some embodiments of the methods of the present invention, the non-invasive sample is a tampon sample, and the method has a sensitivity for the ovarian cancer of at least about 80 %. In some embodiments of the methods of the present invention, the non-invasive sample is a tampon sample, and the method has a sensitivity for the ovarian cancer of at least about 80 % when a 12 pg/mL cut-off is applied. In some embodiments of the methods of the present invention, the non-invasive sample is a tampon sample, and the method has a specificity for the ovarian cancer of at least about 20 %. In some embodiments of the methods of the present invention, the non-invasive sample is a tampon sample, and the method has a specificity for the ovarian cancer of at least about 20 % when a 12 pg/mL cut-off is applied. In some embodiments of the methods of the present invention, the non-invasive sample is a tampon sample, and the method has a sensitivity for the ovarian cancer of at least about 80 % and a specificity for the ovarian cancer of at least about 20 %. In some embodiments of the methods of the present invention, the non-invasive sample is a tampon sample, and the method has a sensitivity for the ovarian cancer of at least about 80 % and a specificity for the ovarian cancer of at least about 20 % when a 12 pg/mL cut-off is applied.

In some embodiments of the methods of the present invention, the non-invasive sample is a tampon sample, and the method has a sensitivity for the endometrial cancer of at least about 45 %. In some embodiments of the methods of the present invention, the non-invasive sample is a tampon sample, and the method has a sensitivity for the endometrial cancer of at least about 45 % when a 70 pg/mLcut-off is applied. In some embodiments of the methods of the present invention, the non-invasive sample is a tampon sample, and the method has a specificity for the endometrial cancer of at least about 75 %. In some embodiments of the methods of the present invention, the non-invasive sample is a tampon sample, and the method has a specificity for the endometrial cancer of at least about 75 % when a 70 pg/mL cut-off is applied. In some embodiments of the methods of the present invention, the non-invasive sample is a tampon sample, and the method has a sensitivity for the endometrial cancer of at least about 45 % and a specificity for the endometrial cancer of at least about 75 %. In some embodiments of the methods of the present invention, the non-invasive sample is a tampon sample, and the method has a sensitivity for the endometrial cancer of at least about 45 % and a specificity for the endometrial cancer of at least about 75 % when a 70 pg/mL cut-off is applied.

In some embodiments of the methods of the present invention, the non-invasive sample is a tampon sample, and the method has a sensitivity for the endometrial cancer of at least about 95 %. In some embodiments of the methods of the present invention, the non-invasive sample is a tampon sample, and the method has a sensitivity for the endometrial cancer of at least about 95 % when a 17 pg/mL cut-off is applied. In some embodiments of the methods of the present invention, the non-invasive sample is a tampon sample, and the method has a specificity for the endometrial cancer of at least about 20 %. In some embodiments of the methods of the present invention, the non-invasive sample is a tampon sample, and the method has a specificity for the endometrial cancer of at least about 20 % when a 17 pg/mL cut-off is applied. In some embodiments of the methods of the present invention, the non-invasive sample is a tampon sample, and the method has a sensitivity for the endometrial cancer of at least about 95 % and a specificity for the endometrial cancer of at least about 20 %. In some embodiments of the methods of the present invention, the non-invasive sample is a tampon sample, and the method has a sensitivity for the endometrial cancer of at least about 95 % and a specificity for the endometrial cancer of at least about 20 % when a 17 pg/mL cut-off is applied.

In some embodiments of the methods of the present invention, the method has a sensitivity for the gynaecological cancer of at least about 81 % and a specificity for the gynaecological cancer of at least about 60 %. In some embodiments of the methods of the present invention, the non-invasive sample is a urine sample and the method has a sensitivity for the gynaecological cancer of at least about 81 % and a specificity for the gynaecological cancer of at least about 60 %.

In some embodiments of the methods of the present invention, the method has a sensitivity for the gynaecological cancer of at least about 61 % and a specificity for the gynaecological cancer of at least about 75 %. In some embodiments of the methods of the present invention, the non-invasive sample is a vaginal swab sample and the method has a sensitivity for the gynaecological cancer of at least about 61 % and a specificity for the gynaecological cancer of at least about 75 %.

In some embodiments of the methods of the present invention, the method has a sensitivity for the gynaecological cancer of at least about 96 % and a specificity for the gynaecological cancer of at least about 43 %. In some embodiments of the methods of the present invention, the non-invasive sample is a tampon sample and the method has a sensitivity for the gynaecological cancer of at least about 96 % and a specificity for the gynaecological cancer of at least about 43 %.

In some embodiments of the methods of the present invention, the method has a sensitivity for the gynaecological cancer of at least about 50 % and a specificity for the gynaecological cancer of at least about 86 %. In some embodiments of the methods of the present invention, the non-invasive sample is a tampon sample and the method has a sensitivity for the gynaecological cancer of at least about 50 % and a specificity for the gynaecological cancer of at least about 86 %.

In some embodiments of the methods of the present invention, the method has a sensitivity for the gynaecological cancer of at least about 96 % and a specificity for the gynaecological cancer of at least about 86 %. In some embodiments of the methods of the present invention, the non-invasive sample is a tampon sample and the method has a sensitivity for the gynaecological cancer of at least about 96 % and a specificity for the gynaecological cancer of at least about 86 %.

In some embodiments of the methods of the present invention, the method has a sensitivity for the endometrial cancer of at least about 40 %, 42 %, 44 %, 46 %, 48 %, or 50 %. In some embodiments of the methods of the present invention, the non-invasive sample is a tampon sample, and the method has a sensitivity for the endometrial cancer of at least about 40 %, 42 %, 44 %, 46 %, 48 %, 50 %.

In some embodiments of the methods of the present invention, the method has a sensitivity for the endometrial cancer of at least about 60 %, 65 %, 70%, 71 %, 72 %, 73 %, or 74 %. In some embodiments of the methods of the present invention, the non-invasive sample is a vaginal swab sample, and the method has a sensitivity for the endometrial cancer of at least about 60 %, 65 %, 70%, 71 %, 72 %, 73 %, or 74 %.

In some embodiments of the methods of the present invention, the method has a sensitivity for the endometrial cancer of at least about 78 %, 80 %, 81 %, 82 %, 83 %, 84 %, 85 %, 86 %, or 87 %. In some embodiments of the methods of the present invention, the non-invasive sample is a urine sample, and the method has a sensitivity for the endometrial cancer of at least about 78 %, 80 %, 81 %, 82 %, 83 %, 84 %, 85 %, 86 %, or 87 %.

In some embodiments of the methods of the present invention, the method has a sensitivity for the endometrial cancer of at least about 90 %, 92 %, 93 %, 94 %, 95 %, 96 %, 97 %, 98 %, 99 %, or 100 %. In some embodiments of the methods of the present invention, the non-invasive sample is a tampon sample, and the method has a sensitivity for the endometrial cancer of at least about 90 %, 92 %, 93 %, 94 %, 95 %, 96 %, 97 %, 98 %, 99 %, or 100 %.

In some embodiments of the methods of the present invention, the method has a specificity for the endometrial cancer of at least about 40 %, 41 %, 42 %, or 43 %. In some embodiments of the methods of the present invention, the non-invasive sample is a tampon sample, and the method has a specificity for the endometrial cancer of at least about 40 %, 41 %, 42 %, or 43 %.

In some embodiments of the methods of the present invention, the method has a specificity for the endometrial cancer of at least about 50 %, 52 %, 54 %, 55 %, 56 %, 57 %, 58 %, 59 %, 60 %, 62 %, 64 %, 66 %, 68 %, 70 %, 71 %, 72 %, 73 %, or 74 %. In some embodiments of the methods of the present invention, the non-invasive sample is a urine sample, and the method has a specificity for the endometrial cancer of at least about 50 %, 52 %, 54 %, 55 %, 56 %, 57 %, 58 %, 59 %, or 60 %, 62 %, 64 %, 66 %, 68 %, 70 %, 71 %, 72 %, 73 %, or 74 %.

In some embodiments of the methods of the present invention, the method has a specificity for the endometrial cancer of at least about 60 %, 62 %, 64 %, 66 %, 68 %, 70 %, 71 %, 72 %, 73 %, 74 %, or 75 %. In some embodiments of the methods of the present invention, the non-invasive sample is a vaginal swab sample, and the method has a specificity for the endometrial cancer of at least about 60 %, 62 %, 64 %, 66 %, 68 %, 70 %, 71 %, 72 %, 73 %, 74 %, or 75 %.

In some embodiments of the methods of the present invention, the method has a specificity for the endometrial cancer of at least about 80%, 82 %, 83 %, 84 %, 85 %, 86 %, or 87%. In some embodiments of the methods of the present invention, the non-invasive sample is a tampon sample, and the method has a specificity for the endometrial cancer of at least about 80%, 82 %, 83 %, 84 %, 85 %, 86 %, or 87 %.

In some embodiments of the methods of the present invention, the method has a sensitivity for the endometrial cancer of at least about 87 % and a specificity for the endometrial cancer of at least about 60 %. In some embodiments of the methods of the present invention, the non-invasive sample is a urine sample and the method has a sensitivity for the endometrial cancer of at least about 87 % and a specificity for the endometrial cancer of at least about 60 %.

In some embodiments of the methods of the present invention, the method has a sensitivity for the endometrial cancer of at least about 85 % and a specificity for the endometrial cancer of at least about 70 %. In some embodiments of the methods of the present invention, the non-invasive sample is a urine sample and the method has a sensitivity for the endometrial cancer of at least about 85 % and a specificity for the endometrial cancer of at least about 70 %.

In some embodiments of the methods of the present invention, the method has a sensitivity for the endometrial cancer of at least about 74 % and a specificity for the endometrial cancer of at least about 75 %. In some embodiments of the methods of the present invention, the non-invasive sample is a vaginal swab sample and the method has a sensitivity for the endometrial cancer of at least about 74 % and a specificity for the endometrial cancer of at least about 75 %.

In some embodiments of the methods of the present invention, the method has a sensitivity for the endometrial cancer of at least about 100 % and a specificity for the endometrial cancer of at least about 43 %. In some embodiments of the methods of the present invention, the non-invasive sample is a tampon sample and the method has a sensitivity for the endometrial cancer of at least about 100 % and a specificity for the endometrial cancer of at least about 43 %.

In some embodiments of the methods of the present invention, the method has a sensitivity for the endometrial cancer of at least about 50 % and a specificity for the endometrial cancer of at least about 86 %. In some embodiments of the methods of the present invention, the non-invasive sample is a tampon sample and the method has a sensitivity for the endometrial cancer of at least about 50 % and a specificity for the endometrial cancer of at least about 86 %.

In some embodiments of the methods of the present invention, the method has a sensitivity for the endometrial cancer of at least about 100 % and a specificity for the endometrial cancer of at least about 86 %. In some embodiments of the methods of the present invention, the non-invasive sample is a tampon sample and the method has a sensitivity for the endometrial cancer of at least about 100 % and a specificity for the endometrial cancer of at least about 86 %.

In some embodiments of the methods of the present invention, the method has a sensitivity for the ovarian cancer of at least about 40 %, 42 %, 44 %, 46 %, 47 %, 48 %, 49 %, or 50 %. In some embodiments of the methods of the present invention, the non-invasive sample is a tampon sample, and the method has a sensitivity for the ovarian cancer of at least about 40 %, 42 %, 44 %, 46 %, 47 %, 48 %, 49 %, or 50 %.

In some embodiments of the methods of the present invention, the method has a sensitivity for the ovarian cancer of at least about 70 %, 75 %, 80 %, 81 %, 82 %, 83 %, 84 %, or 85 %. In some embodiments of the methods of the present invention, the non-invasive sample is a vaginal swab sample, and the method has a sensitivity for the ovarian cancer of at least about 70 %, 75 %, 80 %, 81 %, 82 %, 83 %, 84 %, or 85 %.

In some embodiments of the methods of the present invention, the method has a sensitivity for the ovarian cancer of at least about 55 %, 56 %, 57 %, 58 %, 59 %, 60 %, 60 %, 61 %, 62 %, 63 %, 64 %, or 65 %. In some embodiments of the methods of the present invention, the non-invasive sample is a urine sample, and the method has a sensitivity for the ovarian cancer of at least about 55 %, 56 %, 57 %, 58 %, 59 %, 60 %, 60 %, 61 %, 62 %, 63 %, 64 %, or 65 %.

In some embodiments of the methods of the present invention, the method has a sensitivity for the ovarian cancer of at least about 80 %, 82 %, 84 %, 85 %, 86 %, 87 %, 88 %, 89 %, or 90 %. In some embodiments of the methods of the present invention, the non-invasive sample is a tampon sample, and the method has a sensitivity for the ovarian cancer of at least about 80%, 82 %, 84 %, 85 %, 86 %, 87 %, 88 %, 89 %, or 90 %.

In some embodiments of the methods of the present invention, the method has a specificity for the ovarian cancer of at least about 35 %, 38 %, 40 %, 41 %, 42 %, or 43 %. In some embodiments of the methods of the present invention, the non-invasive sample is a tampon sample, and the method has a specificity for the ovarian cancer of at least about 35 %, 38 %, 40 %, 41 %, 42 %, or 43 %.

In some embodiments of the methods of the present invention, the method has a specificity for the ovarian cancer of at least about 50 %, 55 %, 56 %, 57 %, 58 %, 59 %, 60 %, 62 %, 64 %, 66 %, 68 %, 70 %, 71 %, 72 %, 73 %, or 74 %. In some embodiments of the methods of the present invention, the non-invasive sample is a urine sample, and the method has a specificity for the ovarian cancer of at least about 50 %, 55 %, 56 %, 57%, 58 %, 59 %, 60 %, 62 %, 64 %, 66 %, 68 %, 70 %, 71 %, 72 %, 73 %, or 74 %.

In some embodiments of the methods of the present invention, the method has a specificity for the ovarian cancer of at least about 15 %, 18 %, 20 %, 21 %, 22 %, 23 %, 24 %, or 25 %. In some embodiments of the methods of the present invention, the non-invasive sample is a vaginal swab sample, and the method has a specificity for the ovarian cancer of at least about 15 %, 18 %, 20 %, 21 %, 22 %, 23 %, 24 %, or 25 %.

In some embodiments of the methods of the present invention, the method has a specificity for the ovarian cancer of at least about 75 %, 80 %, 82 %, 83 %, 84 %, 85 %, 86 %, or 87 %. In some embodiments of the methods of the present invention, the non-invasive sample is a tampon sample, and the method has a specificity for the ovarian cancer of at least about 75 %, 80 %, 82 %, 83 %, 84 %, 85 %, 86 %, or 87 %.

In some embodiments of the methods of the present invention, the method has a sensitivity for the ovarian cancer of at least about 65 % and a specificity for the ovarian cancer of at least about 60 %. In some embodiments of the methods of the present invention, the non-invasive sample is a urine sample and the method has a sensitivity for the ovarian cancer of at least about 65 % and a specificity for the ovarian cancer of at least about 60 %.

In some embodiments of the methods of the present invention, the method has a sensitivity for the ovarian cancer of at least about 60 % and a specificity for the ovarian cancer of at least about 70 %. In some embodiments of the methods of the present invention, the non-invasive sample is a urine sample and the method has a sensitivity for the ovarian cancer of at least about 60 % and a specificity for the ovarian cancer of at least about 70 %.

In some embodiments of the methods of the present invention, the method has a sensitivity for the ovarian cancer of at least about 85 % and a specificity for the ovarian cancer of at least about 25 %. In some embodiments of the methods of the present invention, the non-invasive sample is a vaginal swab sample and the method has a sensitivity for the ovarian cancer of at least about 85 % and a specificity for the ovarian cancer of at least about 25 %.

In some embodiments of the methods of the present invention, the method has a sensitivity for the ovarian cancer of at least about 90 % and a specificity for the ovarian cancer of at least about 43 %. In some embodiments of the methods of the present invention, the non-invasive sample is a tampon sample and the method has a sensitivity for the ovarian cancer of at least about 90 % and a specificity for the ovarian cancer of at least about 43 %.

In some embodiments of the methods of the present invention, the method has a sensitivity for the ovarian cancer of at least about 50 % and a specificity for the ovarian cancer of at least about 86 %. In some embodiments of the methods of the present invention, the non-invasive sample is a tampon sample and the method has a sensitivity for the ovarian cancer of at least about 50 % and a specificity for the ovarian cancer of at least about 86 %.

In some embodiments of the methods of the present invention, the method has a sensitivity for the ovarian cancer of at least about 90 % and a specificity for the ovarian cancer of at least about 86 %. In some embodiments of the methods of the present invention, the non-invasive sample is a tampon sample and the method has a sensitivity for the ovarian cancer of at least about 90 % and a specificity for the ovarian cancer of at least about 86 %.

### Gynaecological Cancers

The present invention provides methods for detecting the presence or absence of a gynaecological cancer in a subject. A gynaecological cancer is a cancer associated with the female reproductive system, for example a cancer affecting one or more of the ovaries, fallopian tubes, endometrium, uterus, cervix, vulva, vagina, and placenta.

In some embodiments, the gynaecological cancer is an ovarian cancer. The ovarian cancer may be selected from the group consisting of epithelial ovarian tumour, ovarian teratoma, immature ovarian teratoma, stromal tumour, germ cell ovarian tumour, undifferentiated tumours, borderline ovarian tumour, mucinous tumour, granulosa cell tumour, immature cystic teratoma, serous ovarian tumour, and small cell carcinoma.

In some embodiments, the gynaecological cancer is an endometrial cancer. The endometrial cancer may be selected from the group consisting of endometrial carcinoma, endometrial adenocarcinoma, endometrioid adenocarcinoma, endometrial squamous cell carcinoma, serous carcinoma, serous endometrial intraepithelial carcinoma, endometrial carcinosarcoma, clear cell carcinoma, mucinous carcinoma, undifferentiated endometrial carcinoma, mixed endometrial carcinoma, malignant mixed Müllerian tumour, and endometrial clear cell sarcoma.

In some embodiments, the gynaecological cancer may be selected from the group consisting of ovarian cancer, endometrial cancer, uterine cancer, cervical cancer, vulval cancer, vaginal cancer, fallopian tube tumour, epithelial ovarian tumour, ovarian teratoma, immature ovarian teratoma, stromal tumour, germ cell ovarian tumour, undifferentiated tumours, borderline ovarian tumour, endometrial carcinoma, endometrial adenocarcinoma, endometrioid adenocarcinoma, endometrial squamous cell carcinoma, serous carcinoma, serous endometrial intraepithelial carcinoma, endometrial carcinosarcoma, clear cell carcinoma, mucinous carcinoma, undifferentiated endometrial carcinoma, mixed endometrial carcinoma, mucinous tumour, malignant mixed Müllerian tumour, endometrial clear cell sarcoma, granulosa cell tumour, serous tubal intraepithelial carcinoma, immature cystic teratoma, serous ovarian tumour, and small cell carcinoma.

In some embodiments, the gynaecological cancer is a gynaecological malignancy. The cells of a gynaecological malignancy may be characterised by anaplasia (e.g. poor cellular differentiation and/or the loss of morphological characteristics associated with healthy cells), invasiveness (*i.e.* the ability to spread into a healthy tissue), metastasis (*i.e.* the ability to spread from an initial site to different secondary site), and/or genome instability.

In some embodiments, the methods of the present invention may be used to detect a low grade gynaecological cancer. In some embodiments, the methods of the present invention may be used to detect a high grade gynaecological cancer. High grade gynaecological cancers may be associated with excessive cell proliferation rates, typically longer cell lifespans and poor cellular differentiation. Grading systems used in cancer biology and medicine categorize cancer cells to with respect to their lack of cellular differentiation. This reflects the extent to which the cancer cells differ in morphology from healthy cells found in the tissue from which the cancer cell originated. The grading system can be used to provide an indication of how quickly a particular cancer might be expected to grow. Typically used grades of cancer are Grades (G) X and 1 to 4. GX indicates that the cancer grade cannot be assessed. G1 (low grade) cancer cells have a similar morphology to normal, healthy, cells (*i.e.* they are well differentiated) and would be expected to grow slowly, and are less likely to spread. G2 (intermediate grade) cancer cells are moderately differentiated, *i.e.* they appear more abnormal and would be expected to grow slightly faster than G1 cells. G3 (high grade) cancer cells have a very different morphology compared to normal cells (*i.e.* they are poorly differentiated) and would be expected to grow faster than G1 and G2 cells. G4 (high grade) cancer cells are undifferentiated (also referred to as anaplastic) and would be expected to have the highest capacity for proliferation. Thus, in some embodiments, the methods of the present invention may be used to detect a G1, G2, G3 and/or G4 gynaecological cancer. In some embodiments, the methods of the present invention may be particularly useful for detecting a G3 and/or G4 cancer.

Cancer grading is different to cancer staging, which gives an indication of how a cancer might spread. A common cancer staging system has five stages, namely Stage 0: cancer cells *in situ* (*i.e.* located in their normal tissue); Stage I: cancers are localized to one part of the body; Stage II: cancers are locally advanced; Stage III: cancers are also locally advanced (whether a cancer is designated as Stage II or Stage III can depend on the specific type of cancer); and Stage IV: cancers have often metastasized, or spread to other organs or throughout the body.

The FIGO (Fédération Internationale de Gynecologic et d'Obstetrique) system is commonly used for grading and/or staging of ovarian and endometrial cancers. The FIGO system also has five stages, namely stage 0: carcinoma in situ (common in cervical, vaginal, and vulval cancer); stage I: confined to the organ of origin; stage II: invasion of surrounding organs or tissue; stage III: spread to distant nodes or tissue within the pelvis; and stage IV: distant metastasis(es).

### Non-invasive Samples

Methods described herein may comprise a step of "*obtaining a non-invasive sample from a subject*"*.* As used herein, the term "*non-invasive*" refers to a step that does not require surgical intervention, such as that required to obtain a tissue sample in order to perform biopsy. Non-invasive samples that are used in the present invention are urine samples, tampon samples and vaginal swab samples.

In some embodiments, a urine sample is used in the methods of the present invention. A urine sample can be obtained using any technique known in the art that the skilled person would be aware of. For example, a urine sample may be obtained from a subject by asking the subject to urinate into a collection vessel, such as a sterile container. As used herein, the term "*urine sample*" encompasses any sample derived from the urine sample originally obtained from the subject. For example, the urine sample may be processed by centrifugation to obtain *e.g.* a pellet comprising urinary sediment comprising cells. The supernatant may be discarded and the urinary sediment cell pellet re-suspended in a suitable buffer, such as a lysis buffer according to the invention or otherwise described herein.

In other embodiments, a vaginal swab sample used in the methods of the present invention. A vaginal swab sample can be obtained using any technique known in the art that the skilled person would be aware of. For example, a vaginal swab sample may be obtained from a subject by a suitably trained medical professional inserting a soft endocervical collection brush (referred to as "*swab*") into the vagina of the subject and rotating it. The swab may be inserted 3-5 cm into the vagina and rotated four times (two towards the left and two towards the right). As used herein, the term "*vaginal swab sample*" encompasses any sample derived from vaginal swab sample originally obtained from the subject. For example, the vaginal swab sample may be processed by placing the swab into a vessel containing a suitable buffer, such as PBS, such that cells are transferred from the swab into the buffer. After removing the swab from the vessel, the buffer comprising the cells from the swab can be processed by centrifugation to obtain e.g. a pellet comprising urinary sediment comprising cells. The supernatant may be discarded and the urinary sediment cell pellet re-suspended in a suitable buffer, such as a lysis buffer according to the invention or otherwise described herein.

In yet other embodiments, a tampon sample is used in the methods of the present invention is a tampon sample. A tampon sample can be obtained using any method known in the art that the skilled person would be aware of. For example, a tampon sample may be obtained by asking a subject to wear a tampon. A tampon is a suitably shaped mass of absorbent material that can be inserted into the vagina for a period of time to be specified in order absorb vaginal secretions, such as menstrual blood. As used herein, the term "*tampon sample*" encompasses any sample derived from a tampon originally worn by the subject. In some embodiments, the tampon sample may be derived from tampon worn by the subject for 2 to 14 hours, 4 to 12 hours, or 6 to 8 hours. In some embodiments, the tampon sample may be derived from a tampon worn by the subject for about 4, 5, 6, 7, 8, 9, or 10 hours. The tampon sample may be obtained by removing the tampon from the subject's vagina and placing it in a vessel containing a suitable buffer, such as PBS, such that the tampon is soaked in the buffer. Alternatively, the buffer may be added to the tampon *in situ* in the vessel. The soaked tampon may then be compressed e.g. using a large syringe to release buffer comprising cells from the tampon, which may be collected into a fresh vessel. The buffer comprising the cells may then be processed by centrifugation to obtain e.g. a pellet comprising urinary sediment comprising cells. The supernatant may be discarded and the urinary sediment cell pellet re-suspended in a suitable buffer, such as a lysis buffer according to the invention or otherwise described herein.

Many important biomarkers for gynaecological cancers, such as MCM proteins and in particular MCM5, may be found in the nuclei of cells present in a non-invasive sample. Thus, suitably the non-invasive sample comprises cells. More suitably, those cells may be concentrated by any known technique common in the art, such as filtration or more suitably centrifugal collection of the cells from non-invasive sample. Enriching the cells from the non-invasive sample may increase the signal, and may facilitate detection. For example, where the non-invasive sample is a urine sample, the urine sample may comprise urinary sediment such as sedimented cells collected from urine.

Methods described herein may comprise a step of "*treating the non-invasive sample to release at least one biomarker from cells in the non-invasive sample*"*.* This step can be considered to refer to manipulating the non-invasive sample in such a way that cells comprised in the non-invasive sample release one or more biomarkers (or a substantial portion of these cells release one or more biomarker). The non-invasive sample may be treated to release the at least one biomarker by exposing the non-invasive sample to a lysis buffer capable of releasing the at least one biomarker from cells in the non-invasive sample, for example a lysis buffer disclosed herein. Suitably, the non-invasive sample may be treated to release the at least one biomarker by: passing the non-invasive sample through a filter for capturing cells, such that cells are captured in the filter; passing a lysis buffer, such as a lysis buffer according to the invention or otherwise described herein, through the filter, such that the captured cells are exposed to the lysis buffer; and/or incubating the filter for a period of time, such that the lysis buffer causes the cells to release at least one biomarker. Alternatively, the non-invasive sample may be centrifuged to provide a sample pellet, the supernatant discarded and the sample pellet re-suspended in the lysis buffer. Alternatively, for example, concentrated lysis buffer components may be added to a liquid non-invasive sample to form a solution comprising the non-invasive sample exposed to the lysis buffer. Methods of the present invention may comprise a step of "*treating the urine, tampon or vaginal swab sample to release MCM5 from cells in the urine, tampon or vaginal swab sample".*

### Antibodies

The term "*antibody*" can refer to naturally occurring forms or recombinant antibodies such as single-chain antibodies, chimeric antibodies or humanised antibodies. The terms "*antibody*" and "*antibodies*" may also be considered to encompass fragments of antibodies that can bind to a target protein, such as an MCM protein like MCM5. Such fragments may include Fab'₂, F'(ab)₂, Fv, single chain antibodies or diabodies. The antibodies may be naturally occurring, full length antibodies (rather than fragments). In a further embodiment, the antibodies are not humanised antibodies.

In general, antibodies are formed from two heavy chains and two lights chains. Each heavy chain is made up of heavy chain constant region (CH) and a heavy chain variable region (VH). Similarly each light chain is made up of light chain constant region (CL) and a light chain variable region (VL). The VH and VL regions comprise complementarity defining regions (CDRs). The CDRs are, primarily responsible for specific binding to the target protein.

In some embodiments, an antibody will bind to an epitope (fragment) of MCM5. Thus, the term *"antibody which binds to MCM5* " refers to an antibody that binds to only a single epitope of MCM5. Optionally an antibody that binds to MCM5 is an antibody that "*specifically binds*" to MCM5. The term "*specifically binds*" refer to antibody that binds to a target such as MCM5 with a binding affinity that is at least 2-fold, 10-fold, 50-fold or 100-fold greater than its binding affinity for a non-target molecule.

A detection antibody, such as detection antibody used in an ELISA assay or a sandwich ELISA assay used in the present invention, may be conjugated to a label (for example Europium²⁺ or Horseradish Peroxidase). The label may be directly attached or may be attached via a linker (such as Adipic Acid Dihyrazide or ADH). The label may be attached by chemical conjugation. Methods of conjugating labels to antibodies are known in the art. For example, carbodiimide conjugated (Bauminger & Wilchek (1980) Methods Enzymol. 70, 151-159) may be used to conjugate labels to antibodies. Other methods for conjugating a label to an antibody can also be used. For example, sodium periodate oxidation followed by reductive alkylation or reduction amidation of appropriate reactants can be used, as can glutaraldehyde cross-linking. However, it is recognised that, regardless of which method of producing a conjugate of the invention is selected, a determination must be made that the conjugated antibody maintains its targeting ability and that the conjugated label maintains its function.

It is well within the ability of the person skilled in the art to develop an antibody that binds to a biomarker protein of interest, such as MCM5. This may be performed by immunising a mammal such as a mouse, rabbit or guinea pig, with the biomarker protein *e.g.* MCM5. It may be beneficial to include an adjuvant such as Freund's complete adjuvant. The spleen cells of the immunised mammal are removed and fused with myeloma cells to form hybridoma lines which are immortal given appropriate conditions and which secrete antibodies. The hybridomas are separated into single clones and the antibodies secreted by each clone are evaluated for their binding ability to the biomarker protein (*e.g.* MCM5).

### Antibody Target

In some embodiments of the methods of the invention, the urine, tampon or vaginal swab sample is exposed to a first monoclonal antibody and/or a second monoclonal antibody. As discussed above, such embodiments may involve performing an ELISA assay or a sandwich ELISA assay which comprises exposing the urine, tampon or vaginal swab sample to first monoclonal antibody described herein and/or second monoclonal antibody described herein. In one embodiment, the first monoclonal antibody and/or the second monoclonal antibody bind specifically to MCM5. Preferably the first monoclonal antibody binds to SEQ ID NO: 1. Preferably the second monoclonal antibody binds to SEQ ID NO: 2.

In some embodiments, the first monoclonal antibody or the second monoclonal antibody will bind to an epitope (fragment) of MCM5 (for example SEQ ID NO: 1 or SEQ ID NO: 2). Thus, the term *"antibody which binds to MCM5"* refers to an antibody that binds to only a single epitope of MCM5, such as SEQ ID NO: 1 or SEQ ID NO: 2.

For the purposes of the present invention the term "*binding affinity*" refers to the ability of an antibody to bind to its target. For the purposes of the present invention, the term "*specifically binds*" refers to an antibody that binds to a target, such as MCM5, with a binding affinity that is at least 2-fold, 10-fold, 50-fold or 100-fold greater than its binding affinity for binding to another non-target molecule. In an embodiment the non-target molecule is an MCM protein, other than MCM5, such as MCM2. Preferably, the first monoclonal antibody and/or the second monoclonal antibody is capable of binding to an MCM protein, optionally MCM5, with a binding affinity that is at least 2-fold, 10-fold, 50-fold or 100-fold greater than its binding affinity for binding to another non-target molecule. Even more preferably, the first monoclonal antibody and/or the second monoclonal antibody is capable of binding to SEQ ID NO: 1 (such as the first monoclonal antibody) or SEQ ID NO: 2 (such as the second monoclonal antibody) with a binding affinity that is at least 2-fold, 10-fold, 50-fold or 100-fold greater than its binding affinity for binding to another non-target molecule.

A preferred method for the evaluation of binding affinity for MCM5 is by ELISA. Preferably, the first monoclonal antibody and/or the second monoclonal antibody have an affinity for MCM5 (measured as an EC50 or 50% maximum binding concentration, as described in Example 2) of 2500 ng/ml or lower, 1500 ng/ml or lower, 1000 ng/ml or lower, 600 ng/ml or lower, 50 ng/ml or lower, 30 ng/ml or lower, 20 ng/ml or lower, or 10 ng/ml or lower. The EC50 will typically be higher than 1 ng/ml and thus the EC50 may be between 1 ng/ml and any of the upper limits specified in the preceding sentence. Other standard assays to evaluate the binding ability of ligands such as antibodies towards targets are known in the art, including for example, Western blots, RIAs, and flow cytometry analysis. The binding kinetics (*e.g*. binding affinity) of the antibody also can be assessed by standard assays known in the art, such as by Surface Plasmon Resonance (SPR) (e.g. Biacore ^{™} system) analysis. The affinity constant (KD) for binding to MCM5 is preferably in the range of 0.01-10 nM, 0.01-5 nM, 0.01-1 nM, 0.01-0.5 nM, 0.01-0.25 nM, 0.025-0.25 nM, or 0.04-0.25 nM. In one embodiment, the first and/or second monoclonal antibody used in the ELISA has an affinity for MCM5 of about 0.01 nM, 0.02 nM, 0.03 nM, 0.04 nM, 0.05 nM, 0.06 nM, 0.07 nM, 0.08 nM, 0.09 nM, 0.1 nM, 0.2 nM, 0.3 nM, 0.4 nM, or 0.5 nM. In one embodiment, the first monoclonal antibody and/or second monoclonal antibody used in the ELISA has an affinity for MCM5 of about 0.05 nM. In one embodiment, the first monoclonal antibody and/or second monoclonal antibody used in the ELISA has an affinity for MCM5 of about 0.2 nM. In one embodiment, the first monoclonal antibody and/or second monoclonal antibody used in the ELISA has an affinity for MCM5 of about 0.233 nM. In one embodiment, the first monoclonal antibody has an affinity for MCM5 of about 0.05 nM and the second monoclonal antibody has an affinity for MCM5 of about 0.2 nM. In one embodiment, the first monoclonal antibody has an affinity for MCM5 of about 0.05 nM and the second monoclonal antibody has an affinity for MCM5 of about 0.233 nM. In one embodiment, the first monoclonal antibody is 12A7 and has an affinity for MCM5 of about 0.05 nM and the second monoclonal antibody is 4B4 and has an affinity for MCM5 of about 0.2 nM. In one embodiment, the first monoclonal antibody is 12A7 and has an affinity for MCM5 of about 0.05 nM and the second monoclonal antibody is 4B4 and has an affinity for MCM5 of about 0.233 nM.The association rate (ka) is preferably in the range of 0.4-3.4 × 10⁶ 1/M. The dissociation rate (kd) is preferably in the range of 1-10 × 10⁻³ 1/s. These values may typically be determined by SPR (surface plasmon resonance).

### Antibody Complementary Determining Regions (CDRs)

The methods of the present invention may comprise the use of a first monoclonal antibody and/or a second monoclonal antibody comprising at least one of the CDRs of antibodies 12A7 or 4B4, *i.e.* a CDR selected from the group consisting of:
a. 12A7 CDRH1 which has a sequence of SEQ ID NO: 9 or a sequence that differs from SEQ ID NO: 9 by 1, 2 or 3 amino acid substitutions;
b. 12A7 CDRH2 which has a sequence of SEQ ID NO: 11 or a sequence that differs from SEQ ID NO: 11 by 1, 2 or 3 amino acid substitutions;
c. 12A7 CDRH3 which has a sequence of SEQ ID NO: 13 or a sequence that differs from SEQ ID NO: 13 by 1, 2 or 3 amino acid substitutions;
d. 12A7 CDRL1 which has a sequence of SEQ ID NO: 3 or a sequence that differs from SEQ ID NO: 3 by 1, 2 or 3 amino acid substitutions;
e. 12A7 CDRL2 which has a sequence of SEQ ID NO: 5 or a sequence that differs from SEQ ID NO: 5 by 1, 2 or 3 amino acid substitutions;
f. 12A7 CDRL3 which has a sequence of SEQ ID NO: 7 or a sequence that differs from SEQ ID NO: 7 by 1, 2 or 3 amino acid substitutions;
g. 4B4 CDRH1 which has a sequence of SEQ ID NO: 21 or a sequence that differs from SEQ ID NO: 21 by 1, 2 or 3 amino acid substitutions;
h. 4B4 CDRH2 which has a sequence of SEQ ID NO: 23 or a sequence that differs from SEQ ID NO: 23 by 1, 2 or 3 amino acid substitutions;
i. 4B4 CDRH3 which has a sequence of SEQ ID NO: 25 or a sequence that differs from SEQ ID NO: 25 by 1, 2 or 3 amino acid substitutions
j. 4B4 CDRL1 which has a sequence of SEQ ID NO: 15 or a sequence that differs from SEQ ID NO: 15 by 1, 2 or 3 amino acid substitutions;
k. 4B4 CDRL2 which has a sequence of SEQ ID NO: 17 or a sequence that differs from SEQ ID NO: 17 by 1, 2 or 3 amino acid substitutions; and
l. 4B4 CDRL3 which has a sequence of SEQ ID NO: 19 or a sequence that differs from SEQ ID NO: 19 by 1, 2 or 3 amino acid substitutions.

Antibodies that have the same CDRs as the 4B4 and 12A7 antibodies may differ substantially from the sequences of 4B4 and 12A7 in other regions. Such antibodies may, for example, be antibody fragments.

The phrase "*sequence that differs from SEQ ID NO: 3 by a single amino acid substitution"* refers to the possibility of replacing one amino acid defined in SEQ ID NO: 3 by a different amino acid. Preferably such a replacement is a conservative amino acid substitution. The following eight groups each contain amino acids that are typically conservative substitutions for one another (1) Alanine, Glycine; (2) Aspartic acid, Glutamic acid; (3) Asparagine, Glutamine; (4) Arginine, Lysine; (5) Isoleucine, Leucine, Methionine, Valine; (6) Phenylalanine, Tyrosine, Tryptophan; (7) Serine, Threonine; and (8) Cysteine, Methionine.

In an embodiment the first monoclonal antibody comprises at least one CDR from the heavy chain of 12A7 (12A7 CDRH1, 12A7 CDRH2 or 12A7 CDRH3) as well as at least one CDR from the light chain of 12A7 (12A7 CDRL1, 12A7 CDRL2 or 12A7 CDRL3). In a further embodiment the first monoclonal antibody comprises at least two CDRs from the heavy chain of 12A7 and at least two CDRs from the light chain of 12A7. In a preferred embodiment the first monoclonal antibody comprises all three CDRs from the heavy chain of 12A7 and/or all three CDRs from the light chain of 12A7. In an embodiment the first monoclonal antibody comprises 12A7 CDRL1 and 12A7 CDRL2, 12A7 CDRL1 and 12A7 CDRL3, 12A7 CDRL1 and 12A7 CDRH1, 12A7 CDRL1 and 12A7 CDRH2, 12A7 CDRL1 and 12A7 CDRH3, 12A7 CDRL2 and 12A7 CDRL3, 12A7 CDRL2 and 12A7 CDRH1, 12A7 CDRL2 and 12A7 CDRH2, 12A7 CDRL2 and 12A7 CDRH3, 12A7 CDRL3 and 12A7 CDRH1, 12A7 CDRL3 and 12A7 CDRH2, 12A7 CDRL3 and 12A7 CDRH3, 12A7 CDRH1 and 12A7 CDRH2, 12A7 CDRH1 and 12A7 CDRH3, or 12A7 CDRH2 and 12A7 CDRH3.

In an embodiment, the second monoclonal antibody comprises at least one CDR from the heavy chain of 4B4 (4B4 CDRH1, 4B4 CDRH2 or 4B4 CDRH3) as well as at least one CDR from the light chain of 4B4 (4B4 CDRL1, 4B4 CDRL2 or 4B4 CDRL3). In a further embodiment, the second monoclonal antibody comprises at least two CDRs from the heavy chain of 4B4 and at least two CDRs from the light chain of 4B4. In a preferred embodiment, the second monoclonal antibody comprises all three CDRs from the heavy chain of 4B4 and/or all three CDRs from the light chain of 4B4. In an embodiment, the second monoclonal antibody comprises 4B4 CDRL1 and 4B4 CDRL2, 4B4 CDRL1 and 4B4 CDRL3, 4B4 CDRL1 and 4B4 CDRH1, 4B4 CDRL1 and 4B4 CDRH2, 4B4 CDRL1 and 4B4 CDRH3, 4B4 CDRL2 and 4B4 CDRL3, 4B4 CDRL2 and 4B4 CDRH1, 4B4 CDRL2 and 4B4 CDRH2, 4B4 CDRL2 and 4B4 CDRH3, 4B4 CDRL3 and 4B4 CDRH1, 4B4 CDRL3 and 4B4 CDRH2, 4B4 CDRL3 and 4B4 CDRH3, 4B4 CDRH1 and 4B4 CDRH2, 4B4 CDRH1 and 4B4 CDRH3, or 4B4 CDRH2 and 4B4 CDRH3.

In a preferred embodiment, an antibody comprises at least one CDR having a sequence identical to that described in any one of SEQ ID NO: 3 (12A7 CDRL1), SEQ ID NO: 5 (12A7 CDRL2), SEQ ID NO: 7 (12A7 CDRL3), SEQ ID NO: 9 (12A7 CDRH1), SEQ ID NO: 11 (12A7 CDRH2), SEQ ID NO: 13 (12A7 CDR H3), SEQ ID NO: 15 (4B4 CDRL1), SEQ ID NO: 17 (4B4 CDRL2), SEQ ID NO: 19 (4B4 CDRL3), SEQ ID NO: 21 (4B4 CDRH1), SEQ ID NO: 23 (4B4 CDRH2) or SEQ ID NO: 25 (4B4 CDRH3). In an embodiment, where the first monoclonal antibody comprises 12A7 CDRL2, the 12A7 CDRL2 has the sequence described in SEQ ID NO: 5. In a further embodiment, where the first monoclonal antibody comprises 12A7 CDRL1, the 12A7 CDRL1 has the sequence described in SEQ ID NO: 3. In a further embodiment, where the first monoclonal antibody comprises 12A7 CDRL3, the 12A7 CDRL3 has the sequence described in SEQ ID NO: 7. In a further embodiment where the first monoclonal antibody comprises 12A7 CDRH1, the 12A7 CDRH1 has the sequence described in SEQ ID NO: 9. In a further embodiment, where the first monoclonal antibody comprises 12A7 CDRH2, the 12A7 CDRH2 has the sequence described in SEQ ID NO: 11. In a further embodiment, where the first monoclonal antibody comprises 12A7 CDRH3, the 12A7 CDRH3 has the sequence described in SEQ ID NO: 13. In a further embodiment, where the second monoclonal antibody comprises 4B4 CDRL1, the 4B4 CDRL1 has the sequence described in SEQ ID NO: 15. In a further embodiment, where the second monoclonal antibody comprises 4B4 CDRL2, the 4B4 CDRL2 has the sequence described in SEQ ID NO: 17. In a further embodiment, where the second monoclonal antibody comprises 4B4 CDRL3, the 4B4 CDRL3 has the sequence described in SEQ ID NO: 19. In a further embodiment, where the second monoclonal antibody comprises 4B4 CDRH1, the 4B4 CDRH1 has the sequence described in SEQ ID NO: 21. In a further embodiment, where the second monoclonal antibody comprises 4B4 CDRH2, the 4B4 CDRH2 has the sequence described in SEQ ID NO: 23. In a further embodiment, where the second monoclonal antibody comprises 4B4 CDRH3, the 4B4 CDRH3 has the sequence described in SEQ ID NO: 25.

Preferably the first monoclonal antibody and/or the second monoclonal antibody comprising at least one of the CDRs of 12A7 or 4B4 binds (optionally specifically binds) to MCM5. Even more preferably the first monoclonal antibody and/or the second monoclonal antibody comprising at least one of the CDRs of 12A7 or 4B4 binds (optionally specifically binds) to SEQ ID NO: 1 or SEQ ID NO: 2.

### Heavy and Light Chain Variable Regions

In some embodiments, the first monoclonal antibody used in the present invention comprises a heavy chain variable region having a sequence at least 85%, 90%, 95%, 98%, 99% or 100% identical to SEQ ID NO: 29. In some embodiments, the first monoclonal antibody used in the present invention comprises a light chain variable region sequence having a sequence at least 85%, 90%, 95%, 98%, 99% or 100% identical to SEQ ID NO: 27. In some embodiments, the second monoclonal antibody used in the present invention comprises a heavy chain variable region having a sequence at least 85%, 90%, 95%, 98%, 99% or 100% identical to SEQ ID NO: 33. In some embodiments, the second monoclonal antibody used in the present invention comprises a light chain variable region sequence having a sequence at least 85%, 90%, 95%, 98%, 99% or 100% identical to SEQ ID NO: 31. Such antibodies may be referred to as "*variant antibodies*"*.*

In an embodiment, the first monoclonal antibody comprises a heavy chain variable region having a sequence at least 95% identical to SEQ ID NO: 29. In a further embodiment, the first monoclonal antibody comprises a heavy chain variable region having a sequence at least 98% identical to SEQ ID NO: 29. In one embodiment, the first monoclonal antibody comprises a light chain variable region having a sequence at least 95% identical to SEQ ID NO: 27. In a further embodiment, the first monoclonal antibody comprises a light chain variable region having a sequence at least 98% identical to SEQ ID NO: 27. In a further embodiment, the first monoclonal antibody comprises a light chain variable region having a sequence at least 95% identical to SEQ ID NO: 27 and a heavy chain variable region having a sequence at least 95% identical to SEQ ID NO: 29. In a preferred embodiment, the first monoclonal antibody comprises a heavy chain variable region having a sequence at least 98% identical to SEQ ID NO: 29 and a light chain variable region having a sequence at least 98% identical to SEQ ID NO: 27.

In a further embodiment, the second monoclonal antibody comprises a heavy chain variable region having a sequence at least 95% identical to SEQ ID NO: 33. In a further embodiment, the second monoclonal antibody comprises a heavy chain variable region having a sequence at least 98% identical to SEQ ID NO: 33. In a further embodiment, the second monoclonal antibody comprises a light chain variable region having a sequence at least 95% identical to SEQ ID NO: 31. In a further embodiment, the second monoclonal antibody comprises a light chain variable region having a sequence at least 98% identical to SEQ ID NO: 31. In a further embodiment, the second monoclonal antibody has a heavy chain variable region having a sequence at least 95% identical to SEQ ID NO:33 and a light chain variable region having a sequence at least 95% identical to SEQ ID NO: 31. In a preferred embodiment, the second monoclonal antibody has a heavy chain variable region having a sequence at least 98% identical to SEQ ID NO: 33 and a light chain variable region having a sequence at least 98% identical to SEQ ID NO: 31.

As is known to the person skilled in the art, antibodies contain multiple regions including framework regions. Deletion or addition of amino acids in the framework regions is unlikely to affect the ability of the antibody to bind to its target. On the other hand, mutations in the CDRs are considerably more likely to affect the ability of an antibody to bind to a target. Thus, in certain embodiments of the invention, variant antibodies have CDRs which are identical to the CDRs of the 12A7 or 4B4 antibodies or have CDRs which vary in only a single amino acid substitution (preferably a conservative amino acid substitution). The first monoclonal antibody and/or the second monoclonal antibody may have framework regions which differ in sequence quite significantly from those described in SEQ ID NO: 27, 29, 31 or 33.

Optionally, where the first monoclonal antibody comprises a heavy chain variable region having a sequence at least 85%, 90%, 95%, 98%, 99% or 100% identical to SEQ ID NO: 29, the antibody further comprises at least one of 12A7 CDRH1, 12A7 CDRH2 or 12A7 CDRH3. It is understood by the person skilled in the art that, since target binding specificity is determined by the CDRs, an antibody comprising the CDRs of 12A7 may still bind to MCM5 even if the remainder of the antibody sequence is quite variable. For this reason where the first monoclonal antibody comprises at least one of 12A7 CDRH1, 12A7 CDRH2 or 12A7 CDRH3 the first monoclonal antibody preferably comprises a heavy chain variable region having a sequence at least 90% identical to SEQ ID NO: 29. In a more preferred embodiment the first monoclonal antibody of the invention comprises 12A7 CDRH1, 12A7 CDRH2 and 12A7 CDRH3 and comprises a heavy chain variable region having a sequence at least 95% identical to SEQ ID NO:29.

Optionally, where the second monoclonal antibody comprises a heavy chain variable region having a sequence at least 85%, 90%, 95%, 98%, 99% or 100% identical to SEQ ID NO: 33, the second monoclonal antibody further comprises at least one of 4B4 CDRH1, 4B4 CDRH2 or 4B4 CDRH3. It is understood by the person skilled in the art that, since target binding specificity is determined by the CDRs, an antibody comprising the CDRs of 4B4 may still bind to MCM5 even if the remainder of the antibody sequence is quite variable. For this reason where the second monoclonal antibody comprises at least one of 4B4 CDRH1, 4B4 CDRH2 or 4B4 CDRH3 the second monoclonal antibody preferably comprises a heavy chain variable region having a sequence at least 90% identical to SEQ ID NO: 33. In a more preferred embodiment the second monoclonal antibody comprises 4B4 CDRH1, 4B4 CDRH2 and 4B4 CDRH3 and comprises a heavy chain variable region having a sequence at least 95% identical to SEQ ID NO: 33.

Optionally, where the first monoclonal antibody comprises a light chain variable region having a sequence at least 85%, 90%, 95%, 98%, 99% or 100% identical to SEQ ID NO: 27, the first monoclonal antibody further comprises at least one of 12A7 CDRL1, 12A7 CDRL2 or 12A7 CDRL3. It is understood by the person skilled in the art that, since target binding specificity is determined by the CDRs, an antibody comprising the CDRs of 12A7 may still bind to MCM5 even if the remainder of the antibody sequence is quite variable. For this reason where the first monoclonal antibody comprises at least one of 12A7 CDRL1, 12A7 CDRL2 or 12A7 CDRL3 the first monoclonal antibody preferably comprises a light chain variable region having a sequence at least 90% identical to SEQ ID NO: 27. In a more preferred embodiment the first monoclonal antibody comprises 12A7 CDRL1, 12A7 CDRL2 and 12A7 CDRL3 and comprises a light chain variable region having a sequence at least 95% identical to SEQ ID NO: 27.

Optionally, where the second monoclonal antibody of the invention comprises a light chain variable region having a sequence at least 85%, 90%, 95%, 98%, 99% or 100% identical to SEQ ID NO: 31, the second monoclonal antibody further comprises at least one of 4B4 CDRL1, 4B4 CDRL2 or 4B4 CDRL3. It is understood by the person skilled in the art that, since target binding specificity is determined by the CDRs, an antibody comprising the CDRs of 4B4 may still bind to MCM5 even if the remainder of the antibody sequence is quite variable. For this reason where the second monoclonal antibody comprises at least one of 4B4 CDRL1, 4B4 CDRL2 or 4B4 CDRL3 the second monoclonal antibody preferably comprises a heavy chain variable region having a sequence at least 90% identical to SEQ ID NO: 31. In a more preferred embodiment the second monoclonal antibody comprises 4B4 CDRL1, 4B4 CDRL2 and 4B4 CDRL3 and comprises a heavy chain variable region having a sequence at least 95% identical to SEQ ID NO:31.

In a further embodiment of the invention the first monoclonal antibody comprises:
(i) 12A7 CDRH1, 12A7 CDRH2 and 12A7 CDRH3;
(ii) a heavy chain variable region having a sequence at least 95% identical to SEQ ID NO: 29;
(iii) 12A7 CDRL1, 12A7 CDRL2 and 12A7 CDRL3; and
(iv) a light chain variable region having a sequence at least 95% identical to SEQ ID NO: 27.

In a further embodiment the second monoclonal antibody comprises:
(i) 4B4 CDRH1, 4B4 CDRH2 and 4B4 CDRH3;
(ii) a heavy chain variable region having a sequence at least 95% identical to SEQ ID NO: 33;
(iii) 4B4 CDRL1, 4B4 CDRL2 and 4B4 CDRL3; and
(iv) a heavy chain variable region having a sequence at least 95% identical to SEQ ID NO: 31.

An antibody having a heavy chain variable sequence identical to SEQ ID NO: 29 and a light chain variable sequence identical to SEQ ID NO: 27 may be referred to as antibody 12A7. An antibody having a heavy chain variable sequence identical to SEQ ID NO: 33 and a light chain variable sequence identical to SEQ ID NO: 31 may be referred to as an antibody 4B4.

It is well within the knowledge of the person skilled in the art how to make variant antibodies which bind to MCM5. Such variant antibodies may comprise 1, 2, 3, 4, 5, 6, 7, 8, 9, 10, 15, 20, 25, 50 or 100 substitution or deletion mutations compared to SEQ ID NOs: 27, 29, 31 or 33. '*Deletion*' variant antibodies may comprise the deletion of 1, 2, 3, 4, 5 or more amino acids or, in some cases, the deletion of entire regions of SEQ ID NOs: 27, 29, 31 or 33. '*Substitution*' variants may comprise the replacement of 1, 2, 3, 4, 5 or more amino acids with the same number of new amino acids.

Preferably, the variant antibodies described herein comprise sequences differing from SEQ ID NOs: 27, 29, 31 or 33 by conservative amino acid substitutions (optionally only by conservative amino acid substitutions). The skilled person is well aware that such conservative substitutions are unlikely to alter the binding properties of an antibody.

### Lysis Buffers

Lysis buffers are generally buffers which are able to lyse cells. In addition to releasing one or more biomarkers from cells, the lysis buffer may be compatible with the method used for subsequent analysis. For example, where the analysis method is double-antibody sandwich ELISA, it may be desirable that the lysis buffer does not degrade the capture antibody bound to the surface of the microtitre plate. Lysis buffers generally but not exclusively comprise one or more detergents (also known as surfactants), one or more salts and a buffering agent. In some instance, the concentrations of these components may affect the efficacy of the lysis buffer.

In one embodiment, a buffer can be said to be a "*lysis buffer*" if it is capable of lysing at least 10 %, 20 %, 30 %, 40 %, 50 %, 60 %, 70 %, 80 %, 90%, 91 %, 92 %, 93 %, 94 %, 95 %, 96 %, 97 %, 98 %, 99 %, or substantially all of the cells in a non-invasive sample. In one embodiment, a buffer can be said to be a "*lysis buffer*" if it is capable of lysing at least 10 %, 20 %, 30 %, 40 %, 50 %, 60 %, 70 %, 80 %, 90%, 91 %, 92 %, 93 %, 94 %, 95 %, 96 %, 97 %, 98 %, 99 %, or substantially all of the cells in a urine sample.

In one embodiment, a buffer can be said to be a "*lysis buffer*" if it is capable of lysing at least 10 %, 20 %, 30 %, 40 %, 50 %, 60 %, 70 %, 80 %, 90%, 91 %, 92 %, 93 %, 94 %, 95 %, 96 %, 97 %, 98 %, 99 %, or substantially all of the cells in a non-invasive sample where the cells in the non-invasive sample are exposed to the lysis buffer for at least 1 minute, 5 minutes, 10 minutes, 20 minutes, 30 minutes, 60 minutes, 1 hour, 2 hours, 6 hours, 12 hours, 24 hours, or overnight. In one embodiment, a buffer can be said to be a "*lysis buffer*" if it is capable of lysing at least 10 %, 20 %, 30 %, 40 %, 50 %, 60 %, 70 %, 80 %, 90%, 91 %, 92 %, 93 %, 94 %, 95 %, 96 %, 97 %, 98 %, 99 %, or substantially all of the cells in a urine sample where the cells in the urine are exposed to the lysis buffer for at least 1 minute, 5 minutes, 10 minutes, 20 minutes, 30 minutes, 60 minutes, 1 hour, 2 hours, 6 hours, 12 hours, 24 hours, or overnight.

In one embodiment, a buffer can be said to be a "*lysis buffer*" if it is capable of lysing at least 10 %, 20 %, 30 %, 40 %, 50 %, 60 %, 70 %, 80 %, 90%, 91 %, 92 %, 93 %, 94 %, 95 %, 96 %, 97 %, 98 %, 99 %, or substantially all of the cells in a non-invasive sample where the cells in the non-invasive sample are exposed to the lysis buffer for at least 1 minute, 5 minutes, 10 minutes, 20 minutes, 30 minutes, 60 minutes, 1 hour, 2 hours, 6 hours, 12 hours, 24 hours, or overnight at 4 °C, 20 °C, or room temperature. In one embodiment, a buffer can be said to be a "*lysis buffer*" if it is capable of lysing at least 10 %, 20 %, 30 %, 40 %, 50 %, 60 %, 70 %, 80 %, 90%, 91 %, 92 %, 93 %, 94 %, 95 %, 96 %, 97 %, 98 %, 99 %, or substantially all of the cells in a urine sample where the cells in the urine are exposed to the lysis buffer for at least 1 minute, 5 minutes, 10 minutes, 20 minutes, 30 minutes, 60 minutes, 1 hour, 2 hours, 6 hours, 12 hours, 24 hours, or overnight at 4 °C, 20 °C, or room temperature.

In one embodiment, the lysis buffer is capable of releasing MCM5 from cells in the urine, tampon or vaginal swab sample. A lysis buffer will be considered to be "*capable of releasing a biomarker from cells in the non-invasive sample*" if the amount of the biomarker (such as MCM5) released is greater than 40 %, 50 %, 60 %, 70 %, or 80 % the amount released when a buffer comprising 25 mM Tris pH 7.6, 150 mM sodium chloride, 1 % sodium deoxycholate, 0.1 % sodium dodecyl sulphate (SDS), and 1 % Triton-X100 is used. The amount of a biomarker, such as MCM5, that is released may be measured using an ELISA assay such as the assay described in Example 1. The antibodies used in the assay should be antibodies that bind to the biomarker. For example, where the biomarker is MCM5, a first monoclonal antibody and/or second monoclonal antibody that bind to MCM5 should be used. Preferably 12A7 and 4B4 antibodies are used.

A lysis buffer can be considered to not denature an antibody if the activity of the antibody after exposure to the lysis buffer is 40 %, 50 %, 60 %, 70 % or 80 % the activity of the antibody prior to exposure to the lysis buffer. The activity of the antibody may be tested using an ELISA assay such as that described in Example 1.

In some embodiments, the lysis buffer comprises a detergent (also referred to as a surfactant). In general detergents are compounds that are known to disrupt cell walls. Detergents are amphiphilic having both hydrophobic and hydrophilic regions. Suitable detergents are well known to the person of skill in the art. Examples of detergents that may suitably be used in a lysis buffer according to the present invention include, but are not limited to, Triton X-100, sodium doceyl sulphate (SDS), sodium deoxycolate,

In some embodiments, the lysis buffer comprises a buffer component. A buffer component can be considered to be any component which maintains the pH of the lysis buffer at a pH varying by less than 2.0 pH units, 1.5 pH units or 1.0 pH units. Buffers which are suitable for this purpose are well known to the person skilled in the art. An example of a buffer that may suitably used in a lysis buffer according to the present invention is Tris.

In some embodiments, the lysis buffer comprises a salt. Various salts are well known to the person skilled in the art. In some embodiments, the salt is added to the lysis buffer to provide a particular level of ionic strength. The person skilled in the art understands the relationship between salt concentration and ionic strength and would be capable to select a suitable salt and a suitable concentration of said salt to provide a lysis buffer having a desired ionic strength. An example of a salt that can be suitably used in a lysis buffer according to the invention is sodium chloride.

### Minichromosome Maintenance (MCM) Proteins

Suitably, a preferred biomarker is an MCM protein. Most suitably, according to the present invention, the MCM protein is MCM5.

MCM proteins 2-7 comprise part of the pre-replication complexes which form on chromatin and which are essential prerequisites, or licensing factors, for subsequent DNA replication. The MCM protein complexes act as replicative helicases and thus are core components of the DNA replication machinery. MCM proteins are upregulated in the transition from the G0 to G1/S phase of the cell cycle and actively participate in cell cycle regulation. The MCM proteins form an annular structure around the chromatin.

The human MCM5 gene maps to 22q13.1 and the mature MCM5 protein consists of 734 amino acids (SEQ ID NO: 35; UNIPROT P33992: HUMAN DNA replication licensing factor MCM5). The term "*MCM5*" refers to a polypeptide of SEQ ID NO: 35, a polypeptide 85 %, 90 %, 95 %, 98 %, or 100 % identity to SEQ ID NO: 35.

### Kits

Described herein is a kit comprising (a) a lysis buffer as defined herein; (b) a first monoclonal antibody as defined herein; and/or (c) a second monoclonal antibody as defined herein; and (d) instructions for use of the lysis buffer and/or the first monoclonal antibody and/or the second monoclonal antibody in a method of detecting the presence or absence of a gynaecological cancer in a subject. The kit can be used to perform the methods of the present invention. For example, the lysis buffer can be used to release MCM5 from cells in the urine, tampon or vaginal swab sample. The first monoclonal antibody and/or the second monoclonal antibody can be used in an immunoassay to determine the concentration of the at least one biomarker. According to the present invention, the immunoassay is an ELISA assay, optionally a sandwich ELISA assay. In such embodiments, the first monoclonal antibody may be used as a capture antibody and the second monoclonal antibody may be used as a detection antibody or *vice versa.*

### Uses

Further aspects of the present invention relate to the use of lysis buffers, monoclonal antibodies and kits of the invention or otherwise described herein in a method of detecting the presence or absence of a gynaecological cancer in a subject. The lysis buffers, monoclonal antibodies and kits described herein may be used in any suitable method of detecting the presence or absence of a gynaecological cancer in a subject. The lysis buffers, monoclonal antibodies and kits described herein may be used in a method of detecting the presence or absence of a gynaecological cancer in a subject according to the present invention. The uses and methods of the present invention may be *in vitro* or *ex-vivo* uses or methods.

### EXAMPLES

### Example 1 - Materials and Methods

### Study population

Patients were enrolled into the study at St Mary's Hospital, Manchester, between March 2017 and January 2018, ethical approval was obtained from South Central - Oxford B Research Ethics Committee (16/SC/0643), and informed consent obtained from all patients prior to the collection of urine, tampon or swab samples. All eligible patients with a known or strong suspicion of ovarian or endometrial cancer were enrolled. Patients were excluded if they were *virgo intacta*, if they had a previous diagnosis of bladder or renal cancer, if the patient had undergone any urological instrumentation in the preceding two weeks or if the patient was currently receiving chemotherapy or radiotherapy. Patients were asked to provide two samples, a full void urine sample and either a vaginal swab collected by the research nurse, or a vaginal tampon worn 6-8 hours prior to their appointment.

### Sample processing - urine

A minimum of 25 mL urine was collected from each patient, urine was agitated to ensure a homogenous mix and up to 50 mL was transferred into a clean centrifuge tube. Samples were centrifuged at room temperature at 1500 g for 5 minutes. Supernatant was discarded taking care not to disturb the cell sediment pellet and tubes were placed upside down to drain on absorbent paper. Cell sediment pellets were resuspended in an appropriate volume of lysis buffer comprising 25 mM Tris pH 7.6, 150 mM sodium chloride, 1 % sodium deoxycholate, 0.1 % sodium dodecyl sulphate (SDS), and 1 % Triton-X100 (10 uL Lysis buffer per mL of urine) and incubated at room temperature for 1 hr before being stored at less than -20 °C.

### Sample processing - vaginal tampon

Patients were asked to wear a commercially available plastic applicator vaginal tampon and instructed to place the tampon in their vagina for 6-8 hours prior to their attendance at the clinic. The tampon was removed and placed in a 50 mL centrifuge tube and PBS added. The tampon was transferred to a large syringe and compressed to release the PBS/cells from the tampon. The PBS/cells from the tampon were collected in a fresh 50 mL centrifuge tube. Tubes containing PBS/cells extracted from the syringe were centrifuged at room temperature at 1500 g for 5 minutes. Supernatant was discarded, taking care not to disturb the cell sediment pellet. Tubes were placed upside down on absorbent paper to drain and the cell sediment pellets were resuspended in 300 µL lysis buffer comprising 25 mM Tris pH 7.6, 150 mM sodium chloride, 1 % sodium deoxycholate, 0.1 % sodium dodecyl sulphate (SDS), and 1 % Triton-X100 and incubated at room temperature for 1 hr before being stored at less than -20 °C.

### Sample processing - vaginal swab

Vaginal swabs were collected by the research nurse, briefly; a soft endocervical collection brush was inserted 3-5 cm into the vagina and rotated four times (two towards the left and two towards the right). The swab was then placed into 5 mL of PBS. The vaginal swab was removed from the tube and the PBS was centrifuged at room temperature at 1500 g for 5 minutes. Supernatant was discarded, taking care not to disturb the cell sediment pellet. Tubes were placed upside down to drain on absorbent paper and the cell sediment pellets were resuspended in 300 µL lysis buffer comprising 25 mM Tris pH 7.6, 150 mM sodium chloride, 1 % sodium deoxycholate, 0.1 % sodium dodecyl sulphate (SDS), and 1 % Triton-X100 and incubated at room temperature for 1 hr before being stored at less than -20 °C.

### MCM5 ELISA

Patient lysates were tested with an MCM5 ELISA as per the manufacturer's instructions. Briefly; 100µL of lysate was added to each of two wells of the MCM5 ELISA micro-titre plate (samples and controls were run in duplicates) and incubated for 60 minutes at room temperature on a plate shaker (700 RPM). Following incubation wells were washed six times with 350 µL of 1x wash buffer using an automated plate washer. 100 µL of MCMS-HRP conjugated antibody was added to each well and incubated at room temperature for 30 minutes prior to being washed six times with 350 µL of 1x wash buffer as above. 100µL of TMB was added to each well and incubated for 30 minutes in the dark prior to the addition of a stop solution (0.5 M H₂SO₄). Optical density (OD) was measured at 450 nm and 630 nm (reference wavelength). Concentrations of MCM5 were calculated using a serial dilution standard curve of a known recombinant MCM5 control (1.3 mg/mL) with a negative control (Lysis buffer).

### Example 2 - Detection of Endometrial Cancer

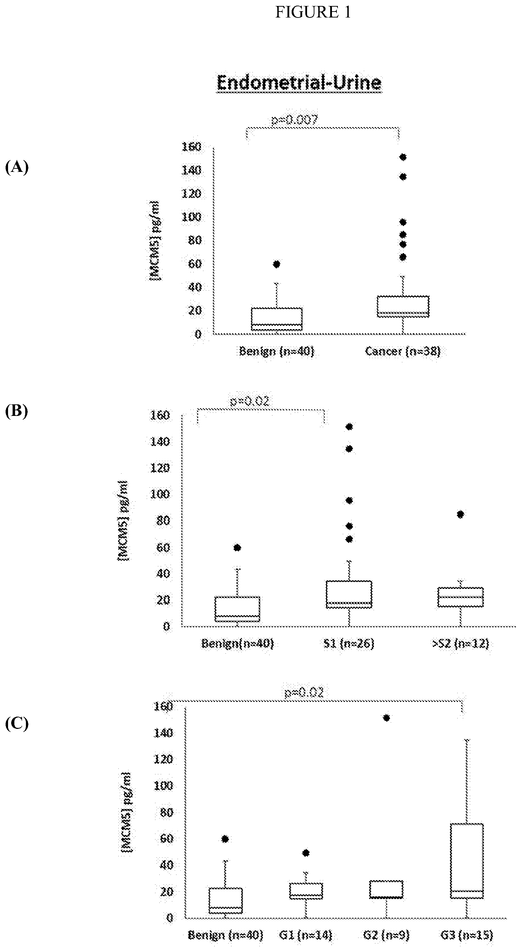

The MCM5 ELISA was capable of detecting MCM5 positive cells in the urine, vaginal swabs and vaginal tampons of patients with endometrial cancer with a high sensitivity. For urine samples the MCM5 ELISA had a sensitivity of 87 % with a specificity of 60 %, at the Youdens Index cut-off (Table 1). In addition, levels of MCM5 were significantly higher in urine from patients with endometrial cancer vs normal urines (Figure 1A; p=0.007). Furthermore, there was a significantly higher level of MCM5 expression in Stage 1 cancers vs Normals (Figure 1B; p=0.02) and in Grade 3 cancers vs Normal (Figure 1C; p= 0.02).

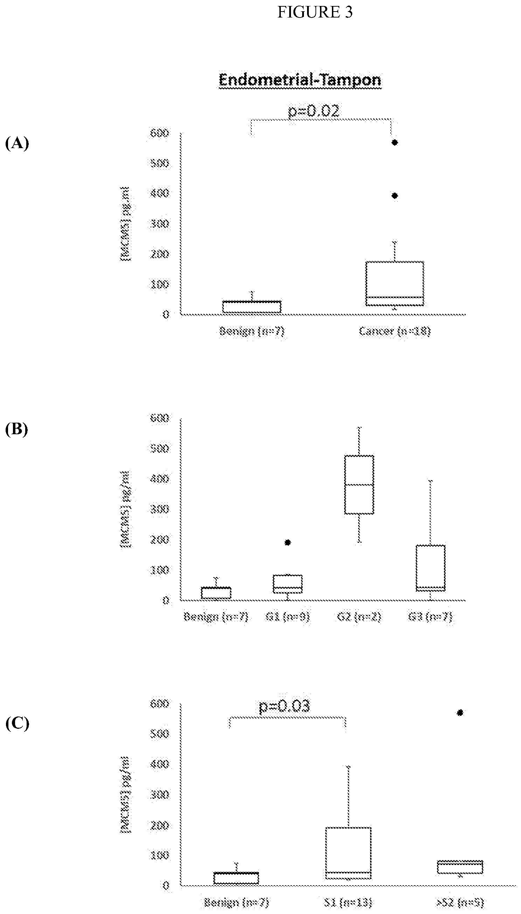

The MCM5 ELISA test had a 74 % sensitivity for the detection of endometrial tumours when applied to vaginal swab samples, with a specificity of 75 % at the Youdens Index cut-off. For vaginal tampon samples however, the Youdens index cut-off gave a sensitivity for the MCM5 ELISA of 100 %, but with a lower specificity of 43 %, a second Youdens Index point on the ROC curve gave a sensitivity of 50 % with a specificity of 86 %. MCM5 levels were found to be significantly higher for the vaginal tampon samples from patients with endometrial cancer than those with benign disease (Figure 3A; p=0.03).

**Table 1**

| Sensitivity and specificity for urine, vaginal swab and vaginal tampon samples, as calculated by the corresponding ROC curves (data not shown), with cut-off points calculated based on the Youdens Index. | | | |
|---|---|---|---|
| **Endometrial** | Urine | Swab | Tampon |
| **Sensitivity** | 87 % | 74 % | 100% (/50%) |
| **Specificity** | 60 % | 75 % | 43% (/86%) |

### Example 3 - Detection of Ovarian Cancer

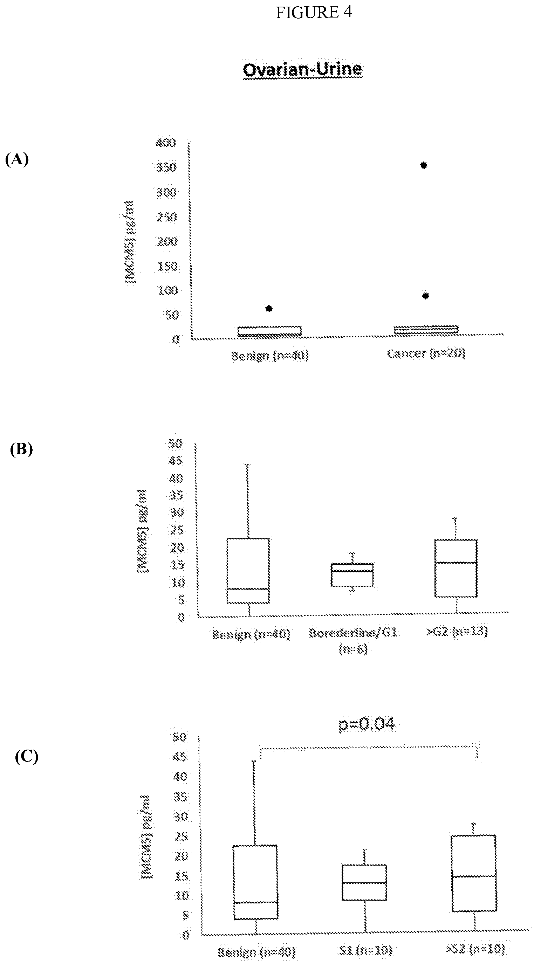

The MCM5 ELISA was capable of detecting MCM5 positive cells in urine samples, vaginal swabs samples and vaginal tampon samples from patients with ovarian cancer with high sensitivity. When applied to urine samples, the MCM5 ELISA had a sensitivity of 65 % with a specificity of 60 %, at the Youdens Index cut-off (Table 2). In addition, levels of MCM5 were higher in urine from patients with ovarian cancer vs normal urines (Figure 4A), with a significantly higher level of MCM5 expression in Stage 2 ovarian cancers vs Normals (Figure 4C; p= 0.04).

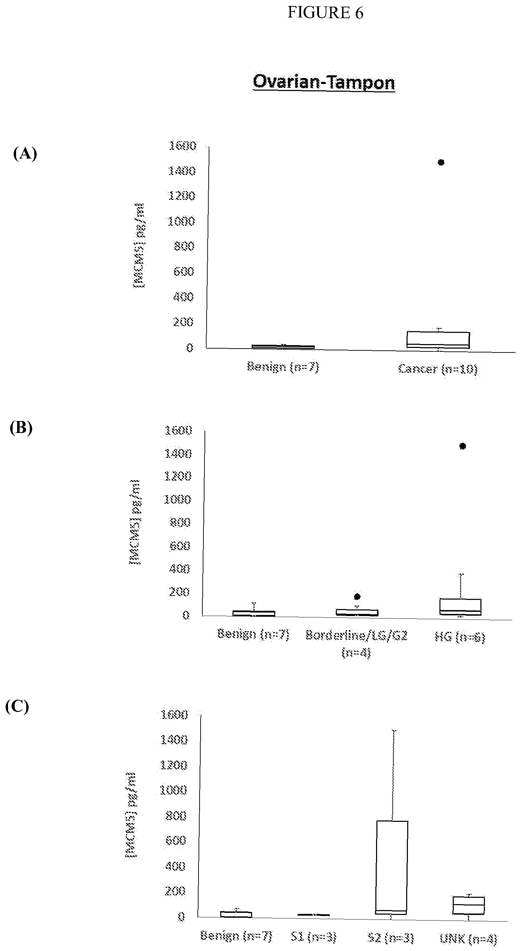

The MCM5 ELISA test also had an 85 % sensitivity for detection of ovarian cancer in vaginal swab samples, but with a low specificity of 25 % at the Youdens Index cut-off. For vaginal tampon samples, the Youdens index cut-off gave a sensitivity for the MCM5 ELISA of 90 %, however, specificity was lower at 43 %, a second Youdens Index point on the ROC curve gave a sensitivity of 50 % with a specificity of 86 %. MCM5 levels were also found to be higher in vaginal tampon samples from patients with ovarian cancer as compared to individuals with benign disease (Figure 6A).

**Table 2**

| Sensitivity and specificity for urine, vaginal swab and vaginal tampon samples, as calculated by the corresponding ROC curves (data not shown), with cut-off points calculated based on the Youdens Index. | | | |
|---|---|---|---|
| **Ovarian** | Urine | Swab | Tampon |
| **Sensitivity** | 65 % | 85 % | 90 % (/50%) |
| **Specificity** | 60 % | 25 % | 43 % (/86%) |

### Example 4 - Detection of Gynaecological Cancer

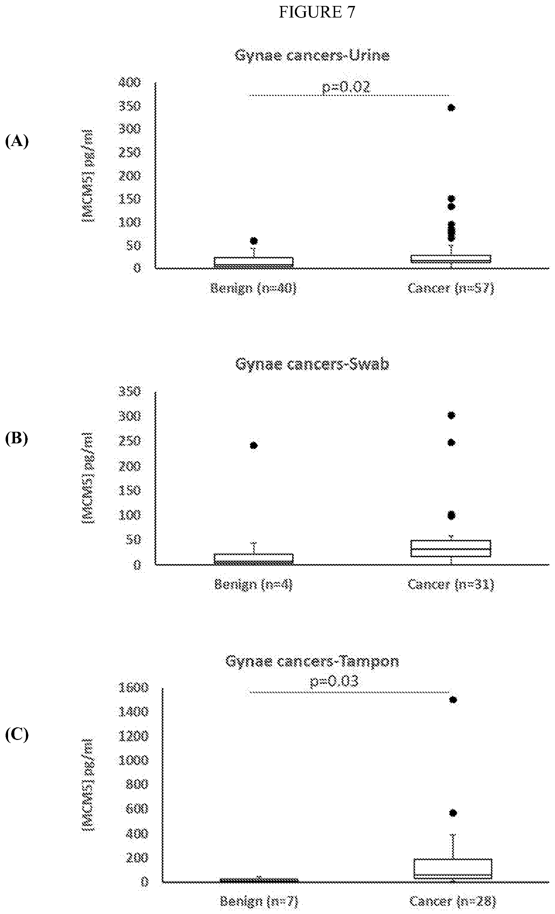

The MCM5 ELISA was capable of detecting MCM5 positive cells in urine samples, vaginal swab samples and vaginal tampon samples from patients with gynaecological cancers with a very high sensitivity. Applied to urine samples, the MCM5 ELISA had a sensitivity of 81 % with a specificity of 60 %, at the Youdens Index cut-off (Table 3). Furthermore, levels of MCM5 were significantly higher in urine from patients with a gynaecological cancer as compared to normal individuals (Figure 7A; p=0.02).

The MCM5 ELISA test had a 61 % sensitivity for detection of a gynaecological caner when applied to vaginal swab samples, with a specificity of 75 % at the Youdens Index cut-off.

For vaginal tampon samples, the Youdens index cut-off gave a sensitivity for the MCM5 ELISA of 90 %, with a specificity of 43 %, a second Youdens Index point on the ROC curve gave a sensitivity of 50% with a specificity of 86 %. MCM5 levels were found to be significantly higher in the vaginal tampons from patients with a gynaecological tumour than those with benign disease (Figure 7C; p=0.03).

**Table 3**

| Sensitivity and specificity for urine, vaginal swab and vaginal tampon samples, as calculated by the corresponding ROC curves (data not shown), with cut-off points calculated based on the Youdens Index. | | | |
|---|---|---|---|
| **Gynaecological** | Urine | Swab | Tampon |
| **Sensitivity** | 81% | 61% | 96% (/50%) |
| **Specificity** | 60% | 75% | 43% (/86%) |

In summary, these results demonstrate that the detection of gynaecological cancers using non-invasive samples has potential as both a diagnostic test in asymptomatic population, or as a screening tool to identify these cancers in an asymptomatic population, thereby enabling earlier diagnosis and treatment, which is known to improve survival in gynaecological cancers.

### Example 5 - Effect of benign gynaecological conditions upon the diagnostic accuracy of the MCM5 ELISA for the detection of gynaecological cancers

### Study population

Patients with known benign gynaecological conditions commonly found in the population, namely endometriosis, fibroids, polycystic ovary syndrome (PCOS) and post-menopausal bleeding (PMB) were recruited for a study designed to determine if the presence of a common benign condition impacted upon the specificity of the MCM5 ELISA test. Ethical approval was obtained from South Central Oxford B Research Ethics Committee (16/SC/0643-substantial amendment 1) and recruitment of patients took place at St Mary's Hospital, Manchester, between September 2018 and July 2019. Informed consent was obtained from all patients prior to the collection of urine and/or tampon samples. All eligible patients with a known benign condition listed above were enrolled. Patients were excluded if they were *virgo intacta*, if they had a previous diagnosis of bladder or renal cancer, if the patient had undergone any urological instrumentation in the preceding 2 weeks or if the patient was currently receiving chemotherapy or radiotherapy. Patients were asked to provide a full void urine sample.

In addition to the benign conditions samples, additional samples were also collected from patients with known ovarian or endometrial cancer as described in Examples 1-4. Urine samples were processed as described in Example 1 above. The MCM5 ELISA was performed as described in Example 1 above.

### Results

### Endometrial cancer

The MCM5 ELISA was capable of detecting MCM5 positive cells in urine samples obtained from patients with endometrial cancer with high sensitivity. The MCM5 ELISA had a sensitivity of 86.1 % (95 % CI: 72.1 % to 94.7 %) with a specificity of 74.6 % (95 % CI: 61.6 % to 85.0 %), at the identified cut-off of 12 pg/mL (see Table 4 below).

**Table 4 - sensitivity and specificity for the MCM5 ELISA applied to urine samples from endometrial cancer patients, as calculated by the corresponding ROC curve (data not shown)**

| **Endometrial** | Urine |
|---|---|
| **Sensitivity** | 86.1 % |
| **Specificity** | 74.6 % |

In addition, levels of MCM5 were significantly higher in urine from patients with endometrial cancer compared urine from patients with benign gynaecological conditions (p<0.0001, Figure 8A). Importantly, both low grade and early stage endometrial cancers demonstrated a statistically significant increased level of MCM5 detection (Figure 8B and C, respectively). In particular, the MCM5 ELISA was capable of detecting MCM5 positive cells in the urine of low grade endometrial cancers with a sensitivity of 83.3 % (95 % CI: 51.6 % to 97.9 %) and in Stage 1 cancers with a sensitivity of 89.2 % (95% CI: 71.8 % to 97.7 %). Thus, detection of MCM5 in urine samples demonstrated very high sensitivity for low grade (83.3 %) and early stage (89.2 %) endometrial cancers.

### Ovarian cancer

The MCM5 ELISA was capable of detecting MCM5 positive cells in urine samples from patients with ovarian cancer with high sensitivity. The MCM5 ELISA had a sensitivity of 61.5 % (95 % CI: 40.6 % to 79.8 %) with a specificity of 74.6 % (95 % CI: 61.6 % to 85.0 %) at the identified cut-off of 12 pg/mL (see Table 5 below). In addition, levels of MCM5 were significantly higher in urine samples from patients with ovarian cancer as compared to patients with common benign gynaecological conditions (p=0.02; Figure 9).

**Table 5 - sensitivity and specificity for the MCM5 ELISA applied to urine samples from ovarian cancer patients, as calculated by the corresponding ROC curve (data not shown)**

| **Ovarian** | **Urine** |
|---|---|
| **Sensitivity** | 61.5% |
| **Specificity** | 74.6% |

The MCM5 ELISA was also capable of detecting MCM5 positive cells in the urine of low grade ovarian cancers with a sensitivity of 71.4 % (95 % CI: 29.0 % to 96.3 %) and in Stage 1 cancers with a sensitivity of 70.0 % (95 % CI: 34.8 % to 93.3 %). Thus, detection of MCM5 in urine samples demonstrated very high sensitivity for low grade (71.4 %) and early stage (70 %) ovarian cancer tumours.

### Benign gynaecological conditions

Urine samples from patients with benign gynaecological conditions showed statistically significantly lower MCM5 levels when compared to the MCM5 levels in urine samples from patients with endometrial cancer (Figure 10). Thus, most common benign gynaecological conditions, namely endometriosis, fibroids, polycystic ovary syndrome (PCOS) and post-menopausal bleeding (PMB) do not have any significant effect of the diagnostic accuracy of the MCM5 ELISA when applied to urine samples: endometriosis (p<0.0001), PCOS (p=0.03), fibroids (p=0.0005) and PMB (p=0.003)).

**Table 4 - Sequences**

| **SEQ ID NO** | **Nucleotide/Polypeptide** |
|---|---|
| 1 | WDETKGE (epitope to which antibody 12A7 binds) |
| 2 | DDRVAIH (epitope to which antibody 4B4 binds |
| 3 | 12A7 light chain CDR 1 polypeptide sequence |
| 4 | 12A7 light chain CDR 1 nucleotide sequence |
| 5 | 12A7 light chain CDR 2 polypeptide sequence |
| 6 | 12A7 light chain CDR 2 nucleotide sequence |
| 7 | 12A7 light chain CDR 3 polypeptide sequence |
| 8 | 12A7 light chain CDR 3 nucleotide sequence |
| 9 | 12A7 heavy chain CDR 1 polypeptide sequence |
| 10 | 12A7 heavy chain CDR 1 nucleotide sequence |
| 11 | 12A7 heavy chain CDR 2 polypeptide sequence |
| 12 | 12A7 heavy chain CDR 2 nucleotide sequence |
| 13 | 12A7 heavy chain CDR 3 polypeptide sequence |
| 14 | 12A7 heavy chain CDR 3 nucleotide sequence |
| 15 | 4B4 light chain CDR 1 polypeptide sequence |
| 16 | 4B4 light chain CDR 1 nucleotide sequence |
| 17 | 4B4 light chain CDR 2 polypeptide sequence |
| 18 | 4B4 light chain CDR 2 nucleotide sequence |
| 19 | 4B4 light chain CDR 3 polypeptide sequence |
| 20 | 4B4 light chain CDR 3 nucleotide sequence |
| 21 | 4B4 heavy chain CDR 1 polypeptide sequence |
| 22 | 4B4 heavy chain CDR 1 nucleotide sequence |
| 23 | 4B4 heavy chain CDR 2 polypeptide sequence |
| 24 | 4B4 heavy chain CDR 2 nucleotide sequence |
| 25 | 4B4 heavy chain CDR 3 polypeptide sequence |
| 26 | 4B4 heavy chain CDR 3 nucleotide sequence |
| 27 | 12A7 full light chain variable region sequence (polypeptide) |
| 28 | 12A7 full light chain variable region sequence (nucleotide) |
| 29 | 12A7 full heavy chain variable region sequence (polypeptide) |
| 30 | 12A7 full heavy chain variable region sequence (nucleotide) |
| 31 | 4B4 full light chain variable region sequence (polypeptide) |
| 32 | 4B4 full light chain variable region sequence (nucleotide) variable region |
| 33 | 4B4 full heavy chain variable region sequence (polypeptide) |
| 34 | 4B4 full heavy chain variable region sequence (nucleotide) |
| 35 | MCM5 polypeptide sequence |
| 36 | MCM5 polynucleotide sequence |

### Sequence Listing

SEQ ID NO: 1
   WDETKGE
SEQ ID NO: 2
   DDRVAIH
SEQ ID NO: 3
   QNLVQSNGNTY
SEQ ID NO: 4
   CAGAACCTTGTACAAAGTAATGGAAACACCTATTTA
SEQ ID NO: 5
   KVS
SEQ ID NO: 6
   AAGTTTCCAA
SEQ ID NO: 7
   SQSTRVPYT
SEQ ID NO: 8
   TCTCAAAGTACACGTGTTCCGTACACA
SEQ ID NO: 9
   GFSLSTSGMG
SEQ ID NO: 10
   GGGTTTTCACTGAGCACTTCTGGTATGGGT
SEQ ID NO: 11
   IFWDDDK
SEQ ID NO: 12
   ATTTTCTGGGATGATGACAAG
SEQ ID NO: 13
   ARRSDYNYYSMDY
SEQ ID NO: 14
   GCGCGGCGAAGTGACTACAATTACTACTCTATGGACTAC
SEQ ID NO: 15
   QDIGSS
SEQ ID NO: 16
   CAGGACATTGGTAGTAGC
SEQ ID NO: 17
   ATS
SEQ ID NO: 18
   GCCACATCC
SEQ ID NO: 19
   LQYASSPPT
SEQ ID NO: 20
   CTACAATATGCTAGTTCTCCTCCGACG
SEQ ID NO: 21
   GFTFSNYA
SEQ ID NO: 22
   GGATTCACTTTCAGTAACTATGCC
SEQ ID NO: 23
   ISRGGSYT
SEQ ID NO: 24
   ATTAGTCGTGGTGGTAGTTACACC
SEQ ID NO: 25
   ARHGYNYDDGAWFAN
SEQ ID NO: 26
   GCAAGACATGGATATAATTACGACGACGGGGCCTGGTTTGCTAAC
SEQ ID NO: 27 
DIMLTQSPLSLSVTLGDQASISCRSSQNLVQSNGNTYLTWYLQKPGQSPKVLINKVSNRFY
GVPDRFSGSGSGTDFTLRISRVEAEDLGIYFCSQSTRVPYTFGGGTKLEIRR
SEQ ID NO: 28 
GATATCATGCTGACCCAATCTCCACTCTCCCTGTCTGTCACTCTTGGAGATCAGGCCTC
CATCTCTTGCAGATCTAGTCAGAACCTTGTACAAAGTAATGGAAACACCTATTTAACTT
GGTACCTGCAGAAGCCAGGCCAGTCTCCAAAGGTCCTGATCAACAAAGTTTCCAACCG
ATTTTATGGGGTCCCAGACAGGTTCAGTGGCAGTGGATCAGGGACAGATTTCACACTC
AGGATCAGCAGAGTGGAGGCTGAGGATCTGGGAATTTATTTCTGCTCTCAAAGTACAC
GTGTTCCGTACACATTCGGAGGGGGGACCAAGCTGGAAATAAGACG
SEQ ID NO: 29 
QQDLQQSGPGILQPTQTLSLTCSFSGFSLSTSGMGVSWIRQSSNMGLEWLAHIFWDDDKRY
NPSLRSRLTLSKDTSSSQVFLMITSVSTADSATYYCARRSDYNYYSMDYWGQGTAVTVSS
SEQ ID NO: 30 
CAGCAAGATCTGCAGCAGTCTGGCCCTGGGATATTGCAGCCCACCCAGACCCTCAGTC
TGACTTGTTCTTTCTCTGGGTTTTCACTGAGCACTTCTGGTATGGGTGTGAGTTGGATTC
GTCAATCTTCAAATATGGGTCTGGAGTGGCTGGCACACATTTTCTGGGATGATGACAA
GCGCTATAATCCCTCCCTGAGGAGCCGACTCACGCTCTCCAAGGATACCTCCAGTAGC
CAGGTATTTCTCATGATCACCAGTGTGAGTACTGCAGATTCTGCCACATACTACTGTGC
GCGGCGAAGTGACTACAATTACTACTCTATGGACTACTGGGGTCAAGGAACCGCAGTC
ACCGTCTCCTCA
SEQ ID NO: 31 
DIMLTQSPSSLSASLGERVSLTCRASQDIGSSLNWLQQEPDGTIKRLIYATSSLDSGVPKRFS
GSRSGSDYSLTISSLESEDFVDYYCLQYASSPPTFGGGTKLEIK
SEQ ID NO: 32 
GATATCATGCTGACCCAATCTCCATCCTCCTTATCTGCCTCTCTGGGAGAAAGAGTCAG
TCTCACTTGTCGGGCAAGTCAGGACATTGGTAGTAGCTTAAACTGGCTTCAACAGGAA
CCAGATGGAACTATTAAACGCCTAATCTACGCCACATCCAGTTTAGATTCTGGTGTCCC
CAAAAGGTTCAGTGGCAGTAGGTCTGGGTCAGATTATTCTCTCACCATCAGCAGCCTT
GAGTCTGAAGATTTTGTAGACTATTACTGTCTACAATATGCTAGTTCTCCTCCGACGTT
CGGTGGAGGCACCAAGCTGGAAATCAAAC
SEQ ID NO: 33 
VKLQESGGGLVKPGGSLKLSCAASGFTFSNYAMSWVRQNPEKRLEWVATISRGGSYTYY
PDSVKGRFTISRDNAKNTLYLQMNSLRSEDTAMYFCARHGYNYDDGAWFANWGQGTLV
TVSA
SEQ ID NO: 34 
TAGGTGAAACTGCAGGAGTCTGGGGGAGGCTTAGTGAAGCCTGGAGGGTCCCTGAAA
CTCTCCTGTGCAGCCTCTGGATTCACTTTCAGTAACTATGCCATGTCTTGGGTTCGCCA
GAATCCGGAGAAGAGGCTGGAGTGGGTCGCAACCATTAGTCGTGGTGGTAGTTACAC
CTACTATCCAGACAGTGTGAAGGGTCGATTCACCATCTCCAGAGACAATGCCAAGAAC
ACCCTGTATCTGCAAATGAACAGTCTGAGGTCTGAGGACACGGCCATGTATTTCTGTG
CAAGACATGGATATAATTACGACGACGGGGCCTGGTTTGCTAACTGGGGCCAAGGGA
CTCTGGTCACTGTCTCTGCA
SEQ ID NO: 35 
10 20 30 40 50 60
MSGFDDPGIF YSDSFGGDAQ ADEGQARKSQ LQRRFKEFLR QYRVGTDRTG FTFKYRDELK

70 80 90 100 110 120
RHYNLGEYWI EVEMEDLASF DEDLADYLYK QPAEHLQLLE EAAKEVADEV TRPRPSGEEV

130 140 150 160 170 180
LQDIQVMLKS DASPSSIRSL KSDMMSHLVK IPGIIIAASA VRAKATRISI QCRSCRNTLT

190 200 210 220 230 240
NIAMRPGLEG YALPRKCNTD QAGRPKCPLD PYFIMPDKCK CVDFQTLKLQ ELPDAVPHGE

250 260 270 280 290 300
MPRHMQLYCD RYLCDKVVPG NRVTIMGIYS IKKFGLTTSR GRDRVGVGIR SSYIRVLGIQ

310 320 330 340 350 360
VDTDGSGRSF AGAVSPQEEE EFRRLAALPN VYEVISKSIA PSIFGGTDMK KAIACLLFGG

370 380 390 400 410 420
SRKRLPDGLT RRGDINLLML GDPGTAKSQL LKFVEKCSPI GVYTSGKGSS AAGLTASVMR

430 440 450 460 470 480
DPSSRNFIME GGAMVLADGG VVCIDEFDKM REDDRVAIHE AMEQQTISIA KAGITTTLNS

490 500 510 520 530 540
RCSVLAAANS VFGRWDETKG EDNIDFMPTI LSRFDMIFIV KDEHNEERDV MLAKHVITLH

550 560 570 580 590 600
VSALTQTQAV EGEIDLAKLK KFIAYCRVKC GPRLSAEAAE KLKNRYIIMR SGARQHERDS

610 620 630 640 650 660
DRRSSIPITV RQLEAIVRIA EALSKMKLQP FATEADVEEA LRLFQVSTLD AALSGTLSGV
 
        670         680         690         700         710         720
EGFTSQEDQE MLSRIEKQLK RRFAIGSQVS EHSIIKDFTK QKYPEHAIHK VLQLMLRRGE

        730
IQHRMQRKVL YRLK
SEQ ID NO: 36
   Homo sapiens minichromosome maintenance complex component 5 (MCM5), mRNA NCBI Reference Sequence: NM_006739.3

1 ggaaaaccag aggcgcagtc atgtcgggat tcgacgatcc tggcattttc tacagcgaca
61 gcttcggggg cgacgcccag gccgacgagg ggcaggcccg caaatcgcag ctgcagaggc
121 gcttcaagga gttcctgcgg cggtaccgag tgggcaccga ccgcacgggc ttcaccttca
181 aatacaggga tgaactcaag cggcattaca acctggggga gtactggatt gaggtggaga
241 tggaggatct ggccagcttt gatgaggacc tggccgacta cttgtacaag cagccagccg
301 agcacctgca gctgctggag gaagctgcca aggaggtagc tgatgaggtg acccggcccc
361 ggccttctgg ggaggaggtg ctccaggaca tccaggtcat gctcaagtcg gacgccagcc
421 cttccagcat tcgtagcctg aagtcggaca tgatgtcaca cctggtgaag atccctggca
481 tcatcatcgc ggcctctgcg gtccgtgcca aggccacccg catctctatc cagtgccgca
541 gctgccgcaa caccctcacc aacattgcca tgcgccctgg cctcgagggc tatgccctgc
601 ccaggaagtg caacacagat caggctgggc gccccaaatg cccattggac ccgtacttca
661 tcatgcccga caaatgcaaa tgcgtggact tccagaccct gaagctgcag gagctgcctg
721 atgcagtccc ccacggggag atgcccagac acatgcagct ctactgcgac aggtacctgt
781 gtgacaaggt cgtccctggg aacagggtta ccatcatggg catctactcc atcaagaagt
841 ttggcctgac taccagcagg ggccgtgaca gggtgggcgt gggcatccga agctcctaca
901 tccgtgtcct gggcatccag gtggacacag atggctctgg ccgcagcttt gctggggccg
961 tgagccccca ggaggaggag gagttccgtc gcctggctgc cctcccaaat gtctatgagg
1021 tcatctccaa gagcatcgcc ccctccatct ttgggggcac agacatgaag aaggccattg
1081 cctgcctgct ctttgggggc tcccgaaaga ggctccctga tggacttact cgccgaggag
1141 acatcaacct gctgatgcta ggggaccctg ggacagccaa gtcccagctt ctgaagtttg
1201 tggagaagtg ttctcccatt ggggtataca cgtctgggaa aggcagcagc gcagctggac
1261 tgacagcctc ggtgatgagg gacccttcgt cccggaattt catcatggag ggcggagcca
1321 tggtcctggc cgatggtggg gtcgtctgta ttgacgagtt tgacaagatg cgagaagatg
1381 accgtgtggc aatccacgaa gccatggagc agcagaccat ctctatcgcc aaggctggga
1441 tcaccaccac cctgaactcc cgctgctccg tcctggctgc tgccaactca gtgttcggcc
1501 gctgggatga gacgaagggg gaggacaaca ttgacttcat gcccaccatc ttgtcgcgct
1561 tcgacatgat cttcatcgtc aaggatgagc acaatgagga gagggatgtg atgctggcca
1621 agcatgtcat cactctgcac gtgagcgcac tgacacagac acaggctgtg gagggcgaga
1681 ttgacctggc caagctgaag aagtttattg cctactgccg agtgaagtgt ggcccccggc
1741 tgtcagcaga ggctgcagag aaactgaaga accgctacat catcatgcgg agcgggcccc
1801 gtcagcacga gagggacagt gaccgccgct ccagcatccc catcactgtg cggcagctgg
1861 aggccattgt gcgcatcgcg gaagccctca gcaagatgaa gctgcagccc ttcgccacag
1921 aggcagatgt ggaggaggcc ctgcggctct tccaagtgtc cacgttggat gctgccttgt
1981 ccggtaccct gtcaggggtg gagggcttca ccagccagga ggaccaggag atgctgagcc
2041 gcatcgagaa gcagctcaag cgccgctttg ccattggctc ccaggtgtct gagcacagca
2101 tcatcaagga cttcaccaag cagaaatacc cggagcacgc catccacaag gtgctgcagc
2161 tcatgctgcg gcgcggcgag atccagcatc gcatgcagcg caaggttctc taccgcctca
2221 agtgagtcgc gccgcctcac tggactcatg gactcgccca cgcctcgccc ctcctgccgc
2281 tgcctgccat tgacaatgtt gctgggacct ctgcctcccc actgcagccc tcgaacttcc
2341 caggcaccct cctttctgcc ccagaggaag gagctgtagt gtcctgctgc ctctgggcgc
2401 ccgcctctag cgcggttctg ggaagtgtgc ttttggcatc cgttaataat aaagccacgg
2461 tgtgttcagg taaaaaaaaa aaaaaaaaaa aaaaaaaaaa aaaaaaaaaa aaaaaaaaaa
2521 aaaaaaaaaa aaaa

## Claims

1. An *in vitro* method for detecting the presence or absence of a gynaecological cancer in a subject, the method comprising steps of:
obtaining a urine, tampon or vaginal swab sample isolated from the subject; and
detecting minichromosome maintenance complex component 5 (MCM5) or determining the concentration of MCM5;
wherein the step of detecting MCM5 or determining the concentration of MCM5 comprises performing an ELISA assay to detect MCM5 or determine the concentration of MCM5.

2. The method of claim 1, further comprising a step of:
comparing the concentration of MCM5 determined to a reference, preferably wherein the reference is an average concentration of MCM5 determined for one or more samples prepared from one or more healthy individuals, optionally wherein:
(i) a gynaecological cancer is likely to be present if the concentration of MCM5 is abnormal compared to the reference; and/or
(ii) a gynaecological cancer is likely to be present if the concentration of MCM5 is higher than the reference;

3. The method of any one of the preceding claims, wherein:
(a) the gynaecological cancer is likely to be present if the concentration of MCM5 is determined to be higher than 5 pg /mL, 6 pg/mL, 7 pg/mL, 8 pg/mL, 9 pg/mL, 10 pg/mL, 11 pg/mL, 12 pg/mL, 13 pg/mL, 14 pg/mL, 15 pg/mL, 16 pg/mL, 17 pg/mL, 18 pg/mL, 19 pg/mL, 20 pg/mL, 21 pg/mL, 22 pg/mL, 23 pg/mL, 24 pg/mL, 25 pg/mL, 26 pg/mL, 27 pg/mL, 28 pg/mL, 29 pg/mL, 30 pg/mL, 35 pg/mL, 45 pg/mL, 50 pg/mL, 55 pg/mL, 60 pg/mL, 65 pg/mL, or 70 pg/mL;
(b) the gynaecological cancer is likely to be present if the concentration of MCM5 is determined to be higher than (i) about 12 pg/mL; (ii) about 17 pg/mL; or (iii) about 70 pg/mL; preferably about 12 pg/mL; and/or
(c) a 12 pg/mL, 17 pg/mL or 70 pg/mL, preferably about 12 pg/mL, cut-off is applied.

4. The method of any one of the preceding claims, wherein the method is a method for diagnosing a gynaecological cancer in a subject, optionally wherein the method further comprises a step of:
diagnosing the subject as having a gynaecological cancer if a gynaecological cancer is likely to be present.

5. An *in vitro* method for:
(a) diagnosing a subject as having a gynaecological cancer or a benign gynaecological condition, the method comprising the steps of:
providing a urine, tampon or vaginal swab sample previously isolated from the subject;
determining the concentration of MCM5 in the urine, tampon or vaginal swab sample,
wherein the step of determining the concentration of MCM5 comprises performing an ELISA assay to detect MCM5 or determine the concentration of MCM5;
comparing the concentration of MCM5 in the urine, tampon or vaginal swab sample to a reference;
diagnosing the subject as having a gynaecological cancer if the concentration of MCM5 in the urine, tampon or vaginal swab sample is higher than the reference or diagnosing the subject as having a benign gynaecological condition if the concentration of MCM5 in the urine, tampon or vaginal swab sample is lower than the reference;
(b) distinguishing between a urine, tampon or vaginal swab sample associated with a gynaecological cancer and a urine, tampon or vaginal swab sample associated with a benign gynaecological cancer, the method comprising the steps of:
providing a urine, tampon or vaginal swab sample previously isolated from a subject;
determining the concentration of MCM5 in the urine, tampon or vaginal swab sample, wherein the step of determining the concentration of MCM5 comprises performing an ELISA assay to detect MCM5 or determine the concentration of MCM5;
comparing the concentration of MCM5 in the urine, tampon or vaginal swab sample to a reference;
determining that the urine, tampon or vaginal swab sample is associated with a gynaecological cancer if the concentration of MCM5 in the urine, tampon or vaginal swab sample is higher than the reference or determining that the urine, tampon or vaginal swab sample is associated with a benign gynaecological condition if the concentration of MCM5 in the urine, tampon or vaginal swab sample is lower than the reference; or
(c) stratifying a subject into a first or a second treatment group, the method comprising the steps of:
providing a urine, tampon or vaginal swab sample previously isolated from the subject;
determining the concentration of MCM5 in the urine, tampon or vaginal swab sample, wherein the step of determining the concentration of MCM5 comprises performing an ELISA assay to detect MCM5 or determine the concentration of MCM5;
allocating the subject to one of the two treatment groups based on the concentration of MCM5 in the urine, tampon or vaginal swab sample, wherein the first treatment group is to receive treatment for a gynaecological cancer and the second treatment group is to receive no treatment or treatment for a benign gynaecological condition; optionally wherein the subject is allocated to the first treatment group if the concentration of MCM5 in the urine, tampon or vaginal swab sample is higher than a reference or the subject is allocated to the second treatment group if the concentration of MCM5 in the urine, tampon or vaginal swab sample is lower than the reference.

6. The method of claim 4 or claim 5, wherein the benign gynaecological condition is selected from post-menopausal bleeding (PMB), endometriosis, fibroids, and polycystic ovary syndrome (PCOS).

7. The method of any one of claims 4 to 6, wherein the reference is about 12 pg/mL.

8. The method of any one of the preceding claims wherein the urine, tampon or vaginal swab sample is a urine sample,

9. The method of any one of the preceding claims, wherein said method further comprises a step of:
treating the urine, tampon or vaginal swab sample to release MCM5 from cells in the urine, tampon or vaginal swab sample;
optionally wherein the step of treating the urine, tampon or vaginal swab sample to release MCM5 comprises exposing the urine, tampon or vaginal swab sample to a lysis buffer capable of releasing MCM5 from cells in the urine, tampon or vaginal swab sample, optionally wherein the step of treating the urine, tampon or vaginal swab sample to release MCM5 comprises:
passing the urine, tampon or vaginal swab sample through a filter for capturing cells, such that cells are captured in the filter;
passing a lysis buffer through the filter, such that the captured cells are exposed to the lysis buffer; and/or
incubating the filter for a period of time, such that the lysis buffer causes the cells to release MCM5.

10. The method of claim 9, wherein:
(a) the lysis buffer is capable of releasing MCM5 from cells in the urine, tampon or vaginal swab sample;
(b) the lysis buffer is capable of releasing MCM5 from cells in the urine, tampon or vaginal swab sample and does not substantially denature MCM5 protein, optionally wherein the lysis buffer does not denature an antibody; and/or
(c)
(i) the lysis buffer comprises a detergent;
(ii) the lysis buffer comprises a detergent which comprises Triton X-100;
(iii) the lysis buffer comprises a detergent which comprises Triton X-100 at a concentration between 0.01 % and 25 %, between 0.01 % and 10 %, between 0.05 % and 5 %, between 0.1 % and 2 %, between 0.5 % and 2 %, between 0.75 % and 1.25 %, or about 1 %;
(iv) the lysis buffer comprises a detergent which comprises or consists of sodium deoxycholate;
(v) the lysis buffer comprises a detergent which comprises or consists of sodium deoxycholate at a concentration between 0.1 % and 20 %, between 0.1 and 10 %, between 0.1 and 5 %, between 0.5 % and 5 %, between 0.5 % and 2.5 %, between 0.75 % and 2.5 %, between 0.75 % and 1.25 %, or about 1 %;
(vi) the lysis buffer comprises a detergent which comprises or consists of sodium dodecyl sulphate (SDS);
(vii) the lysis buffer comprises a detergent which comprises or consists of sodium dodecyl sulphate (SDS) at a concentration between 0.001 % and 10 %, between 0.01 % and 5 %, between 0.05 % and 5 %, between 0.01 % and 1 %, between 0.05 % and 1 %, between 0.05 % and 0.5%, between 0.075% and 0.25 %, or about 0.1 %;
(viii) the lysis buffer comprises a detergent which consists of Triton X-100, sodium deoxycholate, and sodium dodecyl sulphate (SDS);
(ix) the lysis buffer comprises a detergent which consists of between 0.5 % and 2 % of Triton X-100, between 0.5 % and 2 % of sodium deoxycholate, and between 0.05 % and 0.5 % of sodium dodecyl sulphate (SDS);
(x) the lysis buffer comprises a buffer component;
(xi) the lysis buffer comprises a buffer component which has a pH of between pH 4 and pH 9, between pH 5 and pH 8.5, between pH 6 and pH 8, between pH 6.5 and pH 8, between pH 7 and pH 8, between pH 7.3 and pH 7.9, between pH 7.4 and pH 7.8, between pH 7.5 and pH 7.7, or about pH 7.6; and/or maintains the pH of the lysis buffer at between pH 4 and pH 9, between pH 5 and pH 8.5, between pH 6 and pH 8, between pH 6.5 and pH 8, between pH 7 and pH 8, between pH 7.3 and pH 7.9, between pH 7.4 and pH 7.8, between pH 7.5 and pH 7.7, or about pH 7.6;
(xii) the lysis buffer comprises a buffer component which comprises or consists of Tris;
(xiii) the lysis buffer comprises a buffer component which comprises or consists of Tris at a concentration greater than 1 mM, between 1 mM and 350 mM, between 5 and 200 mM, between 5 and 100 mM, between 5 and 50 mM, between 5 mM and 40 mM, between 5 mM and 35 mM, between 10 mM and 35 mM, between 15 mM and 35 mM, between 15 mM and 30 mM, between 20 mM and 30 mM, or about 25 mM;
(xiv) the lysis buffer comprises a buffer component which consists of Tris at a concentration of between 15 mM and 35 mM;
(xv) the lysis buffer comprises a salt;
(xvi) the lysis buffer comprises sodium chloride;
(xvii) the lysis buffer comprises sodium chloride at a concentration between 10 mM and 350 mM, between 20 mM and 300 mM, between 50 mM and 250 mM, between 100 mM and 250, between 100 mM and 200 mM, between 125 mM and 175 mM, or about 150 mM;
(xviii) the lysis buffer comprises sodium chloride at a concentration of between 100 mM and 200 mM;
(xix) the lysis buffer comprises:
a. between 1 mM and 100 mM Tris;
b. between 50 mM and 300 mM sodium chloride;
c. between 0.1 and 5 % sodium deoxycholate;
d. between 0.01 and 1 % sodium dodecyl sulphate; and/or
e. between 0.1 and 5 % Triton-X 100;
(xx) the lysis buffer comprises:
a. between 10 mM and 40 mM Tris;
b. between 100 mM and 200 mM sodium chloride;
c. between 0.5 and 2 % sodium deoxycholate;
d. between 0.05 and 0.5 % sodium dodecyl sulphate; and/or
e. between 0.5 and 2 % Triton-X 100; and/or
(xxi) the lysis buffer comprises:
a. about 25 mM Tris;
b. about 150 mM sodium chloride;
c. about 1 % sodium deoxycholate;
d. about 0.1 % sodium dodecyl sulphate; and/or
e. about 1 % Triton-X100.

11. The method of any one of the preceding claims, wherein the step of detecting MCM5 or determining the concentration of MCM5 comprises:
exposing the urine, tampon or vaginal swab sample to a first monoclonal antibody and/or a second monoclonal antibody; and
detecting MCM5 bound to the first monoclonal antibody and/or the second monoclonal antibody or determining the concentration of MCM5 bound to the first monoclonal antibody and/or the second monoclonal antibody.

12. The method of claim 11, wherein:
(a) the first monoclonal antibody and the second monoclonal antibody bind to MCM5, optionally wherein the first monoclonal antibody is an antibody which:
(i) binds to a polypeptide having an amino acid sequence of SEQ ID NO: 1;
(ii) comprises at least one Complementary Determining Region (CDR) selected from the group consisting of:
a. 12A7 CDRH1 which has a sequence of SEQ ID NO: 9 or a sequence that differs from SEQ ID NO:9 by a single amino acid substitution;
b. 12A7 CDRH2 which has a sequence of SEQ ID NO: 11 or a sequence that differs from SEQ ID NO: 11 by a single amino acid substitution;
c. 12A7 CDRH3 which has a sequence of SEQ ID NO: 13 or a sequence that differs from SEQ ID NO: 13 by a single amino acid substitution;
d. 12A7 CDRL1 which has a sequence of SEQ ID NO: 3 or a sequence that differs from SEQ ID NO:3 by a single amino acid substitution;
e. 12A7 CDRL2 which has a sequence of SEQ ID NO: 5 or a sequence that differs from SEQ ID NO:5 by a single amino acid substitution; and
f. 12A7 CDRL3 which has a sequence of SEQ ID NO: 7 or a sequence that differs from SEQ ID NO:7 by a single amino acid substitution;
(iii) comprises a heavy chain variable region having a sequence at least 85%, 90%, 95%, 98%, 99%, or 100% identical to SEQ ID NO: 29;
(iv) comprises a light chain variable region sequence having a sequence at least 85%, 90%, 95%, 98%, 99%, or 100% identical to SEQ ID NO: 27; or
(v) competes with the antibody of (i), (ii), (iii), or (iv); and/or
the second monoclonal antibody is an antibody which:
(i) binds to a polypeptide having an amino acid sequence of SEQ ID NO: 2;
(ii) comprises at least one Complementary Determining Region (CDR) selected from the group consisting of:
a. 4B4 CDRH1 which has a sequence of SEQ ID NO: 21 or a sequence that differs from SEQ ID NO:21 by a single amino acid substitution;
b. 4B4 CDRH2 which has a sequence of SEQ ID NO: 23 or a sequence that differs from SEQ ID NO:23 by a single amino acid substitution;
c. 4B4 CDRH3 which has a sequence of SEQ ID NO: 25 or a sequence that differs from SEQ ID NO:25 by a single amino acid substitution;
d. 4B4 CDRL1 which has a sequence of SEQ ID NO: 15 or a sequence that differs from SEQ ID NO: 15 by a single amino acid substitution;
e. 4B4 CDRL2 which has a sequence of SEQ ID NO: 17 or a sequence that differs from SEQ ID NO: 17 by a single amino acid substitution; and
f. 4B4 CDRL3 which has a sequence of SEQ ID NO: 19 or a sequence that differs from SEQ ID NO: 19 by a single amino acid substitution;
(iii) comprises a heavy chain variable region having a sequence at least 85%, 90%, 95%, 98%, 99%, or 100% identical to SEQ ID NO: 33;
(iv) comprises a light chain variable region sequence having a sequence at least 85%, 90%, 95%, 98%, 99%, or 100% identical to SEQ ID NO: 31; or
(v) competes with the antibody of (i), (ii), (iii), or (iv); and/or
(b)
(i) the first monoclonal antibody and/or second monoclonal antibody has an affinity for MCM5 in the range of 0.001-1 nM;
(ii) the first monoclonal antibody and/or the second monoclonal antibody is a Fab'₂, a F'(ab)₂, an Fv, a single chain antibody or a diabody;
(iii) the first monoclonal antibody comprises 12A7 CDRH1, 12A7 CDRH2, and 12A7 CDRH3;
(iv) the first monoclonal antibody comprises 12A7 CDRL1, 12A7 CDRL2 and 12A7 CDRL3;
(v) the first monoclonal antibody comprises a 12A7 CDRH1 which has a sequence of SEQ ID NO: 9, a 12A7 CDRH2 which has a sequence of SEQ ID NO: 11, and a 12A7 CDRH3 which has a sequence of SEQ ID NO: 13;
(vi) the first monoclonal antibody comprises a 12A7 CDRL1 which has a sequence of SEQ ID NO: 3, a 12A7 CDRL2 which has a sequence of SEQ ID NO: 5, and a 12A7 CDRL3 which has a sequence of SEQ ID NO: 7;
(vii) the second monoclonal antibody comprises 4B4 CDRH1, 4B4 CDRH2, and 4B4 CDRH3;
(viii) the second monoclonal antibody comprises 4B4 CDRL1, 4B4 CDRL2 and 4B4 CDRL3;
(ix) the second monoclonal antibody comprises a 4B4 CDRH1 which has a sequence of SEQ ID NO: 21, a 4B4 CDRH2 which has a sequence of SEQ ID NO: 23, and a 4B4 CDRH3 which has a sequence of SEQ ID NO: 25;
(x) the second monoclonal antibody has a 4B4 CDRL1 which has a sequence of SEQ ID NO: 15, a 4B4 CDRL2 which has a sequence of SEQ ID NO: 17, and a 4B4 CDRL3 which has a sequence of SEQ ID NO: 19;
(xi) the first monoclonal antibody comprises a heavy chain variable region having a sequence at least 95 % identical to SEQ ID NO: 29;
(xii) the first monoclonal antibody comprises a heavy chain variable region having a sequence at least 98 % identical to SEQ ID NO: 29;
(xiii) the first monoclonal antibody comprises a light chain variable region having a sequence at least 95 % identical to SEQ ID NO: 27;
(xiv) the first monoclonal antibody comprises a light chain variable region having a sequence at least 98 % identical to SEQ ID NO: 27;
(xv) the second monoclonal antibody comprises a heavy chain variable region having a sequence at least 95 % identical to SEQ ID NO: 33;
(xvi) the second monoclonal antibody comprises a heavy chain variable region having a sequence at least 98 % identical to SEQ ID NO: 33;
(xvii) the second monoclonal antibody comprises a light chain variable region having a sequence at least 95 % identical to SEQ ID NO: 31; and/or
(xviii) the second monoclonal antibody comprises a light chain variable region having a sequence at least 98 % identical to SEQ ID NO: 31.

13. The method of any one of the preceding claims, wherein:
(a) the urine, tampon or vaginal swab sample is a urine sample, and the method has a higher sensitivity than an equivalent method performed using a swab sample and/or an equivalent method performed using a tampon sample; and/or
(b) the urine, tampon or vaginal swab sample is a urine sample, and the method has a higher specificity than an equivalent method performed using a swab sample and/or an equivalent method performed using a tampon sample.

14. The method of any one of the preceding claims, wherein:
(a)
(i) the method has a sensitivity for the gynaecological cancer of at least about 20 %, 22 %, 24 %, 26 %, 28 %, 30 %, 32 %, 34 %, 36 %, 38 %, 40 %, 42 %, 44 %, 46 %, 48 %, or 50 %;
(ii) the method has a sensitivity for the gynaecological cancer of at least about 50 %, 52 %, 54 %, 55 %, 56 %, 57 %, 58 %, 59 %, 60 %, or 61%;
(iii) the method has a sensitivity for the gynaecological cancer of at least about 75 %, 76 %, 77 %, 78 %, 79 %, 80 %, or 81 %;
(iv) the method has a sensitivity for the gynaecological cancer of at least about 90 %, 92 %, 93 %, 94 %, 95 %, or 96 %;
(v) the method has a specificity for the gynaecological cancer of at least about 20 %, 22 %, 24 %, 26 %, 28 %, 30 %, 32 %, 34 %, 36 %, 38 %, or 40 %;
(vi) the method has a specificity for the gynaecological cancer of at least about 50 %, 52 %, 54 %, 55 %, 56 %, 57 %, 58 %, 59 %, or 60 %;
(vii) the method has a specificity for the gynaecological cancer of at least about 60 %, 62 %, 64 %, 66 %, 68 %, 70 %, 71%, 72 %, 73 %, 74 %, or 75 %; and/or
(viii) the method has a specificity for the gynaecological cancer of at least about 80 %, 82 %, 83 %, 84 %, 85 %, or 86 %; or
(b)
(i) the urine, tampon or vaginal swab sample is a urine sample and the method has a sensitivity for the gynaecological cancer of at least about 75 % and a specificity for the gynaecological cancer of at least about 55 %;
(ii) the urine, tampon or vaginal swab sample is a vaginal swab sample and the method has a sensitivity for the gynaecological cancer of at least about 55 % and a specificity for the gynaecological cancer of at least about 70 %;
(iii) the urine, tampon or vaginal swab sample is a tampon sample and the method has a sensitivity for the gynaecological cancer of at least about 90 % and a specificity for the gynaecological cancer of at least about 35 %;
(iv) the urine, tampon or vaginal swab sample is a tampon sample and the method has a sensitivity for the gynaecological cancer of at least about 45 % and a specificity for the gynaecological cancer of at least about 80 %; or
(v) the urine, tampon or vaginal swab sample is a tampon sample and the method has a sensitivity for the gynaecological cancer of at least about 90 % and a specificity for the gynaecological cancer of at least about 80 %.

15. Use of a lysis buffer, or a first monoclonal antibody and/or a second monoclonal antibody, or a kit in a method of detecting the presence or absence of a gynaecological cancer in a subject according to any one of the preceding claims, wherein the kit comprises said lysis buffer, said first monoclonal antibody and/or said second monoclonal antibody.

16. The method of any one of claims 1-14 or the use according to claim 15, wherein:
(a) the gynaecological cancer is a gynaecological malignancy;
(b) the gynaecological cancer is selected from the group consisting of ovarian cancer, endometrial cancer, uterine cancer, cervical cancer, vulval cancer, vaginal cancer, fallopian tube tumour, epithelial ovarian tumour, ovarian teratoma, immature ovarian teratoma, stromal tumour, germ cell ovarian tumour, undifferentiated tumours, borderline ovarian tumour, endometrial carcinoma, endometrial adenocarcinoma, endometrioid adenocarcinoma, endometrial squamous cell carcinoma, serous carcinoma, serous endometrial intraepithelial carcinoma, endometrial carcinosarcoma, clear cell carcinoma, mucinous carcinoma, undifferentiated endometrial carcinoma, mixed endometrial carcinoma, mucinous tumour, malignant mixed Müllerian tumour, endometrial clear cell sarcoma, granulosa cell tumour, serous tubal intraepithelial carcinoma, immature cystic teratoma, serous ovarian tumour, and small cell carcinoma;
(c) the gynaecological cancer is an ovarian cancer or an endometrial cancer;
(d) the gynaecological cancer is an ovarian cancer, optionally wherein the ovarian cancer is selected from the group consisting of epithelial ovarian tumour, ovarian teratoma, immature ovarian teratoma, stromal tumour, germ cell ovarian tumour, undifferentiated tumours, borderline ovarian tumour, mucinous tumour, granulosa cell tumour, immature cystic teratoma, serous ovarian tumour, and small cell carcinoma;
(e) the gynaecological cancer is an endometrial cancer, optionally wherein the endometrial cancer is selected from the group consisting of endometrial carcinoma, endometrial adenocarcinoma, endometrioid adenocarcinoma, endometrial squamous cell carcinoma, serous carcinoma, serous endometrial intraepithelial carcinoma, endometrial carcinosarcoma, clear cell carcinoma, mucinous carcinoma, undifferentiated endometrial carcinoma, mixed endometrial carcinoma, malignant mixed Müllerian tumour, and endometrial clear cell sarcoma; and/or
(f) the gynaecological cancer is:
(i) a low grade cancer, optionally a G1 cancer; or
(ii) a high grade cancer, optionally a G2 or above cancer; and/or
(g) the gynaecological cancer is:
(i) an early stage cancer, optionally an S 1 cancer; or
(ii) a late stage cancer, optionally an S2 or above cancer.

17. The method or use of any one of the preceding claims, wherein:
(a)
(i) the gynaecological cancer is an ovarian cancer and the method has a specificity for the ovarian cancer of at least about 25 %;
(ii) the gynaecological cancer is an ovarian cancer and the method has a sensitivity for the ovarian cancer of at least about 50 %;
(iii) the gynaecological cancer is an ovarian cancer and the method has a specificity for the ovarian cancer of at least about 25 % and a sensitivity for the ovarian cancer of at least about 50 %;
(iv) the gynaecological cancer is an endometrial cancer and the method has a specificity for the endometrial cancer of at least about 40 %;
(v) the gynaecological cancer is an endometrial cancer and the method has a sensitivity for the endometrial cancer of at least about 80 %; and/or
(vi) the gynaecological cancer is an endometrial cancer and the method has a specificity for the endometrial cancer of at least about 40 % and a sensitivity for the endometrial cancer of at least about 80 %;
(b)
(i) the urine, tampon or vaginal swab sample is a urine sample, the gynaecological cancer is an ovarian cancer, and the method has a sensitivity for the ovarian cancer of at least about 60 % and a specificity for the ovarian cancer of at least about 70 %, optionally wherein an MCM5 concentration reference of about 12 pg/ml is applied; or
(ii) the urine, tampon or vaginal swab sample is a urine sample, the gynaecological cancer is an endometrial cancer, and the method has a sensitivity for the endometrial cancer of at least about 80 % and a specificity for the endometrial cancer of at least about 70 %, optionally wherein an MCM5 concentration reference of about 12 pg/ml is applied; or
(c)
(i) the urine, tampon or vaginal swab sample is a urine sample, the gynaecological cancer is a low grade ovarian cancer and the method has a sensitivity for the low grade ovarian cancer of at least about 70 %, optionally wherein an MCM5 concentration reference of about 12 pg/ml is applied;
(ii) the urine, tampon or vaginal swab sample is a urine sample, the gynaecological cancer is an early stage ovarian cancer and the method has a sensitivity for the early stage ovarian cancer of at least about 70%, optionally wherein an MCM5 concentration reference of about 12 pg/ml is applied;
(iii) the urine, tampon or vaginal swab sample is a urine sample, the gynaecological cancer is a low grade endometrial cancer and the method has a sensitivity for the low grade endometrial cancer of at least about 80 %, optionally wherein an MCM5 concentration reference of about 12 pg/ml is applied; or
(iv) the urine, tampon or vaginal swab sample is a urine sample, the gynaecological cancer is an early stage endometrial cancer and the method has a sensitivity for the early stage endometrial cancer of at least about 85 %, optionally wherein an MCM5 concentration reference of about 12 pg/ml is applied.

18. The method or use of any one of the preceding claims, wherein the subject is suffering from a benign gynaecological condition, optionally wherein the benign gynaecological condition is selected from post-menopausal bleeding (PMB), endometriosis, fibroids, and polycystic ovary syndrome (PCOS).

## Patentansprüche

1. In-vitro-Verfahren zum Nachweis des Vorhandenseins oder der Abwesenheit eines gynäkologischen Krebses in einem Patienten, wobei das Verfahren folgende Schritte umfasst:
Gewinnen einer Urin-, Tampon- oder Vaginalabstrichprobe, die von dem Patienten isoliert wurde; und
Nachweisen der Minichromosomenerhaltungskomplexkomponente 5 (Minichromosome Maintenance Complex Component 5, MCM5) oder Bestimmen der Konzentration von MCM5;
wobei der Schritt des Nachweisens von MCM5 oder des Bestimmens der Konzentration von MCM5 das Durchführen eines ELISA-Assays zum Nachweis von MCM5 oder zur Bestimmung der Konzentration von MCM5 umfasst.

2. Verfahren nach Anspruch 1, das ferner folgenden Schritt umfasst:
Vergleichen der ermittelten Konzentration von MCM5 mit einer Referenz, vorzugsweise wobei die Referenz eine durchschnittliche Konzentration von MCM5 ist, die für eine oder mehrere Proben bestimmt wurde, die von einer oder mehreren gesunden Personen hergestellt wurden, wobei optional:
(i) ein gynäkologischer Krebs wahrscheinlich vorliegt, wenn die Konzentration von MCM5 im Vergleich zur Referenz abnormal ist; und/oder
(ii) ein gynäkologischer Krebs wahrscheinlich vorliegt, wenn die Konzentration von MCM5 höher ist als die Referenzkonzentration;

3. Verfahren nach einem der vorhergehenden Ansprüche, wobei:
(a) ein gynäkologischer Krebs wahrscheinlich vorliegt, wenn die Konzentration von MCM5 höher bestimmt wird als 5 pg/ml, 6 pg/ml, 7 pg/ml, 8 pg/ml, 9 pg/ml, 10 pg/ml, 11 pg/ml, 12 pg/ml, 13 pg/ml, 14 pg/ml, 15 pg/ml, 16 pg/ml, 17 pg/ml, 18 pg/ml, 19 pg/ml, 20 pg/ml, 21 pg/ml, 22 pg/ml, 23 pg/ml, 24 pg/ml, 25 pg/ml, 26 pg/ml, 27 pg/ml, 28 pg/ml, 29 pg/ml, 30 pg/ml, 35 pg/ml, 45 pg/ml, 50 pg/ml, 55 pg/ml, 60 pg/ml, 65 pg/ml oder 70 pg/ml;
(b) ein gynäkologischer Krebs wahrscheinlich vorliegt, wenn die Konzentration von MCM5 höher ist als (i) etwa 12 pg/ml; (ii) etwa 17 pg/ml; oder (iii) etwa 70 pg/ml; vorzugsweise etwa 12 pg/ml; und/oder
(c) ein Grenzwert von 12 pg/ml, 17 pg/ml oder 70 pg/ml, vorzugsweise etwa 12 pg/ml, angewendet wird.

4. Verfahren nach einem der vorhergehenden Ansprüche, wobei das Verfahren ein Verfahren zur Diagnose eines gynäkologischen Krebses bei einem Patienten ist, wobei das Verfahren optional ferner folgenden Schritt umfasst:
Diagnostizieren von gynäkologischem Krebs bei dem Patienten, wenn ein gynäkologischer Krebs wahrscheinlich vorhanden ist.

5. *In-vitro*-Verfahren zum:
(a) Diagnostizieren eines Patienten, ob er einen gynäkologischen Krebs oder einen gutartigen gynäkologischen Zustand hat, wobei das Verfahren folgende Schritte umfasst:
Bereitstellen einer Urin-, Tampon- oder Vaginalabstrichprobe, die zuvor von dem Patienten isoliert wurde;
Bestimmen der Konzentration von MCM5 in der Urin-, Tampon- oder Vaginalabstrichprobe, wobei der Schritt des Bestimmens der Konzentration von MCM5 das Durchführen eines ELISA-Assays zum Nachweis von MCM5 oder zur Bestimmung der Konzentration von MCM5 umfasst;
Vergleichen der Konzentration von MCM5 in der Urin-, Tampon- oder Vaginalabstrichprobe mit einer Referenz;
Diagnostizieren von gynäkologischem Krebs bei dem Patienten, wenn die Konzentration von MCM5 in der Urin-, Tampon- oder Vaginalabstrichprobe höher ist als die Referenz oder Diagnostizieren eines gutartigen gynäkologischen Zustands bei dem Patienten, wenn die Konzentration von MCM5 in der Urin-, Tampon- oder Vaginalabstrichprobe niedriger ist als die Referenz;
(b) Unterscheiden zwischen einer Urin-, Tampon- oder Vaginalabstrichprobe, die mit einem gynäkologischen Krebs assoziiert ist, und einer Urin-, Tampon- oder Vaginalabstrichprobe, die mit einem gutartigen gynäkologischen Krebs assoziiert ist, wobei das Verfahren folgende Schritte umfasst:
Bereitstellen einer Urin-, Tampon- oder Vaginalabstrichprobe, die zuvor von einem Patienten isoliert wurde;
Bestimmen der Konzentration von MCM5 in der Urin-, Tampon- oder Vaginalabstrichprobe, wobei der Schritt des Bestimmens der Konzentration von MCM5 das Durchführen eines ELISA-Assays zum Nachweis von MCM5 oder zur Bestimmung der Konzentration von MCM5 umfasst;
Vergleichen der Konzentration von MCM5 in der Urin-, Tampon- oder Vaginalabstrichprobe mit einer Referenz;
Bestimmen, dass die Urin-, Tampon- oder Vaginalabstrichprobe mit einem gynäkologischen Krebs assoziiert ist, wenn die Konzentration von MCM5 in der Urin-, Tampon- oder Vaginalabstrichprobe höher ist als die Referenz oder Bestimmen, dass die Urin-, Tampon- oder Vaginalabstrichprobe mit einem gutartigen gynäkologischen Zustand assoziiert ist, wenn die Konzentration von MCM5 in der Urin-, Tampon- oder Vaginalabstrichprobe niedriger ist als die Referenz; oder
(c) Stratifizieren eines Patienten in eine erste oder eine zweite Behandlungsgruppe, wobei das Verfahren folgende Schritte umfasst:
Bereitstellen einer Urin-, Tampon- oder Vaginalabstrichprobe, die zuvor von dem Patienten isoliert wurde;
Bestimmen der Konzentration von MCM5 in der Urin-, Tampon- oder Vaginalabstrichprobe, wobei der Schritt des Bestimmens der Konzentration von MCM5 das Durchführen eines ELISA-Assays zum Nachweis von MCM5 oder zur Bestimmung der Konzentration von MCM5 umfasst;
Zuweisen des Patienten zu einer der beiden Behandlungsgruppen basierend auf der Konzentration von MCM5 in der Urin-, Tampon- oder Vaginalabstrichprobe, wobei die erste Behandlungsgruppe eine Behandlung für einen gynäkologischen Krebs erhalten soll und die zweite Behandlungsgruppe keine Behandlung oder eine Behandlung für einen gutartigen gynäkologischen Zustand erhalten soll; optional wobei der Patient der ersten Behandlungsgruppe zugeordnet wird, wenn die Konzentration von MCM5 in der Urin-, Tampon- oder Vaginalabstrichprobe höher ist als eine Referenz oder wobei der Patient der zweiten Behandlungsgruppe zugeordnet wird, wenn die Konzentration von MCM5 in der Urin-, Tampon- oder Vaginalabstrichprobe niedriger ist als die Referenz.

6. Verfahren nach Anspruch 4 oder Anspruch 5, wobei der gutartige gynäkologische Zustand ausgewählt ist aus postmenopausalen Blutungen (Post-Menopausal Bleeding, PMB), Endometriose, Fibroiden und polyzystischem Ovarialsyndrom (Polycystic Ovary Syndrome, PCOS).

7. Verfahren nach einem der Ansprüche 4 bis 6, wobei der Referenzwert etwa 12 pg/ml beträgt.

8. Verfahren nach einem der vorhergehenden Ansprüche, wobei die Urin-, Tampon- oder Vaginalabstrichprobe eine Urinprobe ist.

9. Verfahren nach einem der vorhergehenden Ansprüche, wobei das Verfahren ferner folgenden Schritt umfasst:
Behandeln der Urin-, Tampon- oder Vaginalabstrichprobe, um MCM5 aus Zellen in der Urin-, Tampon- oder Vaginalabstrichprobe freizusetzen; optional, wobei der Schritt der Behandlung der Urin-, Tampon- oder Vaginalabstrichprobe zur Freisetzung von MCM5 das Aussetzen der Urin-, Tampon- oder Vaginalabstrichprobe einem Lysepuffer umfasst, der in der Lage ist, MCM5 aus Zellen in der Urin-, Tampon- oder Vaginalabstrichprobe freizusetzen, optional, wobei der Schritt des Behandelns der Urin-, Tampon- oder Vaginalabstrichprobe zur Freisetzung von MCM5 Folgendefs umfasst:
Durchleiten der Urin-, Tampon- oder Vaginalabstrichprobe durch einen Filter zum Auffangen von Zellen, so dass Zellen in dem Filter aufgefangen werden;
Durchleiten eines Lysepuffers durch den Filter, so dass die aufgefangenen Zellen dem Lysepuffer ausgesetzt werden; und/oder
Inkubieren des Filters für eine gewisse Zeit, so dass der Lysepuffer die Zellen veranlasst, MCM5 freizusetzen.

10. Verfahren nach Anspruch 9, wobei:
(a) der Lysepuffer in der Lage ist, MCM5 aus Zellen in der Urin-, Tampon- oder Vaginalabstrichprobe freizusetzen;
(b) der Lysepuffer in der Lage ist, MCM5 aus Zellen in der Urin-, Tampon- oder Vaginalabstrichprobe freizusetzen, und das MCM5-Protein nicht wesentlich zu denaturieren, optional, wobei der Lysispuffer einen Antikörper nicht denaturiert; und/oder
(c)
(i) der Lysepuffer ein Detergens umfasst;
(ii) der Lysepuffer ein Detergens umfasst, das Triton X-100 umfasst;
(iii) der Lysepuffer ein Detergens umfasst, das Triton X-100 in einer Konzentration zwischen 0,01 % und 25 %, zwischen 0,01 % und 10 %, zwischen 0,05 % und 5 %, zwischen 0,1 % und 2 %, zwischen 0,5 % und 2 %, zwischen 0,75 % und 1,25 % oder etwa 1 % umfasst;
(iv) der Lysepuffer ein Detergens umfasst, das Natriumdeoxycholat umfasst oder daraus besteht;
(v) der Lysepuffer ein Detergens umfasst, das Natriumdeoxycholat umfasst oder daraus besteht, in einer Konzentration zwischen 0,1 % und 20 %, zwischen 0,1 und 10 %, zwischen 0,1 und 5 %, zwischen 0,5 % und 5 %, zwischen 0,5 % und 2,5 %, zwischen 0,75 % und 2,5 %, zwischen 0,75 % und 1,25 %, oder etwa 1 %;
(vi) der Lysepuffer ein Detergens umfasst, das Natriumdodecylsulfat (Sodium Dodecyl Sulphate, SDS) umfasst oder daraus besteht;
(vii) der Lysepuffer ein Detergens umfasst, das Natriumdodecylsulfat (SDS) in einer Konzentration zwischen 0,001 % und 10 %, zwischen 0,01 % und 5 %, zwischen 0,05 % und 5 %, zwischen 0,01 % und 1 %, zwischen 0,05 % und 1 %, zwischen 0,05 % und 0,5 %, zwischen 0,075 % und 0,25 % oder etwa 0,1 % umfasst;
(viii) der Lysepuffer ein Detergens umfasst, das aus Triton X-100, Natriumdeoxycholat und Natriumdodecylsulfat (SDS) besteht;
(ix) der Lysepuffer ein Detergens umfasst, das aus zwischen 0,5 % und 2 % Triton X-100, zwischen 0,5 % und 2 % Natriumdeoxycholat und zwischen 0,05 % und 0,5 % Natriumdodecylsulfat (SDS) besteht;
(x) der Lysepuffer eine Pufferkomponente umfasst;
(xi) der Lysepuffer eine Pufferkomponente umfasst, die einen pH-Wert von zwischen pH 4 und pH 9, zwischen pH 5 und pH 8,5, zwischen pH 6 und pH 8, zwischen pH 6,5 und pH 8, zwischen pH 7 und pH 8, zwischen pH 7,3 und pH 7,9, zwischen pH 7,4 und pH 7,8, zwischen pH 7,5 und pH 7,7 oder etwa pH 7,6 aufweist; und/oder den pH-Wert des Lysepuffers auf einem Wert zwischen pH 4 und pH 9, zwischen pH 5 und pH 8,5, zwischen pH 6 und pH 8, zwischen pH 6,5 und pH 8, zwischen pH 7 und pH 8, zwischen pH 7,3 und pH 7,9, zwischen pH 7,4 und pH 7,8, zwischen pH 7,5 und pH 7,7 oder etwa pH 7,6 hält;
(xii) der Lysepuffer eine Pufferkomponente umfasst, die Tris umfasst oder daraus besteht;
(xiii) der Lysepuffer eine Pufferkomponente umfasst, die Tris in einer Konzentration von mehr als 1 mM zwischen 1 mM und 350 mM, zwischen 5 und 200 mM, zwischen 5 und 100 mM, zwischen 5 und 50 mM, zwischen 5 mM und 40 mM, zwischen 5 mM und 35 mM, zwischen 10 mM und 35 mM, zwischen 15 mM und 35 mM, zwischen 15 mM und 30 mM, zwischen 20 mM und 10 mM, oder etwa 25 mM umfasst oder daraus besteht;
(xiv) der Lysepuffer eine Pufferkomponente umfasst, die aus Tris in einer Konzentration zwischen 15 mM und 35 mM besteht;
(xv) der Lysepuffer ein Salz umfasst;
(xvi) der Lysepuffer Natriumchlorid umfasst;
(xvii) der Lysepuffer Natriumchlorid in einer Konzentration zwischen 10 mM und 350 mM, zwischen 20 mM und 300 mM, zwischen 50 mM und 250 mM, zwischen 100 mM und 250, zwischen 100 mM und 200 mM, zwischen 125 mM und 175 mM, oder etwa 150 mM umfasst;
(xviii) der Lysepuffer Natriumchlorid in einer Konzentration zwischen 100 mM und 200 mM umfasst;
(xix) der Lysispuffer Folgendes umfasst:
a. zwischen 1 mM und 100 mM Tris;
b. zwischen 50 mM und 300 mM Natriumchlorid;
c. zwischen 0,1 und 5 % Natriumdeoxycholat;
d. zwischen 0,01 und 1 % Natriumdodecylsulfat; und/oder
e. zwischen 0,1 und 5 % Triton-X100;
(xx) der Lysepuffer Folgendes umfasst:
a. zwischen 10 mM und 40 mM Tris;
b. zwischen 100 mM und 200 mM Natriumchlorid;
c. zwischen 0,5 und 2 % Natriumdeoxycholat;
d. zwischen 0,05 und 0,5 % Natriumdodecylsulfat; und/oder
e. zwischen 0,5 und 2 % Triton-X100; und/oder
(xxi) der Lysepuffer Folgendes umfasst:
a. etwa 25 mM Tris;
b. etwa 150 mM Natriumchlorid;
c. etwa 1 % Natriumdeoxycholat;
d. etwa 0,1 % Natriumdodecylsulfat; und/oder
e. etwa 1 % Triton-X100.

11. Verfahren nach einem der vorhergehenden Ansprüche, wobei der Schritt des Nachweises von MCM5 oder der Bestimmung der Konzentration von MCM5 Folgendes umfasst:
Aussetzen der Urin-, Tampon- oder Vaginalabstrichprobe gegenüber einem ersten monoklonalen Antikörper und/oder einem zweiten monoklonalen Antikörper; und
Nachweisen von MCM5, das an den ersten monoklonalen Antikörper und/oder den zweiten monoklonalen Antikörper gebunden ist, oder Bestimmung der Konzentration von MCM5, das an den ersten monoklonalen Antikörper und/oder den zweiten monoklonalen Antikörper gebunden ist.

12. Verfahren nach Anspruch 11, wobei:
(a) der erste monoklonale Antikörper und der zweite monoklonale Antikörper an MCM5 binden, wobei der erste monoklonale Antikörper optional ein Antikörper ist, der:
(i) an ein Polypeptid mit der Aminosäuresequenz von SEQ ID NO: 1 bindet;
(ii) mindestens eine komplementär bestimmende Region (CDR) umfasst, die ausgewählt ist aus der Gruppe bestehend aus:
a. 12A7 CDRH1, die eine Sequenz von SEQ ID NO: 9 oder eine Sequenz aufweist, die sich von SEQ ID NO: 9 durch eine einzelne Aminosäuresubstitution unterscheidet;
b. 12A7 CDRH2, die eine Sequenz von SEQ ID NO: 11 oder eine Sequenz aufweist, die sich von SEQ ID NO: 11 durch eine einzelne Aminosäuresubstitution unterscheidet;
c. 12A7 CDRH3, die eine Sequenz von SEQ ID NO: 13 oder eine Sequenz aufweist, die sich von SEQ ID NO: 13 durch eine einzelne Aminosäuresubstitution unterscheidet;
d. 12A7 CDRL1, die eine Sequenz von SEQ ID NO: 3 oder eine Sequenz aufweist, die sich von SEQ ID NO: 3 durch eine einzelne Aminosäuresubstitution unterscheidet;
e. 12A7 CDRL2, die eine Sequenz von SEQ ID NO: 5 oder eine Sequenz aufweist, die sich von SEQ ID NO: 5 durch eine einzelne Aminosäuresubstitution unterscheidet; und
f. 12A7 CDRL3, die eine Sequenz von SEQ ID NO: 7 oder eine Sequenz aufweist, die sich von SEQ ID NO: 7 durch eine einzelne Aminosäuresubstitution unterscheidet;
(iii) eine variable Region der schweren Kette mit einer Sequenz umfasst, die zu mindestens 85 %, 90 %, 95 %, 98 %, 99 % oder 100 % mit SEQ ID NO: 29 identisch ist;
(iv) eine variable Region der leichten Kette mit einer Sequenz umfasst, die zu mindestens 85 %, 90 %, 95 %, 98 %, 99 % oder 100 % mit SEQ ID NO: 27 identisch ist; oder
(v) mit dem Antikörper von (i), (ii), (iii) oder (iv) konkurriert; und/oder der zweite monoklonale Antikörper ein Antikörper ist, der:
(i) an ein Polypeptid mit der Aminosäuresequenz von SEQ ID NO: 2 bindet;
(ii) mindestens eine komplementär bestimmende Region (CDR) umfasst, die ausgewählt ist aus der Gruppe bestehend aus:
a. 4B4 CDRH1, die eine Sequenz von SEQ ID NO: 21 oder eine Sequenz aufweist, die sich von SEQ ID NO: 21 durch eine einzige Aminosäuresubstitution unterscheidet;
b. 4B4 CDRH2, die eine Sequenz von SEQ ID NO: 23 oder eine Sequenz aufweist, die sich von SEQ ID NO: 23 durch eine einzige Aminosäuresubstitution unterscheidet;
c. 4B4 CDRH3, die eine Sequenz von SEQ ID NO: 25 oder eine Sequenz aufweist, die sich von SEQ ID NO: 25 durch eine einzige Aminosäuresubstitution unterscheidet;
d. 4B4 CDRL1, die eine Sequenz von SEQ ID NO: 15 oder eine Sequenz aufweist, die sich von SEQ ID NO: 15 durch eine einzige Aminosäuresubstitution unterscheidet;
e. 4B4 CDRL2, die eine Sequenz von SEQ ID NO: 17 oder eine Sequenz aufweist, die sich von SEQ ID NO: 17 durch eine einzige Aminosäuresubstitution unterscheidet; und
f. 4B4 CDRL3, die eine Sequenz von SEQ ID NO: 19 oder eine Sequenz aufweist, die sich von SEQ ID NO: 19 durch eine einzige Aminosäuresubstitution unterscheidet;
(iii) eine variable Region der schweren Kette mit einer Sequenz umfasst, die zu mindestens 85 %, 90 %, 95 %, 98 %, 99 % oder 100 % mit SEQ ID NO: 33 identisch ist;
(iv) eine variable Region der leichten Kette mit einer Sequenz umfasst, die zu mindestens 85 %, 90 %, 95 %, 98 %, 99 % oder 100 % mit SEQ ID NO: 31 identisch ist; oder
(v) mit dem Antikörper von (i), (ii), (iii) oder (iv) konkurriert; und/oder
(b)
(i) der erste monoklonale Antikörper und/oder der zweite monoklonale Antikörper eine Affinität für MCM5 im Bereich von 0,001-1 nM aufweist;
(ii) der erste monoklonale Antikörper und/oder der zweite monoklonale Antikörper ein Fab'₂, ein F'(ab)₂, ein Fv, ein Einzelketten-Antikörper oder ein Diabody ist;
(iii) der erste monoklonale Antikörper 12A7 CDRH1, 12A7 CDRH2 und 12A7 CDRH3 umfasst;
(iv) der erste monoklonale Antikörper 12A7 CDRL1, 12A7 CDRL2 und 12A7 CDRL3 umfasst;
(v) der erste monoklonale Antikörper Folgendes umfasst: einen 12A7 CDRH1, der eine Sequenz von SEQ ID NO: 9 aufweist, einen 12A7 CDRH2, der eine Sequenz von SEQ ID NO: 11 aufweist, und einen 12A7 CDRH3, der eine Sequenz von SEQ ID NO: 13 aufweist;
(vi) der erste monoklonale Antikörper Folgendes umfasst: einen 12A7 CDRL1, der eine Sequenz von SEQ ID NO: 3 aufweist, einen 12A7 CDRL2, der eine Sequenz von SEQ ID NO: 5 aufweist, und einen 12A7 CDRL3, der eine Sequenz von SEQ ID NO: 7 aufweist;
(vii) der zweite monoklonale Antikörper 4B4 CDRH1, 4B4 CDRH2 und 4B4 CDRH3 umfasst;
(viii) der zweite monoklonale Antikörper 4B4 CDRL1, 4B4 CDRL2 und 4B4 CDRL3 umfasst;
(ix) der zweite monoklonale Antikörper Folgendes umfasst: einen 4B4 CDRH1, der die Sequenz SEQ ID NO: 21 aufweist, einen 4B4 CDRH2, der die Sequenz SEQ ID NO: 23 aufweist, und einen 4B4 CDRH3, der die Sequenz SEQ ID NO: 25 aufweist;
(x) der zweite monoklonale Antikörper Folgendes aufweist: einen 4B4 CDRL1, der die Sequenz SEQ ID NO: 15 aufweist, einen 4B4 CDRL2, der die Sequenz SEQ ID NO: 17 aufweist, und einen 4B4 CDRL3, der die Sequenz SEQ ID NO: 19 aufweist;
(xi) der erste monoklonale Antikörper eine variable Region der schweren Kette mit einer Sequenz umfasst, die zu mindestens 95 % mit SEQ ID NO: 29 identisch ist;
(xii) der erste monoklonale Antikörper eine variable Region der schweren Kette mit einer Sequenz umfasst, die zu mindestens 98 % mit SEQ ID NO: 29 identisch ist;
(xiii) der erste monoklonale Antikörper eine variable Region der leichten Kette mit einer Sequenz umfasst, die zu mindestens 95 % mit SEQ ID NO: 27 identisch ist;
(xiv) der erste monoklonale Antikörper eine variable Region der leichten Kette mit einer Sequenz umfasst, die zu mindestens 98 % mit SEQ ID NO: 27 identisch ist;
(xv) der zweite monoklonale Antikörper eine variable Region der schweren Kette mit einer Sequenz umfasst, die zu mindestens 95 % mit SEQ ID NO: 33 identisch ist;
(xvi) der zweite monoklonale Antikörper eine variable Region der schweren Kette mit einer Sequenz umfasst, die zu mindestens 98 % mit SEQ ID NO: 33 identisch ist;
(xvii) der zweite monoklonale Antikörper eine variable Region der leichten Kette mit einer Sequenz umfasst, die zu mindestens 95 % mit SEQ ID NO: 31 identisch ist; und/oder
(xviii) der zweite monoklonale Antikörper eine variable Region der leichten Kette mit einer Sequenz umfasst, die zu mindestens 98 % mit SEQ ID NO: 31 identisch ist.

13. Verfahren nach einem der vorhergehenden Ansprüche, wobei:
(a) die Urin-, Tampon- oder Vaginalabstrichprobe eine Urinprobe ist und das Verfahren eine höhere Empfindlichkeit aufweist als ein äquivalentes Verfahren, das unter Verwendung einer Abstrichprobe durchgeführt wird, und/oder ein äquivalentes Verfahren, das unter Verwendung einer Tamponprobe durchgeführt wird; und/oder
(b) die Urin-, Tampon- oder Vaginalabstrichprobe eine Urinprobe ist und das Verfahren eine höhere Spezifität aufweist als ein äquivalentes Verfahren, das unter Verwendung einer Abstrichprobe durchgeführt wird, und/oder ein äquivalentes Verfahren, das unter Verwendung einer Tamponprobe durchgeführt wird.

14. Verfahren nach einem der vorhergehenden Ansprüche, wobei:
(a)
(i) das Verfahren eine Empfindlichkeit für den gynäkologischen Krebs von mindestens etwa 20 %, 22 %, 24 %, 26 %, 28 %, 30 %, 32 %, 34 %, 36 %, 38 %, 40 %, 42 %, 44 %, 46 %, 48 % oder 50 % aufweist;
(ii) das Verfahren eine Empfindlichkeit für den gynäkologischen Krebs von mindestens etwa 50 %, 52 %, 54 %, 55 %, 56 %, 57 %, 58 %, 59 %, 60 % oder 61 % aufweist;
(iii) das Verfahren eine Empfindlichkeit für den gynäkologischen Krebs von mindestens etwa 75 %, 76 %, 77 %, 78 %, 79 %, 80 % oder 81 % aufweist;
(iv) das Verfahren eine Empfindlichkeit für den gynäkologischen Krebs von mindestens etwa 90 %, 92 %, 93 %, 94 %, 95 % oder 96 % aufweist;
(v) das Verfahren eine Spezifität für den gynäkologischen Krebs von mindestens etwa 20 %, 22 %, 24 %, 26 %, 28 %, 30 %, 32 %, 34 %, 36 %, 38 % oder 40 % aufweist;
(vi) das Verfahren eine Spezifität für den gynäkologischen Krebs von mindestens etwa 50 %, 52 %, 54 %, 55 %, 56 %, 57 %, 58 %, 59 % oder 60 % aufweist;
(vii) das Verfahren eine Spezifität für den gynäkologischen Krebs von mindestens etwa 60 %, 62 %, 64 %, 66 %, 68 %, 70 %, 71 %, 72 %, 73 %, 74 % oder 75 % aufweist; und/oder
(viii) das Verfahren eine Spezifität für den gynäkologischen Krebs von mindestens etwa 80 %, 82 %, 83 %, 84 %, 85 % oder 86 % aufweist; oder
(b)
(i) die Urin-, Tampon- oder Vaginalabstrichprobe eine Urinprobe ist und das Verfahren eine Empfindlichkeit für den gynäkologischen Krebs von mindestens etwa 75 % und eine Spezifität für den gynäkologischen Krebs von mindestens etwa 55 % aufweist;
(ii) die Urin-, Tampon- oder Vaginalabstrichprobe eine Vaginalabstrichprobe ist und das Verfahren eine Empfindlichkeit für den gynäkologischen Krebs von mindestens etwa 55 % und eine Spezifität für den gynäkologischen Krebs von mindestens etwa 70 % aufweist;
(iii) die Urin-, Tampon- oder Vaginalabstrichprobe eine Tamponprobe ist und das Verfahren eine Empfindlichkeit für den gynäkologischen Krebs von mindestens etwa 90 % und eine Spezifität für den gynäkologischen Krebs von mindestens etwa 35 % aufweist;
(iv) die Urin-, Tampon- oder Vaginalabstrichprobe eine Tamponprobe ist und das Verfahren eine Empfindlichkeit für den gynäkologischen Krebs von mindestens etwa 45 % und eine Spezifität für den gynäkologischen Krebs von mindestens etwa 80 % aufweist; oder
(v) die Urin-, Tampon- oder Vaginalabstrichprobe eine Tamponprobe ist und das Verfahren eine Empfindlichkeit für den gynäkologischen Krebs von mindestens etwa 90 % und eine Spezifität für den gynäkologischen Krebs von mindestens etwa 80 % aufweist.

15. Verwendung eines Lysepuffers oder eines ersten monoklonalen Antikörpers und/oder eines zweiten monoklonalen Antikörpers oder eines Kits in einem Verfahren zum Nachweis des Vorhandenseins oder der Abwesenheit eines gynäkologischen Krebses in einem Patienten nach einem der vorhergehenden Ansprüche, wobei der Kit den Lysispuffer, den ersten monoklonalen Antikörper und/oder den zweiten monoklonalen Antikörper umfasst.

16. Verfahren nach einem der Ansprüche 1 bis 14 oder Verwendung nach Anspruch 15, wobei:
(a) der gynäkologische Krebs ein gynäkologisches Malignom ist;
(b) der gynäkologische Krebs ausgewählt ist aus der Gruppe bestehend aus Eierstockkrebs, Endometriumkrebs, Gebärmutterkrebs, Gebärmutterhalskrebs, Vulvakrebs, Vaginalkrebs, Eileitertumor, epithelialem Ovarialtumor, Ovarialteratom, unreifem Ovarialteratom, Stromatumor, Keimzelltumor der Eierstöcke, undifferenzierten Tumoren, Borderline-Ovarialtumor, Endometriumkrebs, endometrialem Adenokarzinom, endometrioidem Adenokarzinom, endometrialem Plattenepithelkarzinom, serösem Karzinom, serösem endometrialem intraepithelialem Karzinom, endometrialem Karzinom, klarzelligem Karzinom, muzinösem Karzinom, undifferenziertem Endometriumkrebs, gemischtem Endometriumkrebs, muzinösem Tumor, bösartigem gemischtem Mullertumor, klarzelligem Endometriumsarkom, Granulosazelltumor, serösem tubalem intraepithelialem Karzinom, unreifem zystischem Teratom, serösem Ovarialtumor und kleinzelligem Karzinom;
(c) der gynäkologische Krebs ein Eierstockkrebs oder ein Endometriumkrebs ist;
(d) der gynäkologische Krebs ein Eierstockkrebs ist, wobei der Eierstockkrebs optional ausgewählt ist aus der Gruppe bestehend aus epithelialem Ovarialtumor, Ovarialteratom, unreifem Ovarialteratom, Stromatumor, Keimzell-Ovarialtumor, undifferenzierten Tumoren, Borderline-Ovarialtumor, muzinösem Tumor, Granulosazelltumor, unreifem zystischem Teratom, serösem Ovarialtumor und kleinzelligem Karzinom;
(e) der gynäkologische Krebs ein Endometriumkrebs ist, wobei der Endometriumkrebs optional ausgewählt ist aus der Gruppe bestehend aus Endometriumkrebs, endometrialem Adenokarzinom, endometrioidem Adenokarzinom, endometrialem Plattenepithelkarzinom, serösem Karzinom, serösem endometrialem intraepithelialem Karzinom, endometrialem Karzinosarkom, klarzelligem Karzinom, muzinösem Karzinom, undifferenziertem Endometriumkrebs, gemischtem Endometriumkrebs, bösartigem gemischtem Mullerschem Tumor und klarzelligem Endometriumsarkom; und/oder
(f) der gynäkologische Krebs Folgendes ist:
(i) ein niedriggradiger Krebs, optional ein G1-Krebs; oder
(ii) ein hochgradiger Krebs, optional ein G2-Krebs oder höher; und/oder
(g) der gynäkologische Krebs Folgendes ist:
(i) ein Krebs im Frühstadium, optional ein S1-Krebs; oder
(ii) ein Krebs im Spätstadium, optional ein S2-Krebs oder darüber.

17. Verfahren oder Verwendung nach einem der vorhergehenden Ansprüche, wobei:
(a)
(i) der gynäkologische Krebs ein Eierstockkrebs ist und das Verfahren eine Spezifität für den Eierstockkrebs von mindestens etwa 25 % aufweist;
(ii) der gynäkologische Krebs ein Eierstockkrebs ist und das Verfahren eine Empfindlichkeit für den Eierstockkrebs von mindestens etwa 50 % aufweist;
(iii) der gynäkologische Krebs ein Eierstockkrebs ist und das Verfahren eine Spezifität für den Eierstockkrebs von mindestens etwa 25 % und eine Empfindlichkeit für den Eierstockkrebs von mindestens etwa 50 % aufweist;
(iv) der gynäkologische Krebs ein Endometriumkrebs ist und das Verfahren eine Spezifität für den Endometriumkrebs von mindestens etwa 40 % aufweist;
(v) der gynäkologische Krebs ein Endometriumkrebs ist und das Verfahren eine Empfindlichkeit für den Endometriumkrebs von mindestens etwa 80 % aufweist; und/oder
(vi) der gynäkologische Krebs ein Endometriumkrebs ist und das Verfahren eine Spezifität für den Endometriumkrebs von mindestens etwa 40 % und eine Empfindlichkeit für den Endometriumkrebs von mindestens etwa 80 % aufweist;
(b)
(i) die Urin-, Tampon- oder Vaginalabstrichprobe eine Urinprobe ist, der gynäkologische Krebs ein Eierstockkrebs ist und das Verfahren eine Empfindlichkeit für den Eierstockkrebs von mindestens etwa 60 % und eine Spezifität für den Eierstockkrebs von mindestens etwa 70 % aufweist, wobei optional eine MCM5-Konzentrationsreferenz von etwa 12 pg/ml verwendet wird; oder
(ii) die Urin-, Tampon- oder Vaginalabstrichprobe eine Urinprobe ist, der gynäkologische Krebs ein Endometriumkrebs ist und das Verfahren eine Empfindlichkeit für den Endometriumkrebs von mindestens etwa 80 % und eine Spezifität für den Endometriumkrebs von mindestens etwa 70 % aufweist, wobei optional eine MCM5-Konzentrationsreferenz von etwa 12 pg/ml verwendet wird; oder
(c)
(i) die Urin-, Tampon- oder Vaginalabstrichprobe eine Urinprobe ist, der gynäkologische Krebs ein niedriggradiger Eierstockkrebs ist und das Verfahren eine Empfindlichkeit für den niedriggradigen Eierstockkrebs von mindestens etwa 70 % aufweist, wobei optional eine MCM5-Konzentrationsreferenz von etwa 12 pg/ml verwendet wird;
(ii) die Urin-, Tampon- oder Vaginalabstrichprobe eine Urinprobe ist, der gynäkologische Krebs ein Eierstockkrebs im Frühstadium ist und das Verfahren eine Sensitivität für den Eierstockkrebs im Frühstadium von mindestens etwa 70 % aufweist, wobei optional eine MCM5-Konzentrationsreferenz von etwa 12 pg/ml verwendet wird;
(iii) die Urin-, Tampon- oder Vaginalabstrichprobe eine Urinprobe ist, der gynäkologische Krebs ein niedriggradiger Endometriumkrebs ist und das Verfahren eine Empfindlichkeit für den niedriggradigen Endometriumkrebs von mindestens etwa 80 % aufweist, wobei optional eine MCM5-Konzentrationsreferenz von etwa 12 pg/ml verwendet wird; oder
(iv) die Urin-, Tampon- oder Vaginalabstrichprobe eine Urinprobe ist, der gynäkologische Krebs ein Endometriumkrebs im Frühstadium ist und das Verfahren eine Empfindlichkeit für den Endometriumkrebs im Frühstadium von mindestens etwa 85 % aufweist, wobei optional eine MCM5-Konzentrationsreferenz von etwa 12 pg/ml verwendet wird.

18. Verfahren oder Verwendung nach einem der vorhergehenden Ansprüche, wobei der Patient an einer gutartigen gynäkologischen Erkrankung leidet, wobei der gutartige gynäkologische Zustand optional ausgewählt ist aus postmenopausalen Blutungen (PMB), Endometriose, Fibroiden und polyzystischem Ovarialsyndrom (PCOS).

## Revendications

1. Procédé *in vitro* pour détecter la présence ou l'absence d'un cancer gynécologique chez un sujet, le procédé comprenant les étapes consistant à :
obtenir un échantillon d'urine, de tampon ou de frottis vaginal isolé du sujet ; et
détecter le composant 5 du complexe de maintenance des minichromosomes (MCM5) ou
déterminer la concentration de MCM5 ;
dans lequel l'étape de détection de MCM5 ou de détermination de la concentration de MCM5 comprend la réalisation d'un dosage ELISA pour détecter MCM5 ou déterminer la concentration de MCM5.

2. Procédé selon la revendication 1, comprenant en outre une étape consistant à :
comparer la concentration de MCM5 déterminée à une référence, de préférence dans lequel la référence est une concentration moyenne de MCM5 déterminée pour un ou plusieurs échantillons préparés à partir d'un ou plusieurs individus sains, éventuellement dans lequel :
(i) un cancer gynécologique est susceptible d'être présent si la concentration de MCM5 est anormale par rapport à la référence ; et/ou
(ii) un cancer gynécologique est susceptible d'être présent si la concentration de MCM5 est supérieure à la référence.

3. Procédé selon l'une quelconque des revendications précédentes, dans lequel :
(a) le cancer gynécologique est susceptible d'être présent si la concentration de MCM5 est déterminée comme étant supérieure à 5 pg/mL, 6 pg/mL, 7 pg/mL, 8 pg/mL, 9 pg/mL, 10 pg/mL, 11 pg/mL, 12 pg/mL, 13 pg/mL, 14 pg/mL, 15 pg/mL, 16 pg/mL, 17 pg/mL, 18 pg/mL, 19 pg/mL, 20 pg/mL, 21 pg/mL, 22 pg/mL, 23 pg/mL, 24 pg/mL, 25 pg/mL, 26 pg/mL, 27 pg/mL, 28 pg/mL, 29 pg/mL, 30 pg/mL, 35 pg/mL, 45 pg/mL, 50 pg/mL, 55 pg/mL, 60 pg/mL, 65 pg/mL ou 70 pg/mL ;
(b) le cancer gynécologique est susceptible d'être présent si la concentration de MCM5 est déterminée comme étant supérieure à (i) environ 12 pg/mL; (ii) environ 17 pg/mL; ou (iii) environ 70 pg/mL ; de préférence à environ 12 pg/mL ; et/ou
(c) un seuil de 12 pg/mL, 17 pg/mL ou 70 pg/mL, de préférence d'environ 12 pg/mL, est appliqué.

4. Procédé selon l'une quelconque des revendications précédentes, dans lequel le procédé est un procédé de diagnostic d'un cancer gynécologique chez un sujet, éventuellement dans lequel le procédé comprend en outre une étape consistant à :
diagnostiquer le sujet comme ayant un cancer gynécologique si un cancer gynécologique est susceptible d'être présent.

5. Procédé *in vitro* pour :
(a) diagnostiquer un sujet comme ayant un cancer gynécologique ou une affection gynécologique bénigne, le procédé comprenant les étapes consistant à :
fournir un échantillon d'urine, de tampon ou de frottis vaginal préalablement isolé du sujet ;
déterminer la concentration de MCM5 dans l'échantillon d'urine, de tampon ou de frottis vaginal, dans lequel l'étape de détermination de la concentration de MCM5 comprend la réalisation d'un dosage ELISA pour détecter MCM5 ou déterminer la concentration de MCM5 ;
comparer la concentration de MCM5 dans l'échantillon d'urine, de tampon ou de frottis vaginal à une référence ;
diagnostiquer le sujet comme ayant un cancer gynécologique si la concentration de MCM5 dans l'échantillon d'urine, de tampon ou de frottis vaginal est supérieure à la référence ou
diagnostiquer le sujet comme ayant une affection gynécologique bénigne si la concentration de MCM5 dans l'échantillon d'urine, de tampon ou de frottis vaginal est inférieure à la référence ;
(b) faire la distinction entre un échantillon d'urine, de tampon ou de frottis vaginal associé à un cancer gynécologique et un échantillon d'urine, de tampon ou de frottis vaginal associé à un cancer gynécologique bénin, le procédé comprenant les étapes consistant à :
fournir un échantillon d'urine, de tampon ou de frottis vaginal préalablement isolé d'un sujet ;
déterminer la concentration de MCM5 dans l'échantillon d'urine, de tampon ou de frottis vaginal, dans lequel l'étape de détermination de la concentration de MCM5 comprend la réalisation d'un dosage ELISA pour détecter MCM5 ou déterminer la concentration de MCM5 ;
comparer la concentration de MCM5 dans l'échantillon d'urine, de tampon ou de frottis vaginal à une référence ;
déterminer que l'échantillon d'urine, de tampon ou de frottis vaginal est associé à un cancer gynécologique si la concentration de MCM5 dans l'échantillon d'urine, de tampon ou de frottis vaginal est supérieure à la référence ou déterminer que l'échantillon d'urine, de tampon ou de frottis vaginal est associé à un affection gynécologique bénigne si la concentration de MCM5 dans l'échantillon d'urine, de tampon ou de frottis vaginal est inférieure à la référence ; ou
(c) stratifier un sujet dans un premier ou un deuxième groupe de traitement, le procédé comprenant les étapes consistant à :
fournir un échantillon d'urine, de tampon ou de frottis vaginal préalablement isolé du sujet ;
déterminer la concentration de MCM5 dans l'échantillon d'urine, de tampon ou de frottis vaginal, dans lequel l'étape de détermination de la concentration de MCM5 comprend la réalisation d'un dosage ELISA pour détecter MCM5 ou déterminer la concentration de MCM5 ;
affecter le sujet à l'un des deux groupes de traitement sur la base de la concentration de MCM5 dans l'échantillon d'urine, de tampon ou de frottis vaginal, le premier groupe de traitement devant recevoir un traitement pour un cancer gynécologique et le deuxième groupe de traitement ne devant recevoir aucun traitement ou traitement d'une affection gynécologique bénigne ;
éventuellement dans lequel le sujet est affecté au premier groupe de traitement si la concentration de MCM5 dans l'échantillon d'urine, de tampon ou de frottis vaginal est supérieure à une référence ou le sujet est affecté au deuxième groupe de traitement si la concentration de MCM5 dans l'échantillon d'urine, de tampon ou de frottis vaginal est inférieure à la référence.

6. Procédé selon la revendication 4 ou la revendication 5, dans lequel l'affection gynécologique bénigne est choisie parmi les saignements post-ménopausiques (PMB), l'endométriose, les fibromes et le syndrome des ovaires polykystiques (SOPK).

7. Procédé selon l'une quelconque des revendications 4 à 6, dans lequel la référence est d'environ 12 pg/mL.

8. Procédé selon l'une quelconque des revendications précédentes, dans lequel l'échantillon d'urine, de tampon ou de frottis vaginal est un échantillon d'urine.

9. Procédé selon l'une quelconque des revendications précédentes, ledit procédé comprenant en outre une étape consistant à :
traiter l'échantillon d'urine, de tampon ou de frottis vaginal pour libérer MCM5 des cellules dans l'échantillon d'urine, de tampon ou de frottis vaginal ;
éventuellement dans lequel l'étape de traitement de l'échantillon d'urine, de tampon ou de frottis vaginal pour libérer MCM5 comprend l'exposition de l'échantillon d'urine, de tampon ou de frottis vaginal à un tampon de lyse capable de libérer MCM5 des cellules dans l'échantillon d'urine, de tampon ou de frottis vaginal, éventuellement dans lequel l'étape de traitement de l'échantillon d'urine, de tampon ou de frottis vaginal pour libérer MCM5 comprend :
le passage de l'échantillon d'urine, de tampon ou de frottis vaginal à travers un filtre pour capturer les cellules, de sorte que les cellules soient capturées dans le filtre ;
le passage d'un tampon de lyse à travers le filtre, de sorte que les cellules capturées soient exposées au tampon de lyse ; et/ou
l'incubation du filtre pendant une période de temps, de sorte que le tampon de lyse amène les cellules à libérer MCM5.

10. Procédé selon la revendication 9, dans lequel :
(a) le tampon de lyse est capable de libérer MCM5 des cellules dans l'échantillon d'urine, de tampon ou de frottis vaginal ;
(b) le tampon de lyse est capable de libérer MCM5 des cellules dans l'échantillon d'urine, de tampon ou de frottis vaginal et ne dénature pas substantiellement la protéine MCM5, éventuellement dans lequel le tampon de lyse ne dénature pas un anticorps; et/ou
(c)
(i) le tampon de lyse comprend un détergent ;
(ii) le tampon de lyse comprend un détergent qui comprend du Triton X-100 ;
(iii) le tampon de lyse comprend un détergent qui comprend du Triton X-100 à une concentration entre 0,01 % et 25 %, entre 0,01 % et 10 %, entre 0,05 % et 5 %, entre 0,1 % et 2 %, entre 0,5 % et 2 %, entre 0,75 % et 1,25 %, ou environ 1 % ;
(iv) le tampon de lyse comprend un détergent qui comprend ou est constitué de désoxycholate de sodium;
(v) le tampon de lyse comprend un détergent qui comprend ou est constitué de désoxycholate de sodium à une concentration entre 0,1 % et 20 %, entre 0,1 et 10 %, entre 0,1 et 5 %, entre 0,5 % et 5 %, entre 0,5 % et 2,5 %, entre 0,75 % et 2,5 %, entre 0,75 % et 1,25 %, ou environ 1 %;
(vi) le tampon de lyse comprend un détergent qui comprend ou est constitué de dodécylsulfate de sodium (SDS);
(vii) le tampon de lyse comprend un détergent qui comprend ou est constitué de dodécylsulfate de sodium (SDS) à une concentration entre 0,001 % et 10 %, entre 0,01 % et 5 %, entre 0,05 % et 5 %, entre 0,01 % et 1 % , entre 0,05 % et 1 %, entre 0,05 % et 0,5 %, entre 0,075 % et 0,25 %, ou environ 0,1 %;
(viii) le tampon de lyse comprend un détergent qui est constitué de Triton X-100, de désoxycholate de sodium et de dodécylsulfate de sodium (SDS) ;
(ix) le tampon de lyse comprend un détergent qui est constitué de 0,5 % à 2 % de Triton X-100, de 0,5 % à 2 % de désoxycholate de sodium et de 0,05 % à 0,5 % de dodécylsulfate de sodium (SDS);
(x) le tampon de lyse comprend un composant tampon ;
(xi) le tampon de lyse comprend un composant tampon qui a un pH compris entre pH 4 et pH 9, entre pH 5 et pH 8,5, entre pH 6 et pH 8, entre pH 6,5 et pH 8, entre pH 7 et pH 8, entre pH 7,3 et pH 7,9, entre pH 7,4 et pH 7,8, entre pH 7,5 et pH 7,7, ou environ pH 7,6 ; et/ou maintient le pH du tampon de lyse entre pH 4 et pH 9, entre pH 5 et pH 8,5, entre pH 6 et pH 8, entre pH 6,5 et pH 8, entre pH 7 et pH 8, entre pH 7,3 et pH 7,9, entre pH 7,4 et pH 7,8, entre pH 7,5 et pH 7,7, ou environ pH 7,6;
(xii) le tampon de lyse comprend un composant tampon qui comprend ou est constitué de Tris ;
(xiii) le tampon de lyse comprend un composant tampon qui comprend ou est constitué de Tris à une concentration supérieure à 1 mM, entre 1 mM et 350 mM, entre 5 et 200 mM, entre 5 et 100 mM, entre 5 et 50 mM, entre 5 mM et 40 mM, entre 5 mM et 35 mM, entre 10 mM et 35 mM, entre 15 mM et 35 mM, entre 15 mM et 30 mM, entre 20 mM et 30 mM, ou environ 25 mM ;
(xiv) le tampon de lyse comprend un composant tampon qui est constitué de Tris à une concentration comprise entre 15 mM et 35 mM;
(xv) le tampon de lyse comprend un sel;
(xvi) le tampon de lyse comprend du chlorure de sodium;
(xvii) le tampon de lyse comprend du chlorure de sodium à une concentration comprise entre 10 mM et 350 mM, entre 20 mM et 300 mM, entre 50 mM et 250 mM, entre 100 mM et 250, entre 100 mM et 200 mM, entre 125 mM et 175 mM, ou environ 150 mM;
(xviii) le tampon de lyse comprend du chlorure de sodium à une concentration comprise entre 100 mM et 200 mM ;
(xix) le tampon de lyse comprend:
a. entre 1 mM et 100 mM de Tris;
b. entre 50 mM et 300 mM de chlorure de sodium;
c. entre 0,1 et 5 % de désoxycholate de sodium;
d. entre 0,01 et 1 % de dodécylsulfate de sodium; et/ou
e. entre 0,1 et 5 % de Triton-X 100;
(xx) le tampon de lyse comprend:
a. entre 10 mM et 40 mM de Tris;
b. entre 100 mM et 200 mM de chlorure de sodium;
c. entre 0,5 et 2 % de désoxycholate de sodium;
d. entre 0,05 et 0,5 % de dodécylsulfate de sodium; et/ou
e. entre 0,5 et 2 % de Triton-X 100; et/ou
(xxi) le tampon de lyse comprend:
a. environ 25 mM de Tris;
b. environ 150 mM de chlorure de sodium;
c. environ 1 % de désoxycholate de sodium;
d. environ 0,1 % de dodécylsulfate de sodium; et/ou
e. environ 1 % de Triton-X100.

11. Procédé selon l'une quelconque des revendications précédentes, dans lequel l'étape de détection de MCM5 ou de détermination de la concentration de MCM5 comprend :
l'exposition de l'échantillon d'urine, de tampon ou de frottis vaginal à un premier anticorps monoclonal et/ou à un deuxième anticorps monoclonal ; et
la détection de MCM5 lié au premier anticorps monoclonal et/ou au deuxième anticorps monoclonal ou la détermination de la concentration de MCM5 lié au premier anticorps monoclonal et/ou au deuxième anticorps monoclonal.

12. Procédé selon la revendication 11, dans lequel :
(a) le premier anticorps monoclonal et le deuxième anticorps monoclonal se lient à MCM5, éventuellement dans lequel le premier anticorps monoclonal est un anticorps qui :
(i) se lie à un polypeptide ayant une séquence d'acides aminés de SEQ ID NO : 1 ;
(ii) comprend au moins une Région Déterminante Complémentaire (CDR) choisie dans le groupe constitué par :
a. 12A7 CDRH1 qui a une séquence de SEQ ID NO : 9 ou une séquence qui diffère de SEQ ID NO : 9 par une substitution d'acide aminé unique ;
b. 12A7 CDRH2 qui a une séquence de SEQ ID NO : 11 ou une séquence qui diffère de SEQ ID NO : 11 par une substitution d'acide aminé unique ;
c. 12A7 CDRH3 qui a une séquence de SEQ ID NO : 13 ou une séquence qui diffère de SEQ ID NO : 13 par une substitution d'acide aminé unique ;
d. 12A7 CDRL1 qui a une séquence de SEQ ID NO : 3 ou une séquence qui diffère de SEQ ID NO : 3 par une substitution d'acide aminé unique ;
e. 12A7 CDRL2 qui a une séquence de SEQ ID NO : 5 ou une séquence qui diffère de SEQ ID NO : 5 par une substitution d'acide aminé unique ; et
f. 12A7 CDRL3 qui a une séquence de SEQ ID NO : 7 ou une séquence qui diffère de SEQ ID NO : 7 par une substitution d'acide aminé unique ;
(iii) comprend une région variable de chaîne lourde ayant une séquence au moins à 85 %, 90 %, 95 %, 98 %, 99 % ou 100 % identique à SEQ ID NO : 29 ;
(iv) comprend une séquence de région variable de chaîne légère ayant une séquence au moins à 85 %, 90 %, 95 %, 98 %, 99 % ou 100 % identique à SEQ ID NO : 27 ; ou
(v) entre en compétition avec l'anticorps de (i), (ii), (iii) ou (iv) ; et/ou
le deuxième anticorps monoclonal est un anticorps qui :
(i) se lie à un polypeptide ayant une séquence d'acides aminés de SEQ ID NO : 2 ;
(ii) comprend au moins une Région Déterminante Complémentaire (CDR) choisie dans le groupe constitué par :
a. 4B4 CDRH1 qui a une séquence de SEQ ID NO : 21 ou une séquence qui diffère de SEQ ID NO : 21 par une substitution d'acide aminé unique ;
b. 4B4 CDRH2 qui a une séquence de SEQ ID NO : 23 ou une séquence qui diffère de SEQ ID NO : 23 par une substitution d'acide aminé unique ;
c. 4B4 CDRH3 qui a une séquence de SEQ ID NO : 25 ou une séquence qui diffère de SEQ ID NO : 25 par une substitution d'acide aminé unique ;
d. 4B4 CDRL1 qui a une séquence de SEQ ID NO : 15 ou une séquence qui diffère de SEQ ID NO : 15 par une substitution d'acide aminé unique ;
e. 4B4 CDRL2 qui a une séquence de SEQ ID NO : 17 ou une séquence qui diffère de SEQ ID NO : 17 par une substitution d'acide aminé unique ; et
f. 4B4 CDRL3 qui a une séquence de SEQ ID NO : 19 ou une séquence qui diffère de SEQ ID NO : 19 par une substitution d'acide aminé unique ;
(iii) comprend une région variable de chaîne lourde ayant une séquence au moins à 85 %, 90 %, 95 %, 98 %, 99 % ou 100 % identique à SEQ ID NO : 33 ;
(iv) comprend une séquence de région variable de chaîne légère ayant une séquence au moins à 85 %, 90 %, 95 %, 98 %, 99 % ou 100 % identique à SEQ ID NO : 31 ; ou
(v) entre en compétition avec l'anticorps de (i), (ii), (iii) ou (iv) ; et/ou
(b)
(i) le premier anticorps monoclonal et/ou le deuxième anticorps monoclonal a une affinité pour MCM5 dans la plage de 0,001 à 1 nM ;
(ii) le premier anticorps monoclonal et/ou le deuxième anticorps monoclonal est un Fab'2, un F'(ab)2, un Fv, un anticorps à chaîne unique ou un dianticorps ;
(iii) le premier anticorps monoclonal comprend 12A7 CDRH1, 12A7 CDRH2 et 12A7 CDRH3 ;
(iv) le premier anticorps monoclonal comprend 12A7 CDRL1, 12A7 CDRL2 et 12A7 CDRL3 ;
(v) le premier anticorps monoclonal comprend une 12A7 CDRH1 qui a une séquence de SEQ ID NO : 9, une 12A7 CDRH2 qui a une séquence de SEQ ID NO : 11, et une 12A7 CDRH3 qui a une séquence de SEQ ID NO : 13 ;
(vi) le premier anticorps monoclonal comprend une 12A7 CDRL1 qui a une séquence de SEQ ID NO : 3, une 12A7 CDRL2 qui a une séquence de SEQ ID NO : 5, et une 12A7 CDRL3 qui a une séquence de SEQ ID NO : 7 ;
(vii) le deuxième anticorps monoclonal comprend 4B4 CDRH1, 4B4 CDRH2 et 4B4 CDRH3 ;
(viii) le deuxième anticorps monoclonal comprend 4B4 CDRL1, 4B4 CDRL2 et 4B4 CDRL3 ;
(ix) le deuxième anticorps monoclonal comprend une 4B4 CDRH1 qui a une séquence de SEQ ID NO : 21, une 4B4 CDRH2 qui a une séquence de SEQ ID NO : 23, et une 4B4 CDRH3 qui a une séquence de SEQ ID NO : 25 ;
(x) le deuxième anticorps monoclonal a une 4B4 CDRL1 qui a une séquence de SEQ ID NO : 15, une 4B4 CDRL2 qui a une séquence de SEQ ID NO : 17, et une 4B4 CDRL3 qui a une séquence de SEQ ID NO : 19 ;
(xi) le premier anticorps monoclonal comprend une région variable de chaîne lourde ayant une séquence identique à au moins 95 % à SEQ ID NO : 29 ;
(xii) le premier anticorps monoclonal comprend une région variable de chaîne lourde ayant une séquence identique à au moins 98 % à SEQ ID NO : 29 ;
(xiii) le premier anticorps monoclonal comprend une région variable de chaîne légère ayant une séquence identique à au moins 95 % à SEQ ID NO : 27 ;
(xiv) le premier anticorps monoclonal comprend une région variable de chaîne légère ayant une séquence identique à au moins 98 % à SEQ ID NO : 27 ;
(xv) le deuxième anticorps monoclonal comprend une région variable de chaîne lourde ayant une séquence identique à au moins 95 % à SEQ ID NO : 33 ;
(xvi) le deuxième anticorps monoclonal comprend une région variable de chaîne lourde ayant une séquence identique à au moins 98 % à SEQ ID NO : 33 ;
(xvii) le deuxième anticorps monoclonal comprend une région variable de chaîne légère ayant une séquence identique à au moins 95 % à SEQ ID NO : 31 ; et/ou
(xviii) le deuxième anticorps monoclonal comprend une région variable de chaîne légère ayant une séquence identique à au moins 98 % à SEQ ID NO : 31.

13. Procédé selon l'une quelconque des revendications précédentes, dans lequel :
(a) l'échantillon d'urine, de tampon ou de frottis vaginal est un échantillon d'urine, et le procédé a une sensibilité plus élevée qu'un procédé équivalent réalisé en utilisant un échantillon de frottis et/ou un procédé équivalent réalisé en utilisant un échantillon de tampon ; et/ou
(b) l'échantillon d'urine, de tampon ou de frottis vaginal est un échantillon d'urine, et le procédé a une spécificité plus élevée qu'un procédé équivalent réalisé en utilisant un échantillon de frottis et/ou un procédé équivalent réalisé en utilisant un échantillon de tampon.

14. Procédé selon l'une quelconque des revendications précédentes, dans lequel :
(a)
(i) le procédé a une sensibilité pour le cancer gynécologique d'au moins environ 20 %, 22 %, 24 %, 26 %, 28 %, 30 % , 32 %, 34 %, 36 %, 38 %, 40 %, 42 %, 44 %, 46 %, 48 % ou 50 % ;
(ii) le procédé a une sensibilité pour le cancer gynécologique d'au moins environ 50 %, 52 %, 54 %, 55 %, 56 %, 57 %, 58 %, 59 %, 60 % ou 61 % ;
(iii) le procédé a une sensibilité pour le cancer gynécologique d'au moins environ 75 %, 76 %, 77 %, 78 %, 79 %, 80 % ou 81 % ;
(iv) le procédé a une sensibilité pour le cancer gynécologique d'au moins environ 90 %, 92 %, 93 %, 94 %, 95 % ou 96 % ;
(v) le procédé a une spécificité pour le cancer gynécologique d'au moins environ 20 %, 22 %, 24 %, 26 %, 28 %, 30 %, 32 %, 34 %, 36 %, 38 % ou 40 % ;
(vi) le procédé a une spécificité pour le cancer gynécologique d'au moins environ 50 %, 52 %, 54 %, 55 %, 56 %, 57 %, 58 %, 59 % ou 60 % ;
(vii) le procédé a une spécificité pour le cancer gynécologique d'au moins environ 60 %, 62 %, 64 %, 66 %, 68 %, 70 %, 71 %, 72 %, 73 %, 74 % ou 75 % ; et/ou
(viii) le procédé a une spécificité pour le cancer gynécologique d'au moins environ 80 %, 82 %, 83 %, 84 %, 85 % ou 86 % ; ou
(b)
(i) l'échantillon d'urine, de tampon ou de frottis vaginal est un échantillon d'urine et le procédé a une sensibilité pour le cancer gynécologique d'au moins environ 75 % et une spécificité pour le cancer gynécologique d'au moins environ 55 % ;
(ii) l'échantillon d'urine, de tampon ou de frottis vaginal est un échantillon de frottis vaginal et le procédé a une sensibilité pour le cancer gynécologique d'au moins environ 55 % et une spécificité pour le cancer gynécologique d'au moins environ 70 % ;
(iii) l'échantillon d'urine, de tampon ou de frottis vaginal est un échantillon de tampon et le procédé a une sensibilité pour le cancer gynécologique d'au moins environ 90 % et une spécificité pour le cancer gynécologique d'au moins environ 35 % ;
(iv) l'échantillon d'urine, de tampon ou de frottis vaginal est un échantillon de tampon et le procédé a une sensibilité pour le cancer gynécologique d'au moins environ 45 % et une spécificité pour le cancer gynécologique d'au moins environ 80 % ; ou
(v) l'échantillon d'urine, de tampon ou de frottis vaginal est un échantillon de tampon et le procédé a une sensibilité pour le cancer gynécologique d'au moins environ 90 % et une spécificité pour le cancer gynécologique d'au moins environ 80 %.

15. Utilisation d'un tampon de lyse, ou d'un premier anticorps monoclonal et/ou d'un deuxième anticorps monoclonal, ou d'un kit dans un procédé de détection de la présence ou de l'absence d'un cancer gynécologique chez un sujet selon l'une quelconque des revendications précédentes, dans laquelle le kit comprend ledit tampon de lyse, ledit premier anticorps monoclonal et/ou ledit deuxième anticorps monoclonal.

16. Procédé selon l'une quelconque des revendications 1 à 14 ou utilisation selon la revendication 15, dans lequel :
(a) le cancer gynécologique est une tumeur maligne gynécologique ;
(b) le cancer gynécologique est choisi dans le groupe constitué par le cancer de l'ovaire, le cancer de l'endomètre, le cancer de l'utérus, le cancer du col de l'utérus, le cancer de la vulve, le cancer du vagin, la tumeur des trompes de Fallope, la tumeur épithéliale de l'ovaire, le tératome de l'ovaire, le tératome de l'ovaire immature, la tumeur du stroma, la tumeur de l'ovaire des cellules germinales, les tumeurs indifférenciées, la tumeur limite de l'ovaire, le carcinome de l'endomètre, l'adénocarcinome de l'endomètre, l'adénocarcinome endométrioïde, le carcinome épidermoïde de l'endomètre, le carcinome séreux, le carcinome intraépithélial séreux de l'endomètre, le carcinosarcome de l'endomètre, le carcinome à cellules claires, le carcinome mucineux, le carcinome de l'endomètre indifférencié, le carcinome de l'endomètre mixte, la tumeur mucineuse, la tumeur de Müller mixte maligne, le sarcome à cellules claires de l'endomètre, la tumeur de la granulosa, le carcinome intraépithélial tubaire séreux, le tératome kystique immature, la tumeur ovarienne séreuse et le carcinome à petites cellules ;
(c) le cancer gynécologique est un cancer de l'ovaire ou un cancer de l'endomètre ;
(d) le cancer gynécologique est un cancer de l'ovaire, éventuellement dans lequel le cancer de l'ovaire est choisi dans le groupe constitué par la tumeur épithéliale de l'ovaire, le tératome de l'ovaire, le tératome de l'ovaire immature, la tumeur du stroma, la tumeur de l'ovaire des cellules germinales, les tumeurs indifférenciées, la tumeur de l'ovaire limite, la tumeur mucineuse tumeur, la tumeur des cellules de la granulosa, le tératome kystique immature, la tumeur séreuse de l'ovaire et le carcinome à petites cellules ;
(e) le cancer gynécologique est un cancer de l'endomètre, éventuellement dans lequel le cancer de l'endomètre est choisi dans le groupe constitué par le carcinome de l'endomètre, l'adénocarcinome de l'endomètre, l'adénocarcinome de l'endomètre, le carcinome épidermoïde de l'endomètre, le carcinome séreux, le carcinome intraépithélial séreux de l'endomètre, le carcinosarcome de l'endomètre, le carcinome à cellules claires, le carcinome mucineux, le carcinome indifférencié de l'endomètre, le carcinome mixte de l'endomètre, la tumeur maligne mixte de Müller et le sarcome à cellules claires de l'endomètre ; et/ou
(f) le cancer gynécologique est :
(i) un cancer de bas grade, éventuellement un cancer G1 ; ou
(ii) un cancer de haut grade, éventuellement un cancer G2 ou supérieur ; et/ou
(g) le cancer gynécologique est :
(i) un cancer à un stade précoce, éventuellement un cancer S1 ; ou
(ii) un cancer à un stade avancé, éventuellement un cancer S2 ou supérieur.

17. Procédé ou utilisation selon l'une quelconque des revendications précédentes, dans lequel :
(a)
(i) le cancer gynécologique est un cancer de l'ovaire et le procédé a une spécificité pour le cancer de l'ovaire d'au moins environ 25 % ;
(ii) le cancer gynécologique est un cancer de l'ovaire et le procédé a une sensibilité pour le cancer de l'ovaire d'au moins environ 50 % ;
(iii) le cancer gynécologique est un cancer de l'ovaire et le procédé a une spécificité pour le cancer de l'ovaire d'au moins environ 25 % et une sensibilité pour le cancer de l'ovaire d'au moins environ 50 % ;
(iv) le cancer gynécologique est un cancer de l'endomètre et le procédé a une spécificité pour le cancer de l'endomètre d'au moins environ 40 % ;
(v) le cancer gynécologique est un cancer de l'endomètre et le procédé a une sensibilité pour le cancer de l'endomètre d'au moins environ 80 % ; et/ou
(vi) le cancer gynécologique est un cancer de l'endomètre et le procédé a une spécificité pour le cancer de l'endomètre d'au moins environ 40 % et une sensibilité pour le cancer de l'endomètre d'au moins environ 80 % ;
(b)
(i) l'échantillon d'urine, de tampon ou de frottis vaginal est un échantillon d'urine, le cancer gynécologique est un cancer de l'ovaire, et le procédé a une sensibilité pour le cancer de l'ovaire d'au moins environ 60 % et une spécificité pour le cancer de l'ovaire d'au moins environ 70 %, éventuellement dans lequel une référence de concentration de MCM5 d'environ 12 pg/ml est appliquée ; ou
(ii) l'échantillon d'urine, de tampon ou de frottis vaginal est un échantillon d'urine, le cancer gynécologique est un cancer de l'endomètre, et le procédé a une sensibilité pour le cancer de l'endomètre d'au moins environ 80 % et une spécificité pour le cancer de l'endomètre d'au moins environ 70 %, éventuellement dans lequel une référence de concentration de MCM5 d'environ 12 pg/ml est appliquée ; ou
(c)
(i) l'échantillon d'urine, de tampon ou de frottis vaginal est un échantillon d'urine, le cancer gynécologique est un cancer de l'ovaire de bas grade et le procédé a une sensibilité pour le cancer de l'ovaire de bas grade d'au moins environ 70 %, éventuellement dans lequel une référence de concentration de MCM5 d'environ 12 pg/ml est appliquée ;
(ii) l'échantillon d'urine, de tampon ou de frottis vaginal est un échantillon d'urine, le cancer gynécologique est un cancer de l'ovaire à un stade précoce et le procédé a une sensibilité pour le cancer de l'ovaire à un stade précoce d'au moins environ 70 %, éventuellement dans lequel une référence de concentration de MCM5 d'environ 12 pg/ml est appliqué ;
(iii) l'échantillon d'urine, de tampon ou de frottis vaginal est un échantillon d'urine, le cancer gynécologique est un cancer de l'endomètre de bas grade et le procédé a une sensibilité pour le cancer de l'endomètre de bas grade d'au moins environ 80 %, éventuellement dans lequel une référence de concentration de MCM5 d'environ 12 pg/ml est appliqué ; ou
(iv) l'échantillon d'urine, de tampon ou de frottis vaginal est un échantillon d'urine, le cancer gynécologique est un cancer de l'endomètre à un stade précoce et le procédé a une sensibilité pour le cancer de l'endomètre à un stade précoce d'au moins environ 85 %, éventuellement dans lequel une référence de concentration de MCM5 d'environ 12 pg/ml est appliquée.

18. Procédé ou utilisation selon l'une quelconque des revendications précédentes, dans lequel le sujet souffre d'une affection gynécologique bénigne, éventuellement dans lequel l'affection gynécologique bénigne est choisie parmi les saignements post-ménopausiques (PMB), l'endométriose, les fibromes et le syndrome des ovaires polykystiques (SOPK).
